# EUROPEAN PATENT APPLICATION

(11) **EP 4 049 726 A1**
(43) Date of publication of application: **31.08.2022**
(21) Application number: 21382160.6
(22) Date of filing: 25.02.2021
(51) Int. Cl.: A61P 35/00, C07D 487/04, A61K 31/519

(54) **IMIDAZO[1,2-A]PYRAZINES AS INHIBITORS OF HASPIN AND THERAPEUTIC USES THEREOF**

(71) Applicant: Fundación del Sector Público Estatal Centro Nacional de Investigaciones Oncológicas Carlos III (F.S.P. CNIO), 28029 Madrid (ES)
(72) Inventor: PASTOR FERNÁNDEZ, Joaquín, 28029 Madrid (ES); MARTÍNEZ GONZÁLEZ, Sonia, 28029 Madrid (ES); BLANCO-APARICIO, Carmen, 28029 Madrid (ES); GONZÁLEZ CANTALAPIEDRA, Esther, 28029 Madrid (ES); GARCÍA GARCÍA, Ana Belén, 28029 Madrid (ES); PASTOR FERNÁNDEZ, Miryam, 28029 Madrid (ES); HERNÁNDEZ HIGUERAS, Ana Isabel, 28029 Madrid (ES); ALBARRÁN SANTIÑO, María Isabel, 28029 Madrid (ES); CEBRIÁ GÓMEZ, Antonio, 28029 Madrid (ES)
(74) Representative: Elzaburu S.L.P.

(57) **Abstract**

The present invention relates to a group of compounds with an imidazo[1,2-a]pyrazine core of formula (I): 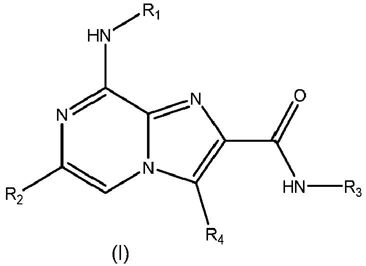 which are inhibitors of HASPIN, whose activity is required for the proliferation of certain tumoral cells, so the compounds of the invention are useful for the prevention and/or treatment of cancer.

## Description

### FIELD OF THE INVENTION

The present invention relates to a group of compounds with an imidazo[1 ,2-a]pyrazine core, which are inhibitors of the protein kinase HASPIN. The compounds of the invention are useful for the treatment of cancer that depends on HASPIN. Therefore, the present invention belongs to the field of pharmacology or medicinal chemistry.

### BACKGROUND OF THE INVENTION

HASPIN (also known as germ cell-specific gene 2 protein/GSG2 or haploid germ cell-specific nuclear protein kinase) is a serine/threonine kinase. Its kinase domain shows a similar conformation to protein kinase ePK domain but diverges in crucial ways from typical ePK members. HASPIN lacks both the conserved ATP/Mg2+ binding motif Asp-Phe-Gly (DFG), which is replaced by Asp-Tyr-Thr (DYT), and the Ala-Pro-Glu (APE) motif usually found at the C terminus of the activation segment. HASPIN do not require phoshorylation of the activation loop to be active and the kinase domain of HASPIN alone is active *in vitro.* Accordingly, HASPIN is often classified as an atypical ePK family member.

HASPIN is overexpressed in some malignant tumors such as Burkitt's lymphoma, chronic lymphocytic leukemias (Dave et al., The New England Journal of Medicine. 2006, 354(23), 2431-2442), pancreatic cancer (PDAC) (Bastea et al., Sci Rep. 2019 Nov 12;9(1):16588), gallbladder carcinoma (GBC) (Zhu et al., Exp Cell Res. 2020 May 15;390 (2)), bladder cancer (Chen et al., Aging (Albany NY). 2020 May 21 ;12(10):8858-8879), prostate cancer (PCa) (Yu et al., IntJ Oncol. 2020 Jul;57(1):139-150) and ovarian cancer (Huang et al., Oncogene.2020 May;39(21):4312-4322). In pancreatic cancer, patients with tumors with high HASPIN expression levels have shown a decrease in survival as compared to patients with tumors that express low levels of it. Moreover, the expression of HASPIN in GBC has been found up-regulated and positively correlated with the pathological grade of GBC. Also, HASPIN has been found up-regulated in bladder cancer tissues compared with the normal tissues and its high expression has been correlated with more advanced malignant grade and lower survival rate. Furthermore, HASPIN expression has been significantly associated with the development and progression of PCa. Finally, in ovarian cancer upregulation of HASPIN positively correlates with tumor grade and AJCC stage and negative correlated with patients' prognosis.

After HASPIN knockdown, the proliferation and clone-formation ability of GBC cells were inhibited as well as the growth of GBC *in vivo* (Zhu et al. Exp Cell Res. 2020 May 15;390 (2)). HASPIN knockdown in pancreatic cancer cells also inhibited cell proliferation, colony formation and migration, blocked cell cycle at G2 phase and induced cell apoptosis (Han, X. et al., Experimental Cell Research 2019, 385(1), 111605). In bladder cancer cells, the overexpression/knockdown of GSG2 promote/inhibit proliferation, colony formation and migration, while inhibiting/promoting cell apoptosis as well as knockdown of HASPIN suppress tumorigenicity of bladder cancer cells *in vivo.* KIF15 has been identified as the potential target of HASPIN in bladder cancer (Chen et al. 2020). Moreover, in prostate cancer cell lines, both *in vitro* and *in vivo,* HASPIN knockdown suppressed cell proliferation and colony formation promoting apoptosis (Yu et al. 2020). In addition, HASPIN has been identified in a whole kinome siRNA screen, together with Plk1, as one of the top hit kinases, whose depletion decreased both cell viability and estrogen receptor transcriptional activity in MCF7 breast cancer cells (Bhola et al., Cancer Research. 2015 Jan 15;75(2):405-414). Also, HASPIN has been described as one of the most upregulated proteins in FGF2-mediated resistance to EGFR inhibition. These cells are very sensitive to HASPIN inhibition and the combination of EGFR/HASPIN-i is much more effective that any treatment alone. Then, a combination of HASPIN-i and EGFR-i could be and option to prevent mitogen mediated resistance to EGFR-I (Koch et al., J. Proteome Res. 2016 Dec 2;15(12):4490-4504). Finally, HASPIN depletion has showed synthetic lethal interaction with VX-680 treatment (AURORA-i), specifically by inhibition of Aurora kinase B (Huang et al. Oncogene.2020 May;39(21):4312-4322).

Document WO2007/145921 refers to imidazo[1,2-a]pyrazine compounds useful as protein kinase inhibitors, regulators or modulators. These compounds are useful for the treatment of cancer, among other diseases. Document US2011/0053929 refers to substituted imidazo-, pyrazolopyrazines and imidazotriazines compounds, which are useful for treating cancer, among other diseases. Document US2012/0083492 describes compounds with a imidazo[1,2-a]pyrazine core, which are useful in the treatment of diseases in which inhibition of a protein or lipid kinase (e.g. a PI3-K and/or mTOR) is desired and/or required, and particularly in the treatment of cancer or a proliferative disease. In US2004/0220189 certain 8-amino-aryl-substituted imidazo[1,2-a]pyrazines which modulate the activity of protein kinases are described.

Therefore, there is a clear need to obtain effective inhibitors of kinases whose activity is required for tumor cells proliferation, such as HASPIN, as it would be a good approach for the treatment of cancer.

### DESCRIPTION OF THE INVENTION

The present invention refers to a group of compounds with an imidazo[1,2-a]pyrazine core, which are inhibitors of HASPIN. Regarding the role of HASPIN in certain tumoral cells as described above, these compounds are useful for the treatment and/or prevention of cancer.

Thus, in a first aspect, the invention refers to a compound of formula (I): 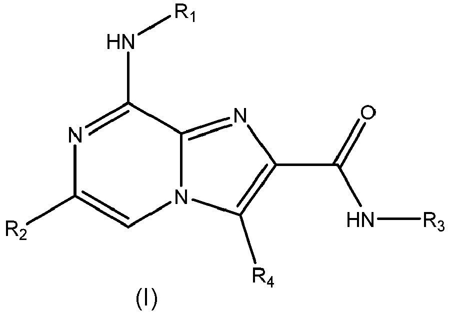 wherein:
R₁ is selected from the following groups:
   - alkyl C₁-C₆ optionally substituted by cycloalkyl C₃-C₆ or heterocycloalkyl C₃-C₆, wherein these substituents may be substituted by a group selected from alkyl C₁-C₆, CN, OH, halo,
   - cycloalkyl C₃-C₆ optionally substituted by a group selected from OH, NH₂, alkyl C₁-C₆ optionally substituted by NH₂,
   - heterocycloalkyl C₃-C₆;
R₂ is a C₅-C₆ heteroaryl optionally substituted by a group selected from NH₂, alkyl C₁-C₆ or halo;
R₃ is selected from H or alkyl C₁-C₆ optionally substituted by O-alkyl C₁-C₄;
R₄ is selected from H or alkyl C₁-C₆;
or a pharmaceutically acceptable ester, amide, solvate or salt thereof.

Preferably, in the compound of formula (I) of the invention, R₁ is selected from the following groups: 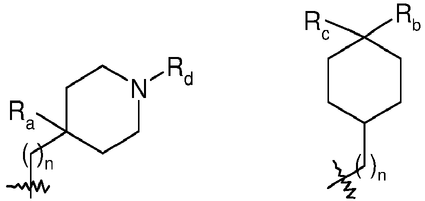 wherein:
n is selected from 0, 1 or 2;
Ra is selected from H, halo, CN, OH;
Rb is selected from H, OH, NH₂;
Rc is selected from H, alkyl C₁-C₄ optionally substituted by NH₂;
Rd is selected from H or alkyl C₁-C₄.

Preferably, in the compound of formula (I) of the invention, R₂ is selected from pyridyl, thiazolyl, pyrazolyl or isothiazolyl. More preferably, R₂ is 4-pyridyl optionally substituted by halo, NH₂ or alkyl C₁-C₄.

Preferably, in the compound of formula (I) of the invention, R₃ is selected from H or alkyl C₁-C₄ optionally substituted by O-alkyl C₁-C₂.

Preferably, in the compound of formula (I) of the invention, R₄ is selected from H or alkyl C₁-C₄.

Preferably, the compound of formula (I) of the invention is selected from the following list:
- 8-(Piperidin-4-ylamino)-6-pyridin-4-yl-imidazo[1,2-a]pyrazine-2-carboxylic acid amide (1)
- 8-[(Piperidin-4-ylmethyl)-amino]-6-pyridin-4-yl-imidazo[1,2-a]pyrazine-2-carboxylic acid amide (2)
- 6-(2-Chloro-pyridin-4-yl)-8-[(piperidin-4-ylmethyl)-amino]-imidazo[1,2-a]pyrazine-2-carboxylic acid amide (3)
- 8-[(Piperidin-4-ylmethyl)-amino]-6-pyridin-4-yl-imidazo[1,2-a]pyrazine-2-carboxylic acid methylamide (4)
- 8-[(Piperidin-4-ylmethyl)-amino]-6-pyridin-4-yl-imidazo[1,2-a]pyrazine-2-carboxylic acid (2-methoxy-ethyl)-amide (5)
- 6-(2-Chloro-pyridin-4-yl)-8-[(4-cyano-piperidin-4-ylmethyl)-amino]-imidazo[1,2-a]pyrazine-2-carboxylic acid amide (6)
- 3-Methyl-8-[(piperidin-4-ylmethyl)-amino]-6-pyridin-4-yl-imidazo[1,2-a]pyrazine-2-carboxylic acid methylamide (7)
- 8-[(4-Cyano-piperidin-4-ylmethyl)-amino]-6-pyridin-4-yl-imidazo[1,2-a]pyrazine-2-carboxylic acid amide (8)
- 3-Methyl-8-[(piperidin-4-ylmethyl)-amino]-6-pyridin-4-yl-imidazo[1,2-a]pyrazine-2-carboxylic acid amide (9)
- 3-Methyl-8-[(piperidin-4-ylmethyl)-amino]-6-pyridin-4-yl-imidazo[1,2-a]pyrazine-2-carboxylic acid (2-methoxy-ethyl)-amide (10)
- 6-(2-Chloro-pyridin-4-yl)-3-methyl-8-[(piperidin-4-ylmethyl)-amino]-imidazo[1,2-a]pyrazine-2-carboxylic acid methylamide (11)
- 6-(2-Chloro-pyridin-4-yl)-3-methyl-8-[(piperidin-4-ylmethyl)-amino]-imidazo[1,2-a]pyrazine-2-carboxylic acid amide (12)
- 6-(2-Chloro-pyridin-4-yl)-3-methyl-8-[(piperidin-4-ylmethyl)-amino]-imidazo[1,2-a]pyrazine-2-carboxylic acid (2-methoxy-ethyl)-amide (13)
- 8-[(4-Fluoro-piperidin-4-ylmethyl)-amino]-3-methyl-6-pyridin-4-yl-imidazo[1,2-a]pyrazine-2-carboxylic acid methylamide (14)
- 8-[(4-Fluoro-piperidin-4-ylmethyl)-amino]-6-pyridin-4-yl-imidazo[1,2-a]pyrazine-2-carboxylic acid amide (15)
- 8-[(4-Fluoro-piperidin-4-ylmethyl)-amino]-3-methyl-6-pyridin-4-yl-imidazo[1,2-a]pyrazine-2-carboxylic acid amide (16)
- 8-[(4-Cyano-piperidin-4-ylmethyl)-amino]-3-methyl-6-pyridin-4-yl-imidazo [1,2-a]pyrazine-2-carboxylic acid methylamide (17)
- 8-[(4-Cyano-piperidin-4-ylmethyl)-amino]-3-methyl-6-pyridin-4-yl-imidazo[1,2-a]pyrazine-2-carboxylic acid (2-methoxy-ethyl)-amide (18)
- 8-[(4-Cyano-piperidin-4-ylmethyl)-amino]-3-methyl-6-pyridin-4-yl-imidazo[1,2-a]pyrazine-2-carboxylic acid amide (19)
- 6-(2-Chloro-pyridin-4-yl)-8-[(4-fluoro-piperidin-4-ylmethyl)-amino]-3-methyl-imidazo[1,2-a]pyrazine-2-carboxylic acid methylamide (20)
- 6-(2-Chloro-pyridin-4-yl)-8-[(4-cyano-piperidin-4-ylmethyl)-amino]-3-methyl-imidazo[1,2-a]pyrazine-2-carboxylic acid methylamide (21)
- 6-(2-Chloro-pyridin-4-yl)-8-[(4-fluoro-piperidin-4-ylmethyl)-amino]-3-methyl-imidazo[1,2-a]pyrazine-2-carboxylic acid (2-methoxy-ethyl)-amide (22)
- 6-(2-Chloro-pyridin-4-yl)-8-[(4-cyano-piperidin-4-ylmethyl)-amino]-3-methyl-imidazo[1,2-a]pyrazine-2-carboxylic acid (2-methoxy-ethyl)-amide (23)
- 6-(2-Chloro-pyridin-4-yl)-8-[(4-cyano-piperidin-4-ylmethyl)-amino]-3-methyl-imidazo[1,2-a]pyrazine-2-carboxylic acid amide (24)
- 6-(2-Chloro-pyridin-4-yl)-8-[(4-fluoro-piperidin-4-ylmethyl)-amino]-3-methyl-imidazo[1,2-a]pyrazine-2-carboxylic acid amide (25)
- 8-[(1-Propyl-piperidin-4-ylmethyl)-amino]-6-pyridin-4-yl-imidazo[1,2-a]pyrazine-2-carboxylic acid methylamide (26)
- 6-(3-Fluoro-pyridin-4-yl)-8-[(piperidin-4-ylmethyl)-amino]-imidazo[1,2-a]pyrazine-2-carboxylic acid amide (27)
- 6-(3-Fluoro-pyridin-4-yl)-8-[(piperidin-4-ylmethyl)-amino]-imidazo[1,2-a]pyrazine-2-carboxylic acid methylamide (28)
- 8-[(4-Cyano-piperidin-4-ylmethyl)-amino]-6-(3-fluoro-pyridin-4-yl)-imidazo[1,2-a]pyrazine-2-carboxylic acid amide (29)
- 6-(3-Chloro-pyridin-4-yl)-8-[(piperidin-4-ylmethyl)-amino]-imidazo[1,2-a]pyrazine-2-carboxylic acid amide (30)
- 6-(3-Chloro-pyridin-4-yl)-8-[(piperidin-4-ylmethyl)-amino]-imidazo[1,2-a]pyrazine-2-carboxylic acid methylamide (31)
- 8-[(4-Cyano-piperidin-4-ylmethyl)-amino]-6-(3-fluoro-pyridin-4-yl)-imidazo[1,2-a]pyrazine-2-carboxylic acid methylamide (32)
- 8-[(4-Fluoro-piperidin-4-ylmethyl)-amino]-6-(3-fluoro-pyridin-4-yl)-imidazo[1,2-a]pyrazine-2-carboxylic acid amide (33)
- 6-(3-Fluoro-pyridin-4-yl)-3-methyl-8-[(piperidin-4-ylmethyl)-amino]-imidazo[1,2-a]pyrazine-2-carboxylic acid methylamide (35)
- 6-(2-Methyl-pyridin-4-yl)-8-[(piperidin-4-ylmethyl)-amino]-imidazo[1,2-a]pyrazine-2-carboxylic acid amide (36)
- 8-(2-Piperidin-4-yl-ethylamino)-6-pyridin-4-yl-imidazo[1,2-a]pyrazine-2-carboxylic acid amide (37)
- 8-(4-trans-Amino-cyclohexylamino)-6-pyridin-4-yl-imidazo[1,2-a]pyrazine-2-carboxylic acid amide (38)
- 8-(cis-4-Amino-cyclohexylamino)-6-pyridin-4-yl-imidazo[1,2-a]pyrazine-2-carboxylic acid amide (39)
- 8-(trans-4-Aminomethyl-cyclohexylamino)-6-pyridin-4-yl-imidazo[1,2-a]pyrazine-2-carboxylic acid amide (40)
- 6-(2-Amino-pyridin-4-yl)-8-[(4-fluoro-piperidin-4-ylmethyl)-amino]-imidazo[1,2-a]pyrazine-2-carboxylic acid amide (41)
- 8-[trans-(4-Amino-cyclohexylmethyl)-amino]-6-pyridin-4-yl-imidazo[1,2-a]pyrazine-2-carboxylic acid amide (42)
- 8-[(4-Hydroxy-piperidin-4-ylmethyl)-amino]-6-pyridin-4-yl-imidazo[1,2-a]pyrazine-2-carboxylic acid amide (44)
- 8-[(4-Fluoro-piperidin-4-ylmethyl)-amino]-6-(1-methyl-1H-pyrazol-3-yl)-imidazo[1,2-a]pyrazine-2-carboxylic acid amide (45)
- 8-[(4-Fluoro-piperidin-4-ylmethyl)-amino]-6-isothiazol-5-yl-imidazo[1,2-a]pyrazine-2-carboxylic acid amide (46)
- 8-[(4-Fluoro-piperidin-4-ylmethyl)-amino]-6-(1H-pyrazol-4-yl)-imidazo[1,2-a]pyrazine-2-carboxylic acid amide (47)
- 6-(3-Fluoro-pyridin-4-yl)-8-(4-hydroxy-4-methyl-cyclohexylamino)-imidazo[1,2-a]pyrazine-2-carboxylic acid amide (48)
- 8-[(4-Fluoro-piperidin-4-ylmethyl)-amino]-6-thiazol-5-yl-imidazo[1,2-a]pyrazine-2-carboxylic acid amide (49)

More preferably, the compound of formula (I) is selected from the following list:
- 8-[(Piperidin-4-ylmethyl)-amino]-6-pyridin-4-yl-imidazo[1,2-a]pyrazine-2-carboxylic acid amide (2)
- 6-(2-Chloro-pyridin-4-yl)-8-[(piperidin-4-ylmethyl)-amino]-imidazo[1,2-a]pyrazine-2-carboxylic acid amide (3)
- 8-[(Piperidin-4-ylmethyl)-amino]-6-pyridin-4-yl-imidazo[1,2-a]pyrazine-2-carboxylic acid methylamide (4)
- 6-(2-Chloro-pyridin-4-yl)-8-[(4-cyano-piperidin-4-ylmethyl)-amino]-imidazo[1,2-a]pyrazine-2-carboxylic acid amide (6)
- 3-Methyl-8-[(piperidin-4-ylmethyl)-amino]-6-pyridin-4-yl-imidazo[1,2-a]pyrazine-2-carboxylic acid methylamide (7)
- 8-[(4-Cyano-piperidin-4-ylmethyl)-amino]-6-pyridin-4-yl-imidazo[1,2-a]pyrazine-2-carboxylic acid amide (8)
- 6-(2-Chloro-pyridin-4-yl)-3-methyl-8-[(piperidin-4-ylmethyl)-amino]-imidazo[1,2-a]pyrazine-2-carboxylic acid methylamide (11)
- 8-[(4-Fluoro-piperidin-4-ylmethyl)-amino]-3-methyl-6-pyridin-4-yl-imidazo[1,2-a]pyrazine-2-carboxylic acid methylamide (14)
- 8-[(4-Fluoro-piperidin-4-ylmethyl)-amino]-6-pyridin-4-yl-imidazo[1,2-a]pyrazine-2-carboxylic acid amide (15)
- 8-[(4-Fluoro-piperidin-4-ylmethyl)-amino]-3-methyl-6-pyridin-4-yl-imidazo[1,2-a]pyrazine-2-carboxylic acid amide (16)
- 8-[(4-Cyano-piperidin-4-ylmethyl)-amino]-3-methyl-6-pyridin-4-yl-imidazo [1,2-a]pyrazine-2-carboxylic acid methylamide (17)
- 8-[(4-Cyano-piperidin-4-ylmethyl)-amino]-3-methyl-6-pyridin-4-yl-imidazo[1,2-a]pyrazine-2-carboxylic acid amide (19)
- 6-(2-Chloro-pyridin-4-yl)-8-[(4-fluoro-piperidin-4-ylmethyl)-amino]-3-methyl-imidazo[1,2-a]pyrazine-2-carboxylic acid methylamide (20)
- 6-(3-Fluoro-pyridin-4-yl)-8-[(piperidin-4-ylmethyl)-amino]-imidazo[1,2-a]pyrazine-2-carboxylic acid amide (27)
- 6-(3-Fluoro-pyridin-4-yl)-8-[(piperidin-4-ylmethyl)-amino]-imidazo[1,2-a]pyrazine-2-carboxylic acid methylamide (28)
- 8-[(4-Cyano-piperidin-4-ylmethyl)-amino]-6-(3-fluoro-pyridin-4-yl)-imidazo[1,2-a]pyrazine-2-carboxylic acid amide (29)
- 8-[(4-Cyano-piperidin-4-ylmethyl)-amino]-6-(3-fluoro-pyridin-4-yl)-imidazo[1,2-a]pyrazine-2-carboxylic acid methylamide (32)
- 8-[(4-Fluoro-piperidin-4-ylmethyl)-amino]-6-(3-fluoro-pyridin-4-yl)-imidazo[1,2-a]pyrazine-2-carboxylic acid amide (33)
- 6-(3-Fluoro-pyridin-4-yl)-3-methyl-8-[(piperidin-4-ylmethyl)-amino]-imidazo[1,2-a]pyrazine-2-carboxylic acid methylamide (35)
- 6-(2-Methyl-pyridin-4-yl)-8-[(piperidin-4-ylmethyl)-amino]-imidazo[1,2-a]pyrazine-2-carboxylic acid amide (36)
- 8-(cis-4-Amino-cyclohexylamino)-6-pyridin-4-yl-imidazo[1,2-a]pyrazine-2-carboxylic acid amide (39)
- 6-(2-Amino-pyridin-4-yl)-8-[(4-fluoro-piperidin-4-ylmethyl)-amino]-imidazo[1,2-a]pyrazine-2-carboxylic acid amide (41)
- 8-[(4-Hydroxy-piperidin-4-ylmethyl)-amino]-6-pyridin-4-yl-imidazo[1,2-a]pyrazine-2-carboxylic acid amide (44)
- 8-[(4-Fluoro-piperidin-4-ylmethyl)-amino]-6-isothiazol-5-yl-imidazo[1,2-a]pyrazine-2-carboxylic acid amide (46).

Another aspect of the invention refers to compound of formula (I) as previously described for use as a medicament.

Another aspect of the invention refers to compound of formula (I) as previously described for use in the prevention or treatment of cancer.

More preferably, the cancer is selected from Burkitt's lymphoma, chronic lymphocytic leukemias, pancreatic cancer, gallbladder carcinoma, bladder cancer, prostate cancer, melanoma, breast cancer, ovarian cancer.

Another aspect of the invention refers to a composition comprising a compound of formula (I) as previously described and a pharmaceutically acceptable excipient, diluent or carrier.

In the present invention, the term "alkyl" refers to linear or branched hydrocarbonated chain radicals, with between 1 and 6 carbon atoms, preferably between 1 and 4, which bind to the rest of the molecule by means of a single bond, for example, propyl, ethyl, methyl, isopropyl, undecanoyl, heptadecanoyl, octadecanoyl, etc. These alkyl radicals may be optionally substituted in one or more positions by one or more groups, such as cycloalkyl, hydroxyl, amines, amides, oxo, cyano, halogens, aryl, etc.

In the present invention, the term "cycloalkyl" refers to non-aromatic monocyclic or polycyclic ring comprising carbon and hydrogen atoms, preferably with between 3 and 6 carbon atoms, and more preferably 6, totally or partially saturated, and formed by only carbon and hydrogen atoms. They may be optionally substituted by one or more groups, such as alkyl, halogens, hydroxyl, amines, amides, cyano, etc. Examples of alkyl groups include, but are not limited to, cyclopropyl, cyclobutyl, cyclopentyl or cyclohexyl.

In the present invention, the term "heterocycloalkyl" refers to non-aromatic monocyclic or polycyclic ring comprising carbon and hydrogen atoms and at least one heteroatom (nitrogen, oxygen or sulfur). Preferably is a 4 to 8-member ring with one or more heteroatoms, more preferably is a 6-member ring with one or more heteroatoms. They may be optionally substituted by one or more groups, such as alkyl, halogens, hydroxyl, amines, amides, cyano, etc. Examples of heterocycloalkyl groups include, but are not limited to, piperazinyl, pyrrolidinyl, dioxanyl, morpholinyl, tetrahydrofuranyl, etc.

In the present invention, the term "heteroaryl" refers to an aromatic ring, which have between 5 and 14 links wherefrom a proton has been eliminated from the ring, having at least one heteroatom (nitrogen, oxygen or sulfur). Preferably, the heteroaryl group has between 5 and 8 carbon atoms, more preferably 5 or 6. The heteroaryl radicals may be optionally substituted by one or more substituents, such as alkyl, hydroxyl, amines, amide, cyano, halogens, etc. Examples of heteroaryl groups include, but are not limited to, pyridyl, pyrazolyl, thiazolyl, isothiazolyl, furyl, imidazolyl, benzimidazolyl, thienyl, quinolinyl, indolyl, etc.

### "Halo" or "halogen" refers to fluorine, chlorine, bromine or iodine.

The compounds of the present invention represented by the formula (I), and more specifically, the specific compounds belonging to this previously described general formula may include isomers, depending on the presence of multiple bonds (for example, Z, E), including optic isomers or enantiomers, depending on the presence of chiral centres. The individual isomers, enantiomers or diastereoisomers and the mixtures thereof fall within the scope of the present invention. The individual enantiomers or diastereoisomers, as well as their mixtures, can be separated by conventional techniques.

The compounds of the invention may be in crystalline form as free compounds or in solvate form, intending both forms to be within the scope of the present invention. In this sense, the term "solvate", as used herein, includes both pharmaceutically acceptable solvates, in other words, solvates of the compound of formula (I) which can be used to produce a medicament, as well as pharmaceutically unacceptable solvates, which can be useful to produce pharmaceutically acceptable solvates or salts. The nature of the pharmaceutically acceptable solvate is not critical on condition that it is pharmaceutically acceptable. In a particular embodiment, the solvate is a hydrate. The solvates can be obtained by conventional solvation methods known to technicians in the art.

For therapeutic application, the compounds of formula (I), their salts or solvates, will come preferably in a pharmaceutically acceptable or substantially pure form, in other words, having a pharmaceutically acceptable level of purity excluding standard drugs such as diluents and carriers, and not including material considered toxic at standard dose levels. The purity levels for the active principle are preferably higher than 50%, more preferably higher than 70%, more preferably higher than 90%. In a preferred embodiment, they are higher than 95% of the compound of formula (I), or the salts or solvates thereof.

Another aspect of the invention refers to a composition comprising a compound of formula (I) as described above and a pharmaceutically acceptable excipient, diluent or carrier. Preferably, said composition further comprises an antitumoral compound, which may be any chemotherapeutic agent authorized to be used in humans or in the process of clinical trials and/or pending of said authorization. Examples of said chemotherapeutic agents may be found, for example, in patent EP3341376.

The term "excipients, diluent or carriers" relates to molecular entities or substances through which the active ingredient is administered. Such pharmaceutical excipients, diluents or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are known in the art by a skilled person.

The compounds of formula (I) for therapeutic use are prepared in solid form or aqueous suspension, in a pharmaceutically acceptable diluent. These preparations may be administered via any appropriate route of administration, wherefore said preparation will be formulated in the suitable pharmaceutical form for the selected route of administration. In a particular embodiment, the compound of formula (I) provided by this invention is administered orally, topically, rectally or parenterally (including subcutaneously, intraperitoneally, intradermally, intramuscularly, intravenously, etc.). A review of the different pharmaceutical forms for administering medicaments and the excipients required to obtain them can be found in the standard Pharmacopoeias of Europe and the US.

The compounds described in the present invention, their pharmaceutically acceptable salts, and solvates, as well as the pharmaceutical compositions containing them can be used in conjunction with other additional drugs in order to provide a combination therapy. Said additional drugs may form part of the same pharmaceutical composition or, alternatively, may be provided in the form of a separate composition for administration simultaneously or not with the administration of the pharmaceutical composition comprising a compound of formula (I), or pharmaceutically acceptable solvate or salt thereof.

Another additional aspect of the present invention refers to a method for treating cancer, comprising the administration of a therapeutically effective amount of a compound of formula (I) as described above.

In the sense used in this description, the expression "therapeutically effective amount" refers to the quantity of agent or compound capable of having an effect on the levels of proliferation in primary cultured cells, calculated to produce the required effect in vivo and, in general, will be determined, among other aspects, by the inherent properties of the compounds, including the age, state of the patient, severity of the alteration or disorder, and route and frequency of administration. In general, the therapeutically effective amount of the compound of formula (I) to be administered will depend, among other factors, on the individual who is to be treated, the severity of the disease suffered by the individual, the selected form of administration, etc. For this reason, the doses mentioned in this invention must be considered solely as guides for the skilled person, who must adjust the doses according to the aforementioned variables. However, a compound of formula (I) may be administered one or more times a day, for example 1, 2, 3 or 4 times a day, in a typical total daily quantity comprised between 0.1 and 1,000 mg/kg body weight/day, preferably 10 mg/kg body mass/day.

The compound of the invention is compatible for use in protocols wherein the compounds of formula (I) or their mixtures are used alone or in combination with other treatments or medical procedures, such as radiotherapy or immunotherapy.

All individual features (e.g. preferred features) mentioned herein may be taken in isolation or in combination with any other feature (including a preferred feature) mentioned herein (hence, preferred features may be taken in conjunction with other preferred features, or independently of them).

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following examples and figures are provided for illustrative purposes and are not intended to limit the scope of this invention.

### EXAMPLES

### A. General methods for the synthesis of the compounds of the invention

Abbreviations: Herein after, the term "DAD" means diode-array detector, "DCM" means dichloromethane, "DIPEA" means diisopropylethylamine, "DME" means 1,2-dimethoxyethane, "DMF" means dimethylformamide, "eq" means equivalents, "EtOAc" means ethyl acetate, "h" means hours, "min" means minutes, "HPLC" means high performance liquid chromatography, "MeOH" means methanol, "mw" means microwave, "nBuOH" means n-butanol, "NMR" means nuclear magnetic resonance, "Pd(PPh₃)₄" means tetrakis(triphenylphosphine)-palladium, "THF" means tetrahydrofuran, "CHCl₃" means chloroform, "PdCl₂dppf" means 1,1'-bis(diphenylphosphino)ferrocene-palladium(II)dichloride dichloromethane complex, "AcCN" means acetonitrile, "Na₂SO₄" means sodium sulphate, "rt" means room temperature, "c-Hex" means cyclohexane, "CDCl₃" means deuterated chloroform, "DMSO" means dimethylsulfoxide, "NaHCO₃" means sodium bicarbonate, "H₂O" means water, "Hept" means heptane, "NaCl" means sodium chloride, "NH₄Cl" means ammonium chloride, "Na₂S₂O₃" means sodium thiosulfate, "EtOH" means ethanol, "AcOH" means acetic acid, "KOH" means potassium hydroxide, "Na₂CO₃" means sodium carbonate, "aq" means aqueous, "AlMe₃" means trimethylaluminium. "Rt": Retention time, "EDA" means ethylendiethylamine.

General Procedure: NMR spectra were recorded in a Bruker Avance II 300 spectrometer and Bruker Avance II 700 spectrometer fitted with 5 mm QXI 700 S4 inverse phase, Z-gradient unit and variable temperature controller. The HPLC measurements were performed using a HP 1100 from Agilent Technologies comprising a pump (binary) with degasser, an autosampler, a column oven, a diode-array detector (DAD) and a column as specified in the respective methods below. Flow from the column was split to a MS spectrometer. The MS detector was configured with an electrospray ionization source or API/APCI. Nitrogen was used as the nebulizer gas. Data acquisition was performed with ChemStation LC/MSD quad, software. Method: Reversed phase HPLC was carried out on a Gemini-NX C18 (100 x 2.0 mm; 5um). Solvent A: water with 0.1% formic acid; Solvent B: acetonitrile with 0.1% formic acid. Gradient: 5% to 100% of B within 5 or 8 min at 50 ºC, DAD. "Found mass" refers to the most abundant isotope detected in the HPLC-MS.

### Example 1: Synthesis of 8-(Piperidin-4-ylamino)-6-pyridin-4-yl-imidazo[1,2-a]pyrazine-2-carboxylic acid amide (1)

A suspension of Intermediate **III** (20 mg, 0.057 mmol) in NH₃ (7N in MeOH, 2 mL) was heated in a pressure tube at 90 ºC for 2 days. A precipitate appears which was filtered off to obtain the final compound. The compound was purified by column chromatography on silica gel using a solvent gradient 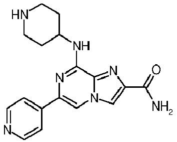 from 0% to 40% of EtOAc in cHex to afford final compound 1 (white solid, 10 mg, 52%). LCMS (ESI): Rt = 0.38 min, m/z = 338.10 [M+ H]⁺. ¹H NMR (300 MHz, DMSO-d6) δ ppm 8.68 - 8.61 (m, 3H), 8.28 (s, 1H), 7.92 (dd, *J* = 4.6, 1.5 Hz, 2H), 7.57 (d, *J* = 6.7 Hz, 2H), 7.37 (d, *J* = 7.7 Hz, 1H), 4.21 (s, 1H), 3.01 (d, *J* = 12.0 Hz, 3H), 2.63 (dd, *J* = 12.0, 10.0 Hz, 2H), 1.95 (d, *J* = 13.2 Hz, 2H), 1.59 - 1.45 (m, 2H).

### Example 2: Synthesis of 8-[(Piperidin-4-ylmethyl)-amino]-6-pyridin-4-yl-imidazo[1,2-a]pyrazine-2-carboxylic acid amide (2)

A suspension of Intermediate **VI** (20 mg, 0.053 mmol) in NH₃ (7N in MeOH, 2 mL) was heated in a pressure tube at 90 ºC for 2 days. The reaction mixture was evaporated under vacuum and the residue was purified by column chromatography on silica gel using a solvent gradient from 0% to 20% of MeOH 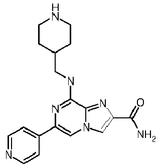 in DCM to afford final compound **2** (6 mg, 33%). LCMS (ESI): Rt = 0.40 min, m/z = 352.20 [M+ H]⁺. ¹H NMR (300 MHz, DMSO-d6) δ ppm 8.70 - 8.58 (m, 3H), 8.28 (s, 1H), 7.93 (d, *J* = 6.1 Hz, 2H), 7.69 (t, *J* = 5.6 Hz, 1H), 7.56 (s, 1H), 7.50 (s, 1H), 3.48 (t, *J* = 6.2 Hz, 2H), 2.91 (d, *J* = 11.5 Hz, 2H), 2.58 - 2.55 (m, 1H), 2.38 (d, *J* = 10.5 Hz, 1H), 1.89 - 1.77 (m, 1H), 1.66 (d, *J* = 12.5 Hz, 2H), 1.19 - 1.04 (m, 2H).

### Example 3: Synthesis of 6-(2-Chloro-pyridin-4-yl)-8-[(piperidin-4-ylmethyl)-amino]-imidazo[1,2-a]pyrazine-2-carboxylic acid amide (3)

TFA (0.18 mL, 2.346 mmol) was added to a mixture of Intermediate **VIII** (57 mg, 0.117 mmol) in DCM (3 mL) at rt. The reaction mixture was stirred at rt for 2 h. The mixture was evaporated under vacuum and the residue was purified by column chromatography on SCX-2 cartridge using a solvent gradient from 0% to 20% of NH₃ (7N in MeOH) in MeOH to render final compound **3** (white 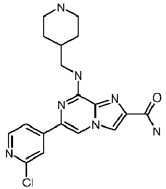 solid, 42 mg, 95%). LCMS (ESI): Rt = 2.49 min, m/z = 386.10 [M+ H]⁺. ¹H NMR (300 MHz, DMSO-*d6)* δ ppm 8.75 (s, 1H), 8.50 (d, *J* = 5.5 Hz, 1H), 8.27 (s, 1H), 8.03 (d, *J* = 0.9 Hz, 1H), 7.97 (dd, *J* = 5.3, 1.5 Hz, 1H), 7.81 (t, *J* = 5.8 Hz, 1H), 7.59 (s, 1H), 7.53 (s, 1H), 3.50 - 3.45 (m, 3H), 2.94 (d, *J* = 12.0 Hz, 2H), 2.41 (dd, *J* = 11.3, 1.4 Hz, 2H), 1.94 - 1.78 (m, 1H), 1.67 (d, *J* = 10.8 Hz, 2H), 1.15 (qd, *J* = 12.3, 3.8 Hz, 2H).

### Example 4: Synthesis of 8-[(Piperidin-4-ylmethyl)-amino]-6-pyridin-4-yl-imidazo[1,2-a]pyrazine-2-carboxylic acid methylamide (4)

TFA (0.22 mL, 2.878 mmol) was added to a mixture of Intermediate **IX** (67 mg, 0.144 mmol) in DCM (3 mL) at rt. The reaction mixture was stirred at rt for 1 h and 15 min. The mixture was evaporated under vacuum. The crude was purified by column chromatography on SCX-2 cartridge using a solvent gradient from 0% to 20% of NH₃ (7N in MeOH) in MeOH rendering final compound **4** (white 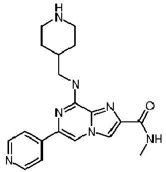 solid, 50 mg, 95%). LCMS (ESI): Rt = 0.42 min, m/z = 366.30 [M+ H]⁺. ¹H NMR (300 MHz, DMSO-*d6)* δ ppm 8.70 - 8.62 (m, 3H), 8.28 (s, 1H), 8.12 (q, *J* = 4.6 Hz, 1H), 7.93 (dd, *J* = 4.6, 1.6 Hz, 2H), 7.58 (t, *J* = 5.9 Hz, 1H), 3.50 (t, *J* = 6.3 Hz, 2H), 2.96 (d, *J* = 11.7 Hz, 2H), 2.83 (d, *J* = 4.8 Hz, 3H), 2.43 (d, *J* = 11.9 Hz, 3H), 1.93 - 1.77 (m, 1H), 1.69 (d, *J* = 11.9 Hz, 2H), 1.15 (dt, *J* = 15.5, 6.3 Hz, 2H).

### Example 5: Synthesis of 8-[(Piperidin-4-ylmethyl)-amino]-6-pyridin-4-yl-imidazo[1,2-a]pyrazine-2-carboxylic acid (2-methoxy-ethyl)-amide (5)

TFA (0.24 mL, 3.140 mmol) was added to a mixture of Intermediate **X** (80 mg, 0.157 mmol) in DCM (3 mL) at rt. The mixture was stirred at rt for 1 h and 15 min. The reaction mixture was evaporated under vacuum. The crude product was purified by column chromatography on Isolute SCX-2 cartridge using a solvent gradient from 0% to 20% of NH₃ (7N in MeOH) in MeOH rendering final 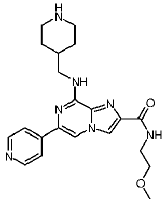 compound **5** (off-white solid, 60 mg, 93%). LCMS (ESI): Rt = 0.41 min, m/z = 410.20 [M+ H]⁺. ¹H NMR (300 MHz, DMSO-d6) δ ppm 8.66 (dd, *J* = 4.6, 1.5 Hz, 2H), 8.63 (s, 1H), 8.30 (s, 1H), 8.01 (s, 1H), 7.93 (dd, *J* = 4.6, 1.6 Hz, 2H), 7.81 (t, *J* = 5.8 Hz, 1H), 3.48 (t, *J* = 3.0 Hz, 7H), 3.29 (s, 3H), 2.96 (d, *J* = 11.7 Hz, 2H), 2.47 - 2.37 (m, 1H), 1.95 - 1.76 (m, 1H), 1.69 (d, *J* = 11.3 Hz, 2H), 1.16 (ddd, J= 15.5, 12.4, 3.9 Hz, 2H).

### Example 6: Synthesis of 6-(2-Chloro-pyridin-4-yl)-8-[(4-cyano-piperidin-4-ylmethyl)-amino]-imidazo[1,2-a]pyrazine-2-carboxylic acid amide (6)

TFA (0.10 mL, 1.252 mmol) was added to a mixture of Intermediate **XIII** (32 mg, 0.053 mmol) in DCM (2 mL) at rt. The reaction mixture was stirred at rt for 3 h, and then evaporated under vacuum. The crude product was purified by column chromatography on Isolute SCX-2 cartridge using a solvent gradient from 0% to 20% of NH₃ (7N in MeOH) in MeOH to give final product **6** (off-white solid, 24 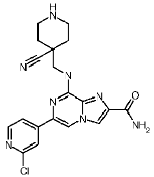 mg, 93%). LCMS (ESI): Rt = 0.71 min, m/z = 411.10 [M+ H]⁺. ¹H NMR (300 MHz, DMSO-d6) δ ppm 8.83 (s, 1H), 8.51 (d, *J* = 5.2 Hz, 1H), 8.32 (s, 1H), 8.07 (d, *J* = 0.8 Hz, 1H), 8.01 (dd, *J* = 5.2, 1.5 Hz, 2H), 7.61 (s, 2H), 3.91 (d, *J* = 6.5 Hz, 2H), 2.97 (d, *J* = 13.0 Hz, 2H), 2.66 (dt, *J* = 11.3, 1.6 Hz, 2H), 2.62 - 2.56 (m, 1H), 1.89 (d, *J* = 13.5 Hz, 2H), 1.61 (td, *J* = 13.0, 3.6 Hz, 2H).

### Example 7: Synthesis of 3-Methyl-8-[(piperidin-4-ylmethyl)-amino]-6-pyridin-4-yl-imidazo[1,2-a]pyrazine-2-carboxylic acid methylamide (7)

TFA (0.09 mL, 1.209 mmol) was added to a mixture of Intermediate **XVII** (29 mg, 0.060 mmol) in DCM (1.20 mL) at rt. The reaction was stirred at rt for 1 h and 30 min. The solvent was evaporated under vacuum. The crude was purified by column chromatography on Isolute SCX-2 cartridge using a solvent gradient from 0% to 20% of NH₃ (7N in MeOH) in MeOH to give the desired product **7** 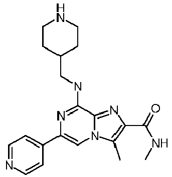 (white solid, 17 mg, 74%). LCMS (ESI): Rt = 0.36 min, m/z = 380.20 [M+ H]⁺. ¹H NMR (300 MHz, CDCl₃) δ ppm 8.68 - 8.57 (m, 2H), 7.77 (dd, *J* = 4.6, 1.6 Hz, 2H), 7.69 (s, 1H), 7.27 (s, 1H), 6.02 (t, *J* = 6.1 Hz, 1H), 3.58 (t, *J* = 6.1 Hz, 2H), 3.19 (d, *J* = 12.7 Hz, 2H), 2.95 (d, *J* = 4.3 Hz, 3H), 2.77 (s, 3H), 2.65 (t, *J* = 11.5 Hz, 2H), 1.94 - 1.72 (m, 3H), 1.42 (dd, *J* = 22.0, 12.0 Hz, 2H).

### Example 8: Synthesis of 8-[(4-Cyano-piperidin-4-ylmethyl)-amino]-6-pyridin-4-yl-imidazo[1,2-a]pyrazine-2-carboxylic acid amide (8)

TFA (0.16 mL, 2.098 mmol) was added to a mixture of Intermediate **XIX** (50 mg, 0.105 mmol) in DCM (4 mL) at rt. The mixture was stirred at rt for 3 h. The mixture was evaporated under vacuum and the crude was purified by column chromatography on Isolute SCX-2 cartridge using a solvent gradient from 0% to 20% of NH₃ (7N in MeOH) in MeOH to render final compound **8** (off-white solid, 36 mg, 91%). LCMS (ESI): Rt = 0.35 min, m/z = 377.10 [M+ 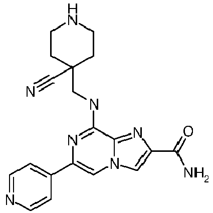 H]⁺. ¹H NMR (300 MHz, DMSO-*d6*) δ ppm 8.73 (s, 1H), 8.67 (dd, *J* = 4.6, 1.6 Hz, 2H), 8.33 (s, 1H), 7.97 (dd, *J* = 4.6, 1.6 Hz, 2H), 7.92 (d, J= 6.7 Hz, 1H), 7.59 (s, 2H), 3.91 (d, *J* = 6.7 Hz, 2H), 2.95 (d, *J* = 12.7 Hz, 2H), 2.63 (t, *J* = 11.5 Hz, 2H), 1.87 (d, *J* = 13.5 Hz, 2H), 1.61 (td, *J* = 13.0, 3.7 Hz, 2H).

### Example 9: Synthesis of 3-Methyl-8-[(piperidin-4-ylmethyl)-amino]-6-pyridin-4-yl-imidazo[1,2-a]pyrazine-2-carboxylic acid amide (9)

TFA (0.09 mL, 1.203 mmol) was added to a mixture of Intermediate **XX** (28 mg, 0.060 mmol) in DCM (1.20 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired final 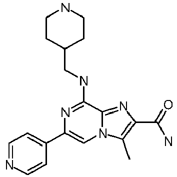 product **9** (white solid, 16 mg, 73%). LCMS (ESI): Rt = 0.38 min, m/z = 366.10 [M+ H]⁺. ¹H NMR (300 MHz, CDCl₃) δ ppm 8.71 (dd, *J* = 4.5, 1.6 Hz, 2H), 7.87 (dd, *J* = 4.5, 1.6 Hz, 2H), 7.76 (s, 1H), 7.17 (s, 1H), 6.11 (t, *J* = 6.1 Hz, 1H), 5.58 (s, 1H), 3.66 (t, *J* = 6.2 Hz, 2H), 3.20 - 3.10 (m, 2H), 2.85 (s, 3H), 2.65 (td, *J* = 12.1, 2.4 Hz, 2H), 1.86 (dd, *J* = 17.0, 3.7 Hz, 4H), 1.43 - 1.24 (m, 2H).

### Example 10: Synthesis of 3-Methyl-8-[(piperidin-4-ylmethyl)-amino]-6-pyridin-4-yl-imidazo[1,2-a]pyrazine-2-carboxylic acid (2-methoxy-ethyl)-amide (10)

TFA (0.08 mL, 1.031 mmol) was added to a mixture of Intermediate **XXI** (27 mg, 0.052 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **10** (pale yellow solid, 16 mg, 73%). LCMS (ESI): Rt = 0.370 & 1.538 min, m/z = 424.20 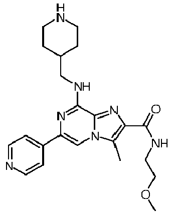 [M+ H]⁺. ¹H NMR (300 MHz, CDCl₃) δ ppm 8.70 (dd, *J* = 4.6, 1.6 Hz, 2H), 7.86 (dd, *J* = 4.6, 1.6 Hz, 2H), 7.75 (s, 1H), 7.64 (t, *J* = 5.5 Hz, 1H), 6.12 (t, *J* = 6.1 Hz, 1H), 3.72 - 3.57 (m, 6H), 3.43 (s, 3H), 3.23 - 3.13 (m, 2H), 2.85 (s, 3H), 2.67 (td, *J* = 12.2, 2.1 Hz, 3H), 1.89 (t, *J* = 10.0 Hz, 2H), 1.48 - 1.28 (m, 2H).

### Example 11: Synthesis of 6-(2-Chloro-pyridin-4-yl)-3-methyl-8-[(piperidin-4-ylmethyl)-amino]-imidazo[1,2-a]pyrazine-2-carboxylic acid methylamide (11)

TFA (0.09 mL, 1.128 mmol) was added to a mixture of Intermediate **XXIII** (29 mg, 0.056 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the final product **11** (white solid, 15 mg, 64%). LCMS (ESI): Rt = 2.89 min, m/z = 414.10 [M+ H]⁺. ¹H NMR (300 MHz, CDCl₃) δ ppm 8.46 (d, *J* = 5.2 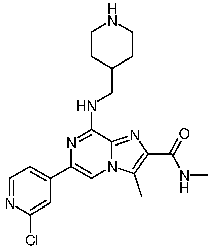 Hz, 1H), 7.90 (d, *J* = 0.9 Hz, 1H), 7.78 - 7.73 (m, 2H), 7.33 (d, *J* = 4.4 Hz, 1H), 6.08 (t, *J* = 6.1 Hz, 1H), 3.64 (t, *J* = 6.1 Hz, 2H), 3.20 (d, *J* = 12.1 Hz, 2H), 3.03 (d, *J* = 4.6 Hz, 3H), 2.86 (s, 3H), 2.75 - 2.62 (m, 2H), 2.01 - 1.79 (m, 3H), 1.41 (qd, *J* = 12.3, 3.5 Hz, 2H).

### Example 12: Synthesis of 6-(2-Chloro-pyridin-4-yl)-3-methyl-8-[(piperidin-4-ylmethyl)-amino]-imidazo[1,2-a]pyrazine-2-carboxylic acid amide (12)

TFA (0.09 mL, 1.120 mmol) was added to a mixture of Intermediate **XXIV** (28 mg, 0.056 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the final compound which was 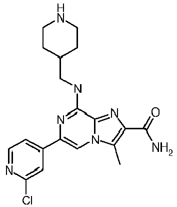 purified by preparative HPLC rendering product **12** (white solid, 20 mg, 89%). LCMS (ESI): Rt = 2.79 min, m/z = 400.10 [M+ H]⁺. ¹H NMR (300 MHz, MeOD) δ ppm 8.29 (d, *J* = 5.3 Hz, 1H), 8.18 (s, 1H), 8.03 (s, 1H), 7.93 (d, *J* = 5.3 Hz, 1H), 3.58 (d, *J* = 6.4 Hz, 2H), 3.33 (d, *J* = 12.4 Hz, 2H), 2.90 (t, *J* = 11.8 Hz, 2H), 2.70 (s, 3H), 2.08 (s, 1H), 1.99 (d, *J* = 14.6 Hz, 2H), 1.48 (dd, *J* = 23.8, 11.7 Hz, 2H).

### Example 13: Synthesis of 6-(2-Chloro-pyridin-4-yl)-3-methyl-8-[(piperidin-4-ylmethyl)-amino]-imidazo[1,2-a]pyrazine-2-carboxylic acid (2-methoxy-ethyl)-amide (13)

TFA (0.13 mL, 1.684 mmol) was added to a mixture of Intermediate **XXV** (47 mg, 0.084 mmol) in DCM (2 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the final compound **13** (white solid, 29 mg, 75%). LCMS (ESI): Rt = 3.04 min, m/z = 458.10 [M+ H]⁺. ¹H NMR (300 MHz, MeOD) δ ppm 8.37 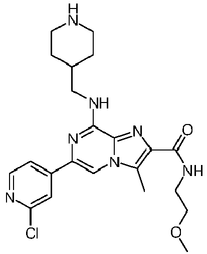 (d, *J* = 5.2 Hz, 1H), 8.23 (s, 1H), 8.09 (s, 1H), 7.99 (d, *J* = 5.0 Hz, 1H), 3.65 (d, *J* = 5.7 Hz, 2H), 3.58 (s, 4H), 3.50 - 3.33 (m, 5H), 3.07 - 2.88 (m, 2H), 2.78 (s, 3H), 2.24 - 2.13 (m, 1H), 2.05 (dd, *J* = 17.5, 9.7 Hz, 2H), 1.67 - 1.47 (m, 2H).

### Example 14: Synthesis of 8-[(4-Fluoro-piperidin-4-ylmethyl)-amino]-3-methyl-6-pyridin-4-yl-imidazo[1,2-a]pyrazine-2-carboxylic acid methylamide (14)

TFA (0.30 mL, 3.919 mmol) was added to a mixture of Intermediate **XXVIII** (39 mg, 0.078 mmol) in DCM (1.60 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the final 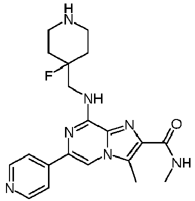 compound **14** (beige solid, 31 mg, 99%). LCMS (ESI): Rt = 0.75 min, m/z = 398.10 [M+ H]⁺. ¹H NMR (300 MHz, CDCl₃) δ ppm 8.69 (t, *J* = 5.5 Hz, 2H), 7.87 - 7.80 (m, 2H), 7.80 - 7.76 (m, 1H), 7.42 - 7.32 (m, 1H), 6.25 (t, *J* = 5.0 Hz, 1H), 3.98 (dt, *J* = 21.3, 5.8 Hz, 2H), 3.02 (t, *J* = 6.3 Hz, 6H), 2.86 (s, 3H), 2.62 - 2.29 (m, 2H), 2.04 - 1.71 (m, 4H).

### Example 15: Synthesis of 8-[(4-Fluoro-piperidin-4-ylmethyl)-amino]-6-pyridin-4-yl-imidazo[1,2-a]pyrazine-2-carboxylic acid amide (15)

TFA (0.06 mL, 0.767 mmol) was added to a mixture of Intermediate **XXXI** (18 mg, 0.038 mmol) in DCM (2 mL) at rt. The reaction was stirred at rt for 3 h. The mixture was evaporated under vacuum and the residue was purified by column chromatography on Isolute SCX-2 cartridge using a solvent gradient from 0% to 20% of NH₃ (7N in MeOH) in MeOH to render final product **15** (white solid, 4 mg, 28%). LCMS (ESI): Rt = 0.39 min, m/z = 370.00 [M+ H]⁺. 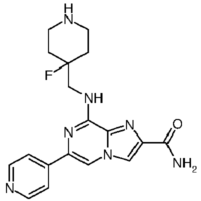 ¹H NMR (300 MHz, DMSO-d6) δ ppm 8.70 (s, 1H), 8.66 (dd, *J* = 4.7, 1.3 Hz, 2H), 8.32 (s, 1H), 7.95 (dd, *J* = 4.7, 1.4 Hz, 2H), 7.67 (s, 1H), 7.62 - 7.48 (m, 2H), 3.90 (dd, *J* = 20.0, 5.9 Hz, 2H), 2.95 - 2.82 (m, 2H), 2.82 - 2.67 (m, 3H), 1.86 - 1.66 (m, 4H).

### Example 16: Synthesis of 8-[(4-Fluoro-piperidin-4-ylmethyl)-amino]-3-methyl-6-pyridin-4-yl-imidazo[1,2-a]pyrazine-2-carboxylic acid amide (16)

TFA (0.30 mL, 3.929 mmol) was added to a mixture of Intermediate **XXXII** (38 mg, 0.079 mmol) in DCM (1.60 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the final compound **16** (pale 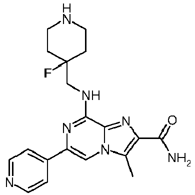 yellow solid, 18 mg, 60%). LCMS (ESI): Rt = 0.37 min, m/z = 384.00 [M+ H]⁺. ¹H NMR (300 MHz, CDCl₃) δ ppm 8.70 (dd, *J* = 4.6, 1.6 Hz, 2H), 7.83 (dd, *J* = 4.6, 1.6 Hz, 2H), 7.78 (s, 1H), 7.21 (s, 1H), 6.30 (t, *J* = 6.2 Hz, 1H), 5.63 (s, 1H), 3.99 (dd, *J* = 21.5, 6.3 Hz, 2H), 2.98 (dd, *J* = 7.8, 3.1 Hz, 4H), 2.84 (d, *J* = 7.6 Hz, 3H), 1.98 (d, *J* = 13.1 Hz, 2H), 1.85 (dd, *J* = 14.8, 7.0 Hz, 1H), 1.79 - 1.66 (m, 1H).

### Example 17: Synthesis of 8-[(4-Cyano-piperidin-4-ylmethyl)-amino]-3-methyl-6-pyridin-4-yl-imidazo[1,2-a]pyrazine-2-carboxylic acid methylamide (17)

TFA (0.18 mL, 2.378 mmol) was added to a mixture of Intermediate **XXXV** (24 mg, 0.048 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **17** (white solid, 18 mg, 94%). LCMS (ESI): Rt = 0.37 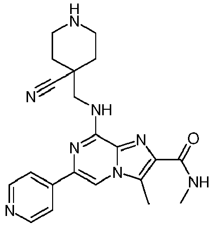 & 0.67 min, m/z = 405.10 [M+ H]⁺. ¹H NMR (300 MHz, MeOD) δ ppm 8.54 (d, *J* = 5.8 Hz, 2H), 8.16 (s, 1H), 8.05 (d, *J* = 6.1 Hz, 2H), 3.99 (s, 2H), 3.10 (d, *J* = 13.0 Hz, 2H), 2.95 (s, 3H), 2.92 - 2.81 (m, 2H), 2.77 (s, 3H), 2.05 (d, *J* = 13.7 Hz, 2H), 1.77 (td, *J* = 13.4, 4.0 Hz, 2H).

### Example 18: Synthesis of 8-[(4-Cyano-piperidin-4-ylmethyl)-amino]-3-methyl-6-pyridin-4-yl-imidazo[1,2-a]pyrazine-2-carboxylic acid (2-methoxy-ethyl)-amide (18)

TFA (0.25 mL, 3.281 mmol) was added to a mixture of Intermediate **XXXVI** (36 mg, 0.066 mmol) in DCM (1.31 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **18** (pale yellow solid, 25 mg, 85%). LCMS (ESI): Rt = 0.37 & 1.14 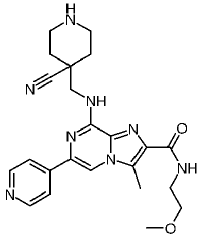 min, m/z = 449.10 [M+ H]⁺. ¹H NMR (300 MHz, MeOD) δ ppm 8.55 (d, *J* = 6.2 Hz, 2H), 8.19 (s, 1H), 8.06 (dd, *J* = 4.9, 1.4 Hz, 2H), 3.97 (s, 2H), 3.60 (s, 4H), 3.41 (s, 3H), 3.09 (d, *J* = 13.0 Hz, 2H), 2.86 (td, *J* = 12.9, 1.9 Hz, 2H), 2.78 (s, 3H), 2.04 (d, *J* = 13.4 Hz, 2H), 1.76 (td, *J* = 13.2, 4.0 Hz, 2H).

### Example 19: Synthesis of 8-[(4-Cyano-piperidin-4-ylmethyl)-amino]-3-methyl-6-pyridin-4-yl-imidazo[1,2-a]pyrazine-2-carboxylic acid amide (19)

TFA (0.19 mL, 2.446 mmol) was added to a mixture of Intermediate **XXXVII** (24 mg, 0.049 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **19** (cream solid, 15 mg, 79%). LCMS (ESI): Rt = 0.36 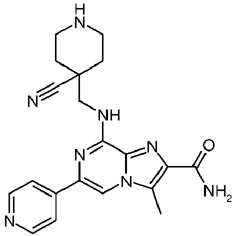 min, m/z = 391.10 [M+ H]⁺. ¹H NMR (300 MHz, MeOD) δ ppm 8.59 (dd, *J* = 4.7, 1.5 Hz, 2H), 8.29 (s, 1H), 8.13 (dd, *J* = 4.7, 1.6 Hz, 2H), 4.02 (s, 2H), 3.11 (d, *J* = 12.9 Hz, 2H), 2.90 (dd, *J* = 12.6, 2.1 Hz, 2H), 2.83 (s, 3H), 2.06 (d, *J* = 13.3 Hz, 2H), 1.79 (td, *J* = 13.6, 4.2 Hz, 2H).

### Example 20: Synthesis of 6-(2-Chloro-pyridin-4-yl)-8-[(4-fluoro-piperidin-4-ylmethyl)-amino]-3-methyl-imidazo[1,2-a]pyrazine-2-carboxylic acid methylamide (20)

TFA (0.20 mL, 2.538 mmol) was added to a mixture of Intermediate **XXXIX** (27 mg, 0.051 mmol) in DCM (1 mL) at rt. The reaction was stirred at rt for 2 h. An additional amount of TFA (0.20 mL) was added to the reaction mixture, and it was heated at rt for 2 more hours. The solvent was evaporated under vacuum. The crude was purified by column chromatography on Isolute SCX-2 cartridge using a solvent gradient from 0% to 20% of NH₃ (7N in MeOH) in MeOH and then by preparative HPLC 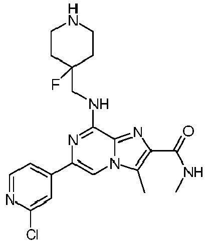 to give the desired product **20** as formic acid salt (pale yellow solid, 17 mg, 78%). LCMS (ESI): Rt = 2.92 min, m/z = 432.00 [M+ H]⁺. ¹H NMR (300 MHz, DMSO-d6) δ ppm 8.59 (s, 1H), 8.48 (d, *J* = 5.1 Hz, 1H), 8.34 (brs, 1H), 8.20 (s, 1H), 8.13 (d, *J* = 4.0 Hz, 2H), 7.49 (t, *J* = 5.8 Hz, 1H), 3.95 (s, 2H), 3.08 (d, *J* = 8.7 Hz, 2H), 2.88 (t, *J* = 12.3 Hz, 2H), 2.81 (d, *J* = 4.7 Hz, 3H), 2.78 (s, 3H), 1.91 (d, *J* = 14.9 Hz, 4H).

### Example 21: Synthesis of 6-(2-Chloro-pyridin-4-yl)-8-[(4-cyano-piperidin-4-ylmethyl)-amino]-3-methyl-imidazo[1,2-a]pyrazine-2-carboxylic acid methylamide (21)

TFA (0.14 mL, 1.762 mmol) was added to a mixture of Intermediate **XLI** (19 mg, 0.035 mmol) in DCM (0.71 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the desired product **21** as formic acid salt (white solid, 14 mg, 91%). LCMS (ESI): Rt = 2.85 min, m/z = 439.10 [M+ H]⁺. ¹H NMR (300 MHz, DMSO-d6) δ ppm 8.61 (s, 1H), 8.49 (d, *J* 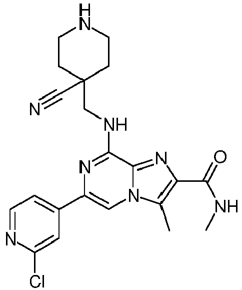 = 5.2 Hz, 1H), 8.28 (brs, 1H), 8.22 (d, *J* = 0.9 Hz, 1H), 8.14 (dd, *J* = 5.2, 1.4 Hz, 1H), 8.06 (q, *J* = 4.4 Hz, 1H), 7.80 (t, *J* = 6.5 Hz, 1H), 3.93 (d, *J* = 6.3 Hz, 2H), 3.09 (d, *J* = 12.8 Hz, 2H), 2.82 (d, *J* = 4.8 Hz, 3H), 2.78 (s, 3H), 2.74 - 2.66 (m, 2H), 1.96 (d, *J* = 13.3 Hz, 2H), 1.73 (t, *J* = 11.4 Hz, 2H).

### Example 22: Synthesis of 6-(2-Chloro-pyridin-4-yl)-8-[(4-fluoro-piperidin-4-ylmethyl)-amino]-3-methyl-imidazo[1,2-a]pyrazine-2-carboxylic acid (2-methoxy-ethyl)-amide (22)

TFA (0.19 mL, 2.517 mmol) was added to a mixture of Intermediate **XLII** (29 mg, 0.050 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the desired product **22** (beige solid, 12 mg, 50%). LCMS (ESI): Rt = 3.09 min, m/z = 476.10 [M+ H]⁺. ¹H NMR (300 MHz, 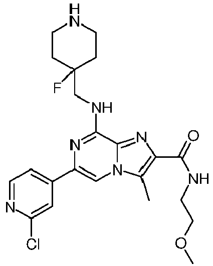 DMSO-*d6*) δ ppm 8.56 (s, 1H), 8.48 (d, *J* = 5.2 Hz, 1H), 8.19 (s, 1H), 8.11 (dd, *J* = 5.3, 1.4 Hz, 1H), 7.92 (t, *J* = 5.1 Hz, 2H), 5.67 (s, 1H), 4.14 (d, *J* = 4.8 Hz, 2H), 3.28 (s, 3H), 2.89 (t, *J* = 5.5 Hz, 3H), 2.78 (s, 4H), 2.09 (s, 2H).

### Example 23: Synthesis of 6-(2-Chloro-pyridin-4-yl)-8-[(4-cyano-piperidin-4-ylmethyl)-amino]-3-methyl-imidazo[1,2-a]pyrazine-2-carboxylic acid (2-methoxy-ethyl)-amide (23)

TFA (0.14 mL, 1.801 mmol) was added to a mixture of Intermediate **XLIII** (21 mg, 0.036 mmol) in DCM (0.72 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the desired product **23** as formic acid salt (beige solid, 7 mg, 40%). LCMS 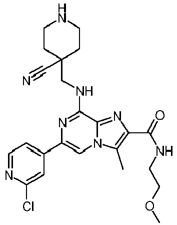 (ESI): Rt = 3.02 min, m/z = 483.10 [M+ H]⁺. ¹H NMR (300 MHz, DMSO-d6) δ ppm 8.61 (s, 1H), 8.49 (d, *J* = 5.1 Hz, 1H), 8.29 (s, 1H), 8.23 (s, 1H), 8.15 (d, *J* = 4.8 Hz, 1H), 8.05 (t, *J* = 6.3 Hz, 1H), 7.99 (d, *J* = 3.8 Hz, 1H), 3.91 (d, *J* = 6.1 Hz, 2H), 3.28 (s, 3H), 3.07 (d, *J* = 10.6 Hz, 2H), 2.79 (s, 3H), 2.76 - 2.63 (m, 2H), 1.95 (d, *J* = 12.7 Hz, 2H), 1.81 - 1.65 (m, 2H).

### Example 24: Synthesis of 6-(2-Chloro-pyridin-4-yl)-8-[(4-cyano-piperidin-4-ylmethyl)-amino]-3-methyl-imidazo[1,2-a]pyrazine-2-carboxylic acid amide (24)

TFA (0.14 mL, 1.809 mmol) was added to a mixture of Intermediate **XLIV** (19 mg, 0.036 mmol) in DCM (0.72 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the desired product **24** (beige solid, 15 mg, 98%). LCMS (ESI): Rt = 2.74 min, 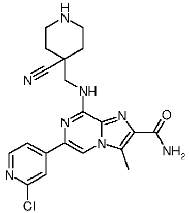 m/z = 425.10 [M+ H]⁺. ¹H NMR (300 MHz, DMSO-d6) δ ppm 8.60 (s, 1H), 8.49 (d, *J* = 5.3 Hz, 1H), 8.22 (d, *J* = 0.9 Hz, 1H), 8.14 (dd, *J* = 5.3, 1.5 Hz, 1H), 7.86 (t, *J* = 6.6 Hz, 1H), 7.51 (s, 1H), 7.45 (s, 1H), 3.89 (d, *J* = 6.5 Hz, 2H), 2.94 (d, *J* = 12.8 Hz, 2H), 2.78 (s, 3H), 2.62 (t, *J* = 11.5 Hz, 2H), 1.86 (d, *J* = 13.1 Hz, 2H), 1.59 (td, *J* = 12.9, 3.8 Hz, 2H).

### Example 25: Synthesis of 6-(2-Chloro-pyridin-4-yl)-8-[(4-fluoro-piperidin-4-ylmethyl)-amino]-3-methyl-imidazo[1,2-a]pyrazine-2-carboxylic acid amide (25)

TFA (0.19 mL, 2.510 mmol) was added to a mixture of Intermediate **XLV** (26 mg, 0.050 mmol) in DCM (1 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **25** (white solid, 19 mg, 91%). LCMS (ESI): Rt = 2.70 min, m/z = 418.10 [M+ H]⁺. ¹H NMR 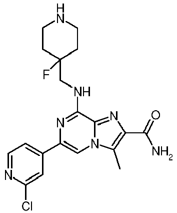 (300 MHz, MeOD) δ ppm 8.38 (d, *J* = 5.3 Hz, 1H), 8.25 (s, 1H), 8.09 (d, *J* = 0.9 Hz, 1H), 8.01 (dd, *J* = 5.3, 1.5 Hz, 1H), 3.95 (d, *J* = 20.6 Hz, 2H), 2.91 (dd, *J* = 9.3, 3.9 Hz, 4H), 2.79 (s, 3H), 1.98 - 1.68 (m, 4H).

### Example 26: Synthesis of 8-[(1-Propyl-piperidin-4-ylmethyl)-amino]-6-pyridin-4-yl-imidazo[1,2-a]pyrazine-2-carboxylic acid methylamide (26)

AcOH (0.001 mL, 0.010 mmol) was added to a mixture of final product **4** (37 mg, 0.101 mmol) in 1,2-DCE (2 mL) followed by the addition of propionaldehyde (0.011 mL, 0.152 mmol) and NaBH₃CN (10 mg, 0.152 mmol) at rt. The reaction was stirred at rt for 90 min. The reaction mixture was diluted with DCM and washed with a saturated aqueous solution of NaHCO₃. The organic layer was dried over Na₂SO₄, filtered and concentrated. The resulting residue was purified by column chromatography on silica gel using a solvent gradient from 0% to 20% of 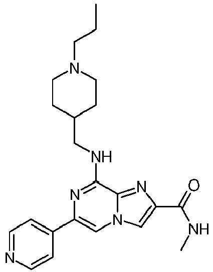 NH₃ (7N in MeOH) in EtOAc to afford desired product **26** (white solid, 13 mg, 31%). LCMS (ESI): Rt = 0.41 min, m/z = 408.10 [M+ H]⁺. ¹H NMR (300 MHz, DMSO-*d6*) δ ppm 8.75 - 8.68 (m, 3H), 8.34 (s, 1H), 8.19 (d, *J* = 4.9 Hz, 1H), 8.00 (dd, *J* = 4.6, 1.6 Hz, 2H), 7.67 (t, *J* = 6.0 Hz, 1H), 3.65 - 3.56 (m, 2H), 2.95 (d, *J* = 11.8 Hz, 2H), 2.89 (d, *J* = 4.8 Hz, 3H), 2.38 - 2.25 (m, 2H), 1.96 (t, *J* = 11.2 Hz, 2H), 1.79 (d, *J* = 11.5 Hz, 3H), 1.49 (dd, J = 14.9, 7.4 Hz, 2H), 1.39 - 1.26 (m, 2H), 0.90 (t, *J* = 7.4 Hz, 3H).

### Example 27: Synthesis of 6-(3-Fluoro-pyridin-4-yl)-8-[(piperidin-4-ylmethyl)-amino]-imidazo[1,2-a]pyrazine-2-carboxylic acid amide (27)

TFA (0.12 mL, 1.576 mmol) was added to a mixture of Intermediate **XLVII** (37 mg, 0.080 mmol) in DCM (2 mL) at rt. The mixture was stirred at rt for 3 h. The solvent was evaporated under vacuum. The crude was purified by column chromatography on Isolute SCX-2 cartridge using a solvent gradient from 0% to 20% of NH₃ (7N in MeOH) in MeOH to give the desired product **27** (white solid, 21 mg, 72%). LCMS (ESI): Rt = 0.55 min, m/z = 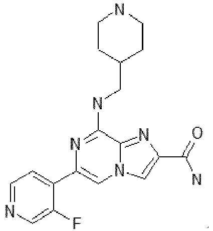 370.10 [M+ H]⁺. ¹H NMR (300 MHz, DMSO-d6) δ ppm 8.66 (d, *J* = 3.6 Hz, 1H), 8.56 (d, *J* = 5.1 Hz, 1H), 8.54 (s, 1H), 8.44 (s, 1H), 8.16 (dd, *J* = 6.9, 5.2 Hz, 1H), 7.75 (t, *J* = 6.2 Hz, 1H), 7.58 (s, 1H), 7.47 (s, 1H), 3.49 - 3.43 (m, 2H), 2.92 (d, *J* = 12.3 Hz, 2H), 2.41 (t, *J* = 12.2 Hz, 2H), 1.91 - 1.73 (m, 1H), 1.66 (d, *J* = 12.0 Hz, 2H), 1.21 - 1.02 (m, 2H).

### Example 28: Synthesis of 6-(3-Fluoro-pyridin-4-yl)-8-[(piperidin-4-ylmethyl)-amino]-imidazo[1,2-a]pyrazine-2-carboxylic acid methylamide (28)

TFA (0.18 mL, 2.316 mmol) was added to a mixture of Intermediate **XLVIII** (56 mg, 0.116 mmol) in DCM (3 mL) at rt. The reaction was stirred at rt for 2 h. The mixture was evaporated under vacuum. The crude was purified by column chromatography on Isolute SCX-2 cartridge using a solvent gradient from 0% to 20% of NH₃ (7N in MeOH) in MeOH to give the desired product 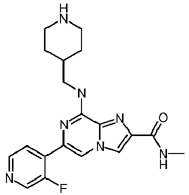 **28** (white solid, 31 mg, 70%). LCMS (ESI): Rt = 4.59 min, m/z = 384.10 [M+ H]⁺. ¹H NMR (300 MHz, DMSO-*d6*) δ ppm 8.66 (d, *J* = 3.6 Hz, 1H), 8.56 (dd, *J* = 5.1, 1.0 Hz, 1H), 8.54 (s, 1H), 8.44 (s, 1H), 8.16 (dd, *J* = 7.1, 5.1 Hz, 1H), 8.11 (d, *J* = 5.0 Hz, 1H), 7.65 (t, *J* = 5.5 Hz, 1H), 3.48 (dd, *J* = 7.5, 4.6 Hz, 2H), 2.96 (d, *J* = 11.7 Hz, 2H), 2.82 (d, *J* = 4.8 Hz, 3H), 2.48 - 2.42 (m, 2H), 1.93 - 1.76 (m, 1H), 1.68 (d, *J* = 12.5 Hz, 2H), 1.15 (ddd, *J* = 10.9, 9.0, 6.5 Hz, 2H).

### Example 29: Synthesis of 8-[(4-Cyano-piperidin-4-ylmethyl)-amino]-6-(3-fluoro-pyridin-4-yl)-imidazo[1,2-a]pyrazine-2-carboxylic acid amide (29)

TFA (0.17 mL, 2.244 mmol) was added to a mixture of Intermediate **L** (47 mg, 0.090 mmol) in DCM (2 mL) at rt. The reaction was stirred at rt for 3 h and 30 min. The solvent was evaporated under vacuum. The crude was purified by column chromatography on Isolute SCX-2 cartridge using a solvent gradient from 0% to 20% of NH₃ (7N in MeOH) in MeOH and then triturated from MeCN/H₂O to give the desired product **29** (white solid, 10 mg, 28%). LCMS 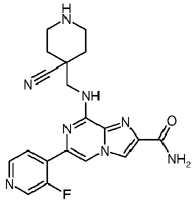 (ESI): Rt = 0.66 min, m/z = 395.20 [M+ H]⁺. ¹H NMR (300 MHz, DMSO-d6) δ ppm 8.68 (d, J= 3.5 Hz, 1H), 8.63 (s, 1H), 8.57 (d, *J* = 5.0 Hz, 1H), 8.49 (s, 1H), 8.23 (dd, *J* = 7.0, 5.1 Hz, 1H), 7.97 (t, *J* = 6.6 Hz, 1H), 7.60 (s, 1H), 7.56 (s, 1H), 3.89 (d, *J* = 6.6 Hz, 2H), 2.94 (d, *J* = 12.6 Hz, 2H), 2.66 - 2.56 (m, 2H), 1.87 (d, *J* = 13.3 Hz, 2H), 1.59 (td, *J* = 13.0, 3.9 Hz, 2H).

### Example 30: Synthesis of 6-(3-Chloro-pyridin-4-yl)-8-[(piperidin-4-ylmethyl)-amino]-imidazo[1,2-a]pyrazine-2-carboxylic acid amide (30)

TFA (0.16 mL, 2.058 mmol) was added to a mixture of Intermediate **LII** (40 mg, 0.082 mmol) in DCM (2 mL) at rt. The reaction was stirred at rt for 90 min. The mixture was evaporated under vacuum. The crude was purified by column chromatography on Isolute SCX-2 cartridge using a solvent gradient from 0% to 20% of NH₃ (7N in MeOH) in MeOH to render final product **30** (white solid, 14 mg, 44%). LCMS (ESI): Rt = 0.48 min, m/z = 386.10 [M+ H]⁺. ¹H NMR (300 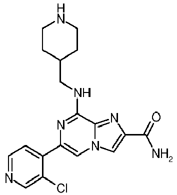 MHz, DMSO-*d6*) δ ppm 8.73 (s, 1H), 8.62 (d, *J* = 5.0 Hz, 1H), 8.38 (s, 1H), 8.34 (s, 1H), 7.77 (d, *J* = 5.0 Hz, 1H), 7.73 (d, *J* = 5.9 Hz, 1H), 7.57 (s, 1H), 7.48 (s, 1H), 2.94 (d, *J* = 12.2 Hz, 2H), ∼2.50 (m, 2H under DMSO signal), 1.92 - 1.77 (m, 1H), 1.64 (d, *J* = 11.7 Hz, 2H), 1.20 - 1.00 (m, 2H).

### Example 31: Synthesis of 6-(3-Chloro-pyridin-4-yl)-8-[(piperidin-4-ylmethyl)-amino]-imidazo[1,2-a]pyrazine-2-carboxylic acid methylamide (31)

TFA (0.11 mL, 1.400 mmol) was added to a mixture of Intermediate **LIII** (28 mg, 0.056 mmol) in DCM (1 mL) at rt. The reaction was stirred at rt for 90 min. The mixture was evaporated under vacuum, and the residue was purified by column chromatography on Isolute SCX-2 cartridge using a solvent gradient from 0% to 20% of NH₃ (7N in MeOH) in MeOH to yield final product **31** (white solid, 11 mg, 49%). LCMS (ESI): Rt = 0.59 min, m/z 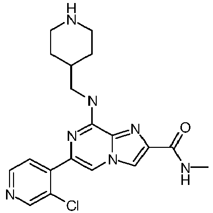 = 400.20 [M+ H]⁺. ¹H NMR (300 MHz, DMSO-d6) δ ppm 8.73 (s, 1H), 8.61 (d, *J* = 5.0 Hz, 1H), 8.37 (s, 1H), 8.33 (s, 1H), 8.15 - 8.07 (m, 1H), 7.76 (d, *J* = 5.0 Hz, 1H), 7.62 (t, *J* = 6.0 Hz, 1H), ∼3.35 (m, 2H under H₂O signal), 2.96 (d, *J* = 12.2 Hz, 2H), 2.82 (d, *J* = 4.8 Hz, 3H), ∼2.50 (m, 2H under DMSO signal), 1.92 - 1.77 (m, 1H), 1.65 (d, *J* = 12.5 Hz, 2H), 1.12 (qd, *J* = 12.9, 3.7 Hz, 2H).

### Example 32: Synthesis of 8-[(4-Cyano-piperidin-4-ylmethyl)-amino]-6-(3-fluoro-pyridin-4-yl)-imidazo[1,2-a]pyrazine-2-carboxylic acid methylamide (32)

TFA (0.15 mL, 1.972 mmol) was added to a mixture of Intermediate **LIV** (39 mg, 0.077 mmol) in DCM (2 mL) at rt. The reaction was stirred at rt for 3h. The mixture was evaporated under vacuum. The residue was purified by column chromatography on Isolute SCX-2 cartridge using a solvent gradient from 0% to 20% of NH₃ (7N in MeOH) in MeOH and then by preparative HPLC to render final product **32** as formic acid salt (white solid, 6 mg, 19%). 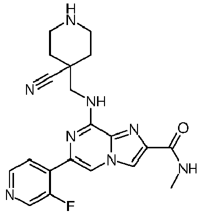 LCMS (ESI): Rt = 2.15 min, m/z = 409.10 [M+ H]⁺. ¹H NMR (300 MHz, DMSO-d6) δ ppm 8.68 (d, *J* = 3.5 Hz, 1H), 8.63 (s, 1H), 8.57 (d, *J* = 5.0 Hz, 1H), 8.49 (s, 1H), 8.21 (ddd, *J* = 15.5, 9.0, 4.0 Hz, 3H), 7.86 (t, *J* = 6.6 Hz, 1H), 3.92 (d, *J* = 6.5 Hz, 2H), 3.02 (d, *J* = 12.8 Hz, 2H), 2.83 (d, *J* = 4.8 Hz, 3H), 2.73 - 2.62 (m, 2H), 1.92 (d, *J* = 13.3 Hz, 2H), 1.66 (td, *J* = 13.1, 3.7 Hz, 2H).

### Example 33: Synthesis of 8-[(4-Fluoro-piperidin-4-ylmethyl)-amino]-6-(3-fluoro-pyridin-4-yl)-imidazo[1,2-a]pyrazine-2-carboxylic acid amide (33)

Amberlyst-^{®}15 (20 eq) was added to a mixture of Intermediate **LV** (120 mg, 0.246 mmol) in MeOH (5 mL) at rt. The reaction was stirred at rt for 24 h. Then, the resine was washed with MeOH and the product was cleaved with 7N NH₃ in MeOH. The solvent was evaporated under vacuum. The residue was purified by column chromatography on silica gel using a solvent gradient from 0% to 10% of NH₃ (7N in MeOH) in DCM to render final 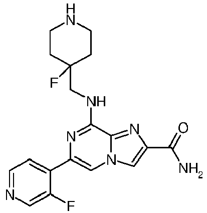 product **33** (white solid, 65 mg, 68%). LCMS (ESI): Rt = 3.93 min, m/z = 388.10 [M+ H]⁺. ¹H NMR (300 MHz, DMSO-*d6*) δ ppm 8.67 (d, *J* = 3.5 Hz, 1H), 8.60 (s, 1H), 8.57 (d, *J* = 5.0 Hz, 1H), 8.47 (s, 1H), 8.19 (dd, *J* = 7.1, 5.2 Hz, 1H), 7.64 (s, 1H), 7.55 (s, 1H), 7.48 (t, *J* = 6.5 Hz, 1H), 3.90 (d, *J* = 6.4 Hz, 1H), 3.83 (s, 1H), 2.72 (t, *J* = 9.6 Hz, 4H), 1.71 (d, *J* = 15.2 Hz, 4H).

### Example 34: Synthesis of 6-(3-Fluoro-pyridin-4-yl)-3-methyl-8-[(piperidin-4-ylmethyl)-amino]-imidazo[1,2-a]pyrazine-2-carboxylic acid methylamide (35)

TFA (0.49 mL, 6.331 mmol) was added to a mixture of Intermediate **LVIII** (63 mg, 0.127 mmol) in DCM (2.50 mL) at rt. The reaction was stirred at rt for 2 h. The solvent was evaporated under vacuum. The crude was purified by column chromatography on Isolute SCX-2 cartridge using a solvent gradient from 0% to 10% of NH₃ (7N in MeOH) in MeOH to give the desired product **35** (white solid, 11 mg, 22%). LCMS (ESI): Rt = 0.48 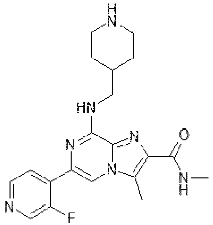 min, m/z = 398.40 [M+ H]⁺. ¹H NMR (300 MHz, CDCl₃) δ ppm 8.61 - 8.49 (m, 2H), 8.20 (dd, *J* = 6.9, 5.1 Hz, 1H), 8.06 (s, 1H), 7.34 (d, *J* = 4.2 Hz, 1H), 6.07 (t, *J* = 6.0 Hz, 1H), 3.66 (t, *J* = 6.2 Hz, 2H), 3.27 (dd, *J* = 13.8, 6.8 Hz, 2H), 3.04 (d, *J* = 4.8 Hz, 3H), 2.85 (s, 3H), 2.79 - 2.68 (m, 2H), 1.93 (d, *J* = 12.7 Hz, 3H), 1.52 (dd, *J* = 23.9, 12.1 Hz, 2H).

### Example 35: Synthesis of 6-(2-Methyl-pyridin-4-yl)-8-[(piperidin-4-ylmethyl)-amino]-imidazo[1,2-a]pyrazine-2-carboxylic acid amide (36)

TFA (0.12 mL, 1.504 mmol) was added to a mixture of Intermediate **LX** (28 mg, 0.060 mmol) in DCM (2 mL) at rt. The reaction was stirred at rt for 2 h. The mixture was evaporated under vacuum. The residue was purified by column chromatography on Isolute SCX-2 cartridge using a solvent gradient from 0% to 20% of NH₃ (7N in MeOH) in MeOH and then by preparative HPLC to render final product **36** as formic acid salt (white solid, 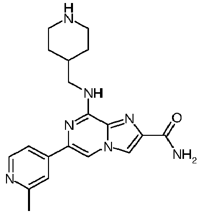 7 mg, 32%). LCMS (ESI): Rt = 0.45 min, m/z = 366.30 [M+ H]⁺. ¹H NMR (300 MHz, DMSO-d6) δ ppm 8.62 (s, 1H), 8.51 (d, *J* = 5.1 Hz, 1H), 8.39 (s, 1H), 8.29 (s, 1H), 7.87 (t, *J* = 6.0 Hz, 1H), 7.79 (s, 1H), 7.77 - 7.71 (m, 1H), 7.58 (s, 1H), 7.49 (s, 1H), ∼3.30 (m, 2H under H₂O signal), 3.18 (d, *J* = 11.9 Hz, 2H), 2.79 - 2.66 (m, 2H), 2.54 (s, 3H), 2.00 (s, 1H), 1.83 (d, *J* = 12.9 Hz, 2H), 1.37 (dd, *J* = 22.3, 11.0 Hz, 2H).

### Example 36: Synthesis of 8-(2-Piperidin-4-yl-ethylamino)-6-pyridin-4-yl-imidazo[1,2-a]pyrazine-2-carboxylic acid amide (37)

TFA (0.90 mL, 11.556 mmol) was added to a mixture of Intermediate **LXIII** (269 mg, 0.578 mmol) in DCM (12 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give the desired product **37** 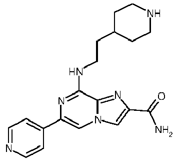 (pale yellow solid, 100 mg, 47%). LCMS (ESI): Rt = 0.37 & 0.47 min, m/z = 366.20 [M+ H]⁺. ¹H NMR (700 MHz, DMSO-d6) δ ppm 8.68 (dd, *J* = 4.7, 1.3 Hz, 2H), 8.67 (s, 1H), 8.31 (s, 1H), 7.96 (dd, *J* = 4.6, 1.4 Hz, 2H), 7.74 (s, 1H), 7.60 (s, 1H), 7.50 (s, 1H), 3.65 (d, *J* = 6.0 Hz, 2H), 2.93 (d, *J* = 11.1 Hz, 2H), 2.44 (t, *J* = 11.3 Hz, 2H), 1.72 (d, *J* = 11.8 Hz, 2H), 1.64 (d, *J* = 6.7 Hz, 2H), 1.48 (s, 1H), 1.09 (dd, *J* = 21.1, 10.9 Hz, 2H).

### Example 37: Synthesis of 8-(4-trans-Amino-cyclohexylamino)-6-pyridin-4-yl-imidazo[1,2-a]pyrazine-2-carboxylic acid amide (38)

TFA (0.96 mL, 12.491 mmol) was added to a mixture of Intermediate **LXVI** (282 mg, 0.625 mmol) in DCM (12 mL) at rt. The reaction was stirred at rt for 2 h. An additional amount of TFA (1 mL) was added to the reaction mixture, and it was stirred at rt for 2 h. The solvent was evaporated under 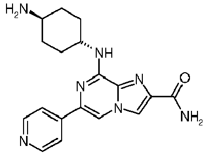 vacuum. The crude was purified by column chromatography on Isolute SCX-2 cartridge using a solvent gradient from 0% to 20% of NH₃ (7N in MeOH) in MeOH to give final product 38 (white solid, 80 mg, 37%). LCMS (ESI): Rt = 0.35 min, m/z = 352.20 [M+ H]⁺. ¹H NMR (700 MHz, DMSO-*d6)* δ ppm 8.67 (dd, *J* = 4.5, 1.5 Hz, 2H), 8.65 (s, 1H), 8.29 (s, 1H), 7.93 (dd, *J* = 4.6, 1.6 Hz, 2H), 7.56 (s, 2H), 7.33 (d, *J* = 8.0 Hz, 1H), 4.12 (s, 1H), 2.61 (tt, *J* = 11.4, 3.8 Hz, 1H), 2.02 (d, *J* = 11.0 Hz, 2H), 1.86 (d, *J* = 11.9 Hz, 2H), 1.55 - 1.45 (m, 2H), 1.25 (td, *J* = 13.3, 3.1 Hz, 2H).

### Example 38: Synthesis of 8-(cis-4-Amino-cyclohexylamino)-6-pyridin-4-yl-imidazo[1,2-a]pyrazine-2-carboxylic acid amide (39)

TFA (1.24 mL, 16.123 mmol) was added to a mixture of Intermediate **LXIX** (364 mg, 0.806 mmol) in DCM (16 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH to give final 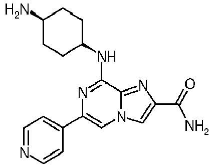 product **39** (yellow solid, 145 mg, 51%). LCMS (ESI): Rt = 0.37 min, m/z = 352.10 [M+ H]⁺. ¹H NMR (700 MHz, DMSO-d6) δ ppm 8.49 - 8.46 (m, 3H), 8.10 (s, 1H), 7.74 (dd, *J* = 4.5, 1.6 Hz, 2H), 7.55 (s, 1H), 7.36 (s, 1H), 6.77 (d, *J* = 7.2 Hz, 1H), 4.06 (s, 1H), 2.73 (s, 1H), 1.80 - 1.73 (m, 2H), 1.54 (ddd, *J* = 12.0, 7.5, 3.7 Hz, 2H), 1.47 (ddd, *J* = 12.7, 7.8, 3.6 Hz, 2H), 1.37 - 1.29 (m, 2H).

### Example 39: Synthesis of 8-(trans-4-Aminomethyl-cyclohexylamino)-6-pyridin-4-yl-imidazo[1,2-a]pyrazine-2-carboxylic acid amide (40)

TFA (0.40 mL, 5.198 mmol) was added to a mixture of Intermediate **LXXII** (121 mg, 0.260 mmol) in DCM (5 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by 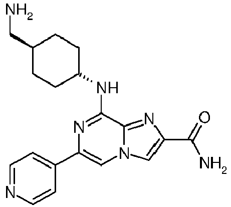 preparative HPLC to render final product **40** as formic acid salt (yellow solid, 60 mg, 63%). LCMS (ESI): Rt = 0.37 min, m/z = 366.20 [M+ H]⁺. ¹H NMR (700 MHz, DMSO-*d6)* δ ppm 8.67 (d, *J* = 7.2 Hz, 3H), 8.42 (s, 1H), 8.30 (s, 1H), 7.93 (d, *J* = 6.1 Hz, 2H), 7.56 (d, *J* = 19.0 Hz, 2H), 7.44 (d, *J* = 8.1 Hz, 1H), 4.15 (s, 2H), 2.68 (d, *J* = 6.7 Hz, 2H), 2.09 (d, *J* = 10.1 Hz, 2H), 1.89 (d, *J* = 12.2 Hz, 2H), 1.57 (s, 1H), 1.53 - 1.44 (m, 2H), 1.18 (dd, *J* = 23.9, 11.4 Hz, 2H).

### Example 40: Synthesis of 6-(2-Amino-pyridin-4-yl)-8-[(4-fluoro-piperidin-4-ylmethyl)-amino]-imidazo[1,2-a]pyrazine-2-carboxylic acid amide (41)

TFA (0.08 mL, 0.991 mmol) was added to a mixture of Intermediate **LXXIV** (16 mg, 0.033 mmol) in DCM (1 mL) at rt. The reaction was stirred at rt for 2 h. The mixture was evaporated under vacuum. The residue was purified by column chromatography on Isolute SCX-2 cartridge using a solvent gradient from 0% to 20% of NH₃ (7N in MeOH) in MeOH to render final product **41** (white solid, 4 mg, 31%). LCMS (ESI): Rt = 0.43 min, m/z = 385.30 [M+ H]⁺. 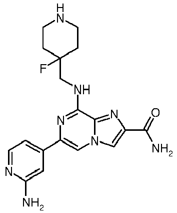 ¹H NMR (700 MHz, DMSO-*d6*) δ ppm 8.46 (s, 1H), 8.31 (d, *J* = 5.1 Hz, 1H), 7.96 (d, *J* = 5.4 Hz, 1H), 7.65 (s, 1H), 7.53 (s, 1H), 7.37 - 7.32 (m, 1H), 7.07 (s, 1H), 6.98 (dd, *J* = 5.4, 1.5 Hz, 1H), 6.03 (s, 2H), 3.87 (dd, *J* = 19.5, 6.1 Hz, 2H), 2.82 - 2.75 (m, 2H), 2.70 (t, *J* = 10.2 Hz, 2H), 1.78 - 1.70 (m, 3H), 1.66 (tt, *J* = 29.3, 10.0 Hz, 1H).

### Example 41: Synthesis of 8-[trans-(4-Amino-cyclohexylmethyl)-amino]-6-pyridin-4-yl-imidazo[1,2-a]pyrazine-2-carboxylic acid amide (42)

TFA (0.92 mL, 12.012 mmol) was added to a mixture of Intermediate **LXXVII** (282 mg, 0.601 mmol) in DCM (12 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC 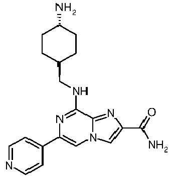 to give final product **42** as formic acid salt (pale yellow solid, 164 mg, 73%). LCMS (ESI): Rt = 0.37 min, m/z = 366.20 [M+ H]⁺. ¹H NMR (700 MHz, DMSO-d6) δ ppm 8.66 (dd, *J* = 4.6, 1.5 Hz, 2H), 8.65 (s, 1H), 8.44 (s, 1H), 8.30 (s, 1H), 7.93 (dd, *J* = 4.6, 1.5 Hz, 2H), 7.77 (t, *J* = 5.9 Hz, 1H), 7.58 (s, 1H), 7.49 (s, 1H), 3.50 - 3.46 (m, 4H), 2.88 (ddd, *J* = 11.5, 7.9, 3.9 Hz, 1H), 1.93 (d, *J* = 10.4 Hz, 2H), 1.85 (d, *J* = 12.0 Hz, 2H), 1.76 - 1.69 (m, 1H), 1.24 (qd, *J* = 12.6, 2.9 Hz, 2H), 1.14 - 1.06 (m, 2H).

### Example 42: Synthesis of 8-[(4-Hydroxy-piperidin-4-ylmethyl)-amino]-6-pyridin-4-yl-imidazo[1,2-a]pyrazine-2-carboxylic acid amide (44)

TFA (0.13 mL, 1.626 mmol) was added to a mixture of Intermediate **LXXXII** (38 mg, 0.081 mmol) in DCM (1.63 mL) at rt. The reaction was stirred at rt for 2 h. The solvent was evaporated under vacuum. The crude was purified by column chromatography on Isolute SCX-2 cartridge using a solvent gradient from 0% to 20% of NH₃ (7N in MeOH) in MeOH and then by preparative HPLC to give the desired final product 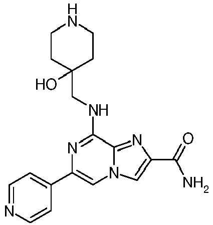 **44** as formic acid salt (pale yellow solid, 20 mg, 60%). LCMS (ESI): Rt = 0.33 min, m/z = 368.20 [M+ H]⁺. ¹H NMR (700 MHz, DMSO-d6) δ ppm 8.62 (s, 1H), 8.59 (dd, *J* = 4.6, 1.5 Hz, 2H), 8.35 (s, 1H), 8.25 (s, 1H), 7.88 (dd, *J* = 4.6, 1.5 Hz, 2H), 7.66 (s, 1H), 7.48 (s, 1H), 7.37 (t, *J* = 6.1 Hz, 1H), 3.64 (d, *J* = 5.7 Hz, 2H), 2.91 (ddd, J= 18.0, 14.3, 7.5 Hz, 4H), 1.74 - 1.65 (m, 2H), 1.58 (d, *J* = 13.5 Hz, 2H).

### Example 43: Synthesis of 8-[(4-Fluoro-piperidin-4-ylmethyl)-amino]-6-(1-methyl-1H-pyrazol-3-yl)-imidazo[1,2-a]pyrazine-2-carboxylic acid amide (45)

Amberlyst-^{®}15 (20 eq) was added to a mixture of Intermediate **LXXXIV** (30 mg, 0.060 mmol) in MeOH (2 mL) at rt. The reaction was stirred at rt for 24 h. Then, the resine was washed with MeOH and the product was cleaved with 7N NH₃ in MeOH. The solvent was evaporated under vacuum. The residue was purified by column chromatography on silica gel using a solvent gradient from 0% to 10% of NH₃ (7N in MeOH) in DCM to render final product **45** (white solid, 18 mg, 80%). LCMS (ESI): Rt = 3.67 min, m/z = 373.10 [M+ 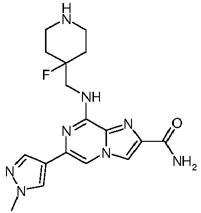 H]⁺. ¹H NMR (300 MHz, DMSO-*d6*) δ ppm 8.21 (s, 1H), 8.12 (s, 1H), 8.10 (s, 1H), 7.85 (s, 1H), 7.59 (s, 1H), 7.48 (s, 1H), 7.23 (t, *J* = 6.4 Hz, 1H), 3.88 (s, 3H), 3.87 - 3.77 (m, 2H), 2.87 - 2.69 (m, 4H), 1.73 (d, *J =* 17.4 Hz, 4H).

### Example 44: Synthesis of 8-[(4-Fluoro-piperidin-4-ylmethyl)-amino]-6-isothiazol-5-yl-imidazo[1,2-a]pyrazine-2-carboxylic acid amide (46)

Amberlyst-^{®}15 (20 eq) was added to a mixture of Intermediate **LXXXVI** (15 mg, 0.032 mmol) in MeOH (1 mL) at rt. The reaction was stirred at rt for 24 h. Then, the resine was washed with MeOH and the product was cleaved with 7N NH₃ in MeOH. The solvent was evaporated under vacuum. The residue was purified by column chromatography on silica gel using a solvent 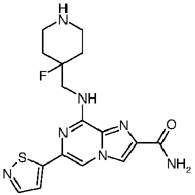 gradient from 0% to 10% of NH₃ (7N in MeOH) in DCM to render final product **46** (white solid, 4 mg, 33%). LCMS (ESI): Rt = 4.31 min, m/z = 376.00 [M+ H]⁺. ¹H NMR (300 MHz, DMSO-d6) δ ppm 8.64 (s, 1H), 8.59 (d, *J* = 1.4 Hz, 1H), 8.32 (s, 1H), 7.83 (ddd, *J* = 5.6, 3.6, 1.5 Hz, 1H), 7.79 (d, *J* = 1.2 Hz, 1H), 7.58 (s, 2H), 3.82 (dd, *J* = 19.1, 6.0 Hz, 2H), 3.04 - 2.94 (m, 2H), 2.90 - 2.79 (m, 2H), 1.83 (dd, *J* = 15.2, 2.7 Hz, 4H).

### Example 45: Synthesis of 8-[(4-Fluoro-piperidin-4-ylmethyl)-amino]-6-(1H-pyrazol-4-yl)-imidazo[1,2-a]pyrazine-2-carboxylic acid amide (47)

Amberlyst-^{®}15 (20 eq) was added to a mixture of Intermediate **LXXXVIII** (20 mg, 0.044 mmol) in MeOH (1 mL) at rt. The reaction was stirred at rt for 1 h. Then, the resine was washed with MeOH and the product was cleaved with 7N NH₃ in MeOH. The solvent was evaporated under vacuum. The residue was purified by column chromatography on silica gel using a solvent gradient from 0% to 10% of NH₃ (7N in MeOH) in DCM to render final 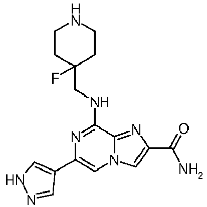 product **47** (white solid, 5 mg, 21%). LCMS (ESI): Rt = 0.39 & 3.26 min, m/z = 359.10 [M+ H]⁺. ¹H NMR (300 MHz, DMSO-*d6*) δ ppm 12.98 (s, 1H), 8.20 (s, 1H), 8.15 (s, 1H), 8.03 (s, 1H), 7.60 (s, 1H), 7.48 (s, 1H), 7.23 (t, *J* = 6.0 Hz, 1H), 3.85 (dd, *J* = 20.1, 6.0 Hz, 2H), 2.80 - 2.64 (m, 4H), 1.80 - 1.65 (m, 4H).

### Example 46: Synthesis of 6-(3-Fluoro-pyridin-4-yl)-8-(4-hydroxy-4-methyl-cyclohexylamino)-imidazo[1,2-a]pyrazine-2-carboxylic acid amide (48)

A mixture of Intermediate **XC** (100 mg, 0.241 mmol) and NH₃ (7N in MeOH, 2 mL) was heated in a pressure tube at 100 ºC for 24 h. After cooling to rt, the mixture was evaporated under vacuum. The residue was triturated from DCM/MeOH (9:1) and then washed with H₂O to render final product **48** (white solid, 15 mg, 16%). LCMS (ESI): Rt = 3.99 min, m/z = 385.10 [M+ H]⁺. ¹H NMR (300 MHz, DMSO-d6) δ ppm 8.66 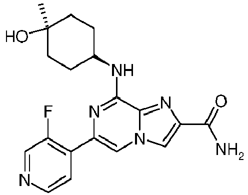 (d, *J* = 3.5 Hz, 1H), 8.55 (d, *J* = 4.2 Hz, 2H), 8.44 (s, 1H), 8.16 (dd, *J* = 6.8, 5.3 Hz, 1H), 7.57 (s, 2H), 7.25 (d, *J* = 7.6 Hz, 1H), 4.11 (s, 1H), 4.05 (s, 1H), 1.89 - 1.73 (m, 4H), 1.63 (d, J= 12.6 Hz, 2H), 1.54 - 1.44 (m, 2H), 1.15 (s, 3H).

### Example 47: Synthesis of 8-[(4-Fluoro-piperidin-4-ylmethyl)-amino]-6-thiazol-5-yl-imidazo[1,2-a]pyrazine-2-carboxylic acid amide (49)

Amberlyst-^{®}15 (20 eq) was added to a mixture of Intermediate XCII (30 mg, 0.063 mmol) in MeOH (2 mL) at rt. The reaction was stirred at rt for 1 h. Then, the resine was washed with MeOH and the product was cleaved with 7N NH₃ in MeOH. The solvent was evaporated under vacuum. The residue was purified by column chromatography on silica gel using a solvent gradient from 0% to 10% of NH₃ (7N in MeOH) in DCM to give final 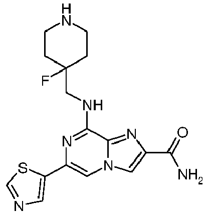 product **49** (white solid, 10 mg, 42%). LCMS (ESI): Rt = 3.97 min, m/z = 376.10 [M+ H]⁺. ¹H NMR (300 MHz, DMSO-*d6*) δ ppm 9.08 (s, 1H), 8.48 (s, 1H), 8.37 (s, 1H), 8.28 (s, 1H), 7.70 - 7.51 (m, 3H), 3.82 (dd, *J* = 19.8, 7.1 Hz, 2H), 2.84 - 2.73 (m, 4H), 1.78 - 1.62 (m, 4H).

### Synthesis of Intermediates

### 6-Bromo-8-(1-tert-butoxycarbonyl-piperidin-4-ylamino)-imidazo[1,2-a]pyrazine-2-carboxylic acid ethyl ester (Intermediate I)

A mixture of 6,8-dibromo-imidazo[1,2-a]pyrazine-2-carboxylic acid ethyl ester (300 mg, 0.860 mmol), 4-amino-1-Boc-piperidine (172 mg, 0.860 mmol) and DIPEA (0.15 mL, 0.860 mmol) in DCM (3.40 mL) was stirred at rt 16 h. The reaction mixture was evaporated under vacuum. The residue was 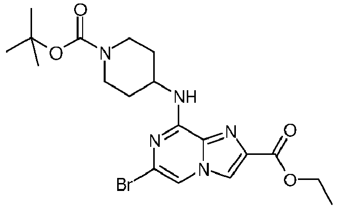 partitioned between DCM and a saturated aqueous solution of NaHCO₃. The aqueous layer was extracted 3 times with DCM. The combined organic layers were dried over Na₂SO₄, filtered and concentrated. The crude was purified by column chromatography on silica gel using a solvent gradient from 0% to 40% of EtOAc in cHex to afford Intermediate I (yellow solid, 190 mg, 47%). LCMS (ESI): Rt = 4.75 min, m/z = 490.10 [M+ Na]⁺. ¹H NMR (300 MHz, DMSO-d6) δ ppm 8.40 (s, 1H), 8.29 (d, *J* = 8.3 Hz, 1H), 7.98 (s, 1H), 4.31 (q, *J* = 7.1 Hz, 2H), 4.14 (brs, 1H), 3.97 (d, *J* = 11.4 Hz, 2H), 2.84 (brs, 2H), 1.79 (d, *J =* 10.0 Hz, 2H), 1.58 (dd, *J =* 20.3, 12.5 Hz, 2H), 1.40 (s, 9H), 1.30 (t, *J*= 7.1 Hz, 3H).

### 8-(1-tert-Butoxycarbonyl-piperidin-4-ylamino)-6-pyridin-4-yl-imidazo[1,2-a]pyrazine-2-carboxylic acid methyl ester (Intermediate II)

A mixture of Intermediate **I** (190 mg, 0.406 mmol), pyridine-4-boronic acid (60 mg, 0.487 mmol), Pd(dppf)Cl₂ (33 mg. 0.041 mmol) and a saturated aqueous solution of K₂CO₃ (0.50 mL) in 1,2-DME (1.80 mL) was heated at 130 ºC in a pressure tube for 16 h. The reaction mixture was cooled down to rt, and the solvent was 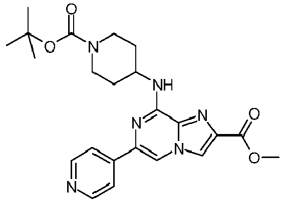 removed under vacuum. The residue was purified by column chromatography on silica gel using a solvent gradient from 0% to 40% of MeOH in EtOAc to give Intermediate **II** (yellow solid, 100 mg, 55%). LCMS (ESI): Rt = 3.52 min, m/z = 453.30 [M+ H]⁺. ¹H NMR (300 MHz, DMSO-d6) δ ppm 8.65 (d, *J* = 5.7 Hz, 2H), 8.60 (s, 1H), 8.48 (s, 1H), 8.02 (d, *J* = 7.9 Hz, 1H), 7.92 (d, *J* = 6.0 Hz, 2H), 4.39 (brs, 1H), 4.00 (d, *J =* 12.4 Hz, 2H), 3.86 (s, 3H), 2.92 (brs, 2H), 1.92 (dd, *J* = 12.8, 2.5 Hz, 2H), 1.62 (ddd, *J =* 24.7, 12.5, 4.0 Hz, 2H), 1.42 (s, 9H).

### 8-(Piperidin-4-ylamino)-6-pyridin-4-yl-imidazo[1,2-a]pyrazine-2-carboxylic acid methyl ester (Intermediate III)

Amberlyst-^{®}15 (243 mg, 1.140 mmol) was added to a mixture of Intermediate **II** (100 mg, 0.228 mmol) in MeOH (2.30 mL) at rt. The reaction was stirred at rt for 48 h. Then, the resine was washed with MeOH and the product was cleaved with 7N NH₃ in MeOH. The solvent was evaporated under vacuum to render Intermediate **III** (light brown solid, 25 mg, 31%). LCMS (ESI): Rt = 0.34 min, m/z = 353.20 [M+ H]⁺. 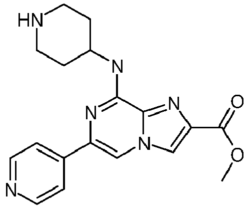 ¹H NMR (300 MHz, DMSO-*d6*) δ ppm 8.60 (dd, *J* = 4.6, 1.6 Hz, 2H), 8.56 (s, 1H), 8.43 (s, 1H), 8.02 (d, *J* = 7.6 Hz, 1H), 7.87 (dd, *J* = 4.6, 1.6 Hz, 2H), 4.29 (brs, 1H), 3.80 (s, 3H), 3.13 - 3.10 (m, 2H), 2.86 - 2.76 (m, 2H), 1.95 (dd, *J* = 12.5, 2.5 Hz, 2H), 1.77 - 1.63 (m, 2H).

### 6-Bromo-8-[(1-tert-butoxycarbonyl-piperidin-4-ylmethyl)-amino]-imidazo[1,2-a]pyrazine-2-carboxylic acid ethyl ester (Intermediate IV)

A mixture of 6,8-dibromo-imidazo[1,2-a]pyrazine-2-carboxylic acid ethyl ester (300 mg, 0.860 mmol) and 1-Boc-4-(aminomethyl)piperidine (0.18 mL, 0.860 mmol) in DCM (3.40 mL) was stirred at rt for 72 h. The reaction mixture was evaporated under vacuum. The residue was partitioned between DCM and a saturated aqueous solution of NaHCO₃. The aqueous layer was extracted 3 times with DCM. The combined organic layers were dried over Na₂SO₄, filtered and 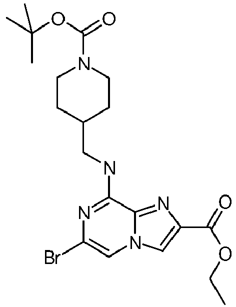 concentrated. The crude was purified by column chromatography on silica gel using a solvent gradient from 0% to 40% of MeOH in EtOAc to give Intermediate **IV** (yellow solid, 80 mg, 19%). LCMS (ESI): Rt = 4.77 min, m/z = 504.20/506.20 [M+ Na]⁺.

### 8-[(1-tert-Butoxycarbonyl-piperidin-4-ylmethyl)-amino]-6-pyridin-4-yl-imidazo[1,2-a]pyrazine-2-carboxylic acid ethyl ester (Intermediate V)

A mixture of Intermediate **IV** (80 mg, 0.166 mmol), pyridine-4-boronic acid (24 mg, 0.199 mmol), Pd(dppf)Cl₂ (14 mg. 0.017 mmol) and a saturated aqueous solution of K₂CO₃ (0.50 mL) in 1,2-DME (0.70 mL) was heated at 130 ºC in a pressure tube for 16 h. The reaction mixture was cooled down to rt, and the solvent removed under vacuum. The crude was purified by column chromatography on silica gel using a solvent gradient from 0% to 40% of MeOH in EtOAc to afford Intermediate **V** (yellow solid, 60 mg, 75%). LCMS (ESI): Rt = 3.69 min, 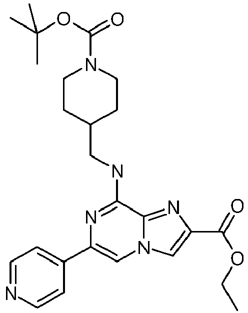 m/z = 481.20 [M+ H]⁺.

### 8-[(Piperidin-4-ylmethyl)-amino]-6-pyridin-4-yl-imidazo[1,2-a]pyrazine-2-carboxylic acid ethyl ester (Intermediate VI)

Amberlyst-^{®}15 (132 mg, 0.625 mmol) was added to a mixture of Intermediate **V** (60 mg, 0.125 mmol) in MeOH (1.20 mL) at rt. The reaction was stirred at rt for 48 h. Then, the resine was washed with MeOH and the product was cleaved with 7N NH₃ in MeOH. The solvent was evaporated under vacuum to render Intermediate **VI** (25 mg, 53%). LCMS (ESI): Rt = 0.41 min, m/z = 382.10 [M+ H]⁺. ¹H NMR (300 MHz, DMSO-*d6*) δ ppm 8.60 (dd, *J* = 4.6, 1.5 Hz, 2H), 8.51 (s, 1H), 8.40 (d, *J* = 1.7 Hz, 1H), 8.13 (t, *J* = 5.9 Hz, 1H), 7.85 (dd, *J* = 4.6, 1.5 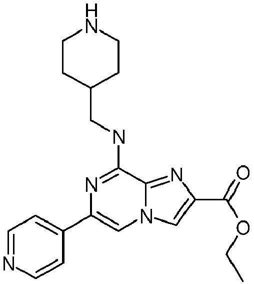 Hz, 2H), 3.80 (s, 3H), 3.44 - 3.35 (m, 2H), 2.84 (d, *J* = 11.7 Hz, 2H), 2.34 - 2.23 (m, 2H), 1.79 (brs, 1H), 1.61 - 1.47 (m, 2H), 1.12 - 0.88 (m, 2H).

### 8-[(1-tert-Butoxycarbonyl-piperidin-4-ylmethyl)-amino]-6-(2-chloro-pyridin-4-yl)-imidazo[1,2-a]pyrazine-2-carboxylic acid ethyl ester (Intermediate VII)

A mixture of Intermediate **IV** (100 mg, 0.207 mmol), 2-chloropyridine-4-boronic acid (49 mg, 0.311 mmol), Pd(dppf)Cl₂ (17 mg, 0.021 mmol) and Cs₂CO₃ (135 mg, 0.415 mmol) in 1,4-dioxane (2 mL) and H₂O (0.25 mL) was heated in a pressure tube at 150 ºC for 2 h. The mixture was partitioned between EtOAc and H₂O. The aqueous phase was extracted 3 times with EtOAc. The combined organic layers were dried over MgSO₄, filtered and concentrated. The crude was purified by column chromatography on silica gel using a solvent gradient from 20% to 100% of EtOAc in cHex to afford Intermediate **VII** (white solid, 48 mg, 45%). 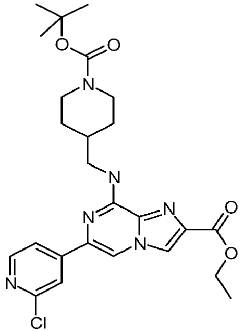 LCMS (ESI): Rt = 5.03 min, m/z = 415.10/417.10 [M+ H-Boc]⁺. ¹H NMR (300 MHz, CDCl₃) δ ppm 8.47 (d, *J* = 5.3 Hz, 1H), 8.13 (s, 1H), 7.95 (s, 1H), 7.86 (s, 1H), 7.71 (d, *J* = 4.4 Hz, 1H), 6.69 (s, 1H), 4.49 (q, *J* = 7.1 Hz, 2H), 4.12 (d, *J* = 7.2 Hz, 2H), 3.62 (t, *J* = 6.3 Hz, 2H), 2.72 (t, *J* = 12.1 Hz, 2H), 1.96 - 1.73 (m, 3H), 1.46 (s, 9H), 1.45 (t, *J* = 7.1 Hz, 3H), 1.27 (dt, *J* = 7.2, 4.5 Hz, 2H).

### 4-{[2-Carbamoyl-6-(2-chloro-pyridin-4-yl)-imidazo[1,2-a]pyrazin-8-ylamino]-methyl}-piperidine-1-carboxylic acid tert-butyl ester (intermediate VIII)

A mixture of Intermediate **VII** (27 mg, 0.052 mmol) and NH₃ (7N in MeOH, 0.75 mL, 5.243 mmol) was heated in a pressure tube at 100 ºC for 17 h. The solvent was concentrated *in vacuo* to render Intermediate **VIII** (yellowish solid, 21 mg, 84%). LCMS (ESI): Rt = 4.59 min, m/z = 386.10 [M+ H-Boc]⁺.

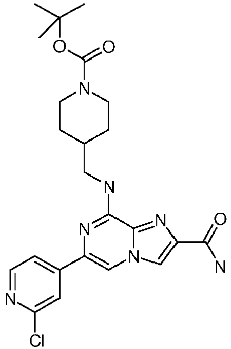

### 4-[(2-Methylcarbamoyl-6-pyridin-4-yl-imidazo[1,2-a]pyrazin-8-ylamino)-methyl]-piperidine-1-carboxylic acid tert-butyl ester (Intermediate IX)

A mixture of Intermediate **V** (70 mg, 0.146 mmol) and MeNH₂ (2M in MeOH, 3.64 mL, 7.283 mmol) was heated in a pressure tube at 100 ºC for 16 h. The solvent was evaporated under vacuum to render Intermediate **IX** (white solid, 65 mg, 96%). LCMS (ESI): Rt = 3.44 min, m/z = 466.20 [M+ H]⁺. ¹H NMR (300 MHz, DMSO-*d6*) δ ppm 8.66 (dd, *J* = 4.6, 1.5 Hz, 3H), 8.29 (s, 1H), 8.11 (d, *J* = 4.9 Hz, 1H), 7.94 (dd, *J* = 4.6, 1.6 Hz, 2H), 7.69 (t, *J* = 5.9 Hz, 1H), 3.95 (d, *J* = 12.1 Hz, 2H), 3.56 - 3.48 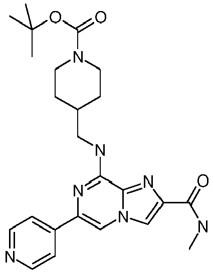 (m, 4H), 2.83 (d, *J* = 4.8 Hz, 3H), 1.95 (brs, 1H), 1.74 (dd, *J* = 12.9, 2.3 Hz, 2H), 1.39 (s, 9H), 1.13 (ddd, *J* = 25.1, 12.2, 4.0 Hz, 2H).

### 4-{[2-(2-Methoxy-ethylcarbamoyl)-6-pyridin-4-yl-imidazo[1,2-a]pyrazin-8-ylamino]-methyl}-piperidine-1-carboxylic acid tert-butyl ester (Intermediate X)

AlMe₃ (2M in hex, 0.16 mL, 0.312 mmol) was added to a mixture of Intermediate **V** (75 mg, 0.156 mmol) in EtOH (3 mL), followed by the addition of 2-methoxyethylamine (0.13 mL, 1.561 mmol) at rt. The reaction mixture was heated in a pressure tube at 150 ºC for 17 h. The mixture was cooled down to rt, and quenched with H₂O. The aqueous layer was extracted 3 times with EtOAc. The combined organic layers were dried over MgSO₄, filtered and concentrated to render Intermediate **X** (80 mg, 99%). LCMS (ESI): Rt = 3.56 min, m/z = 410.20 [M+ H-Boc]⁺. ¹H NMR (300 MHz, CDCl₃) δ ppm 8.62 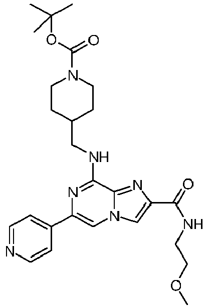 (s, 2H), 8.04 (s, 1H), 7.92 (s, 1H), 7.74 (d, *J* = 5.5 Hz, 2H), 7.49 (t, *J* = 5.6 Hz, 1H), 6.09 (t, *J =* 6.1 Hz, 1H), 4.11 (s, 1H), 3.61 (dd, *J* = 11.2, 5.1 Hz, 4H), 3.54 (dd, *J* = 9.5, 4.7 Hz, 3H), 3.35 (s, 3H), 2.67 (t, *J* = 12.4 Hz, 2H), 1.93 - 1.82 (m, 1H), 1.78 (d, *J* = 14.6 Hz, 2H), 1.39 (s, 9H), 1.32-1.21 (m, 2H).

### 6-Bromo-8-[(1-tert-butoxycarbonyl-4-cyano-piperidin-4-ylmethyl)-amino]-imidazo[1,2-a]pyrazine-2-carboxylic acid ethyl ester (Intermediate XI)

A mixture of 6,8-dibromo-imidazo[1,2-a]pyrazine-2-carboxylic acid ethyl ester (300 mg, 0.860 mmol) and tert-butyl 4-(aminomethyl)-4-cyanopiperidine-1-carboxylate (247 mg, 1.032 mmol) in 1,2-DCE (6 mL) was heated at 60 ºC in a pressure tube for 20 h. An additional amount of amine (0.2 eq) was added to the reaction mixture and it was heated at 100 ºC for 2 h. The mixture was evaporated under vacuum. The crude was purified by column chromatography on silica gel using a solvent gradient 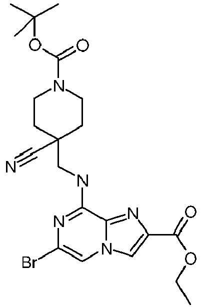 from 0% to 50% of EtOAc in cHex affording Intermediate **XI** (yellow solid, 156 mg, 36%). LCMS (ESI): Rt = 4.78 min, m/z = 407.10/409.10 [M+ H-Boc]⁺.

### 8-[(1-tert-Butoxycarbonyl-4-cyano-piperidin-4-ylmethyl)-amino]-6-(2-chloro-pyridin-4-yl)-imidazo[1,2-a]pyrazine-2-carboxylic acid ethyl ester (Intermediate XII)

A mixture of Intermediate **XI** (78 mg, 0.154 mmol), 2-chloropyridine-4-boronic acid (36 mg, 0.231 mmol), Pd(dppf)Cl₂ (13 mg, 0.015 mmol) and Cs₂CO₃ (100 mg, 0.307 mmol) in 1,4-dioxane (2 mL) and H₂O (0.20 mL) was heated in a pressure tube at 115 ºC for 2 h. The reaction mixture was partitioned between EtOAc and H₂O. The aqueous phase was extracted 3 times with EtOAc. The combined organic layers were dried over Na₂SO₄, filtered and concentrated. The 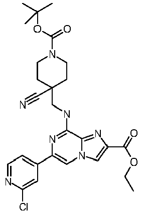 crude was purified by column chromatography on silica gel using a solvent gradient from 0% to 100% of EtOAc in cHex to render Intermediate **XII** (yellow solid, 32 mg, 38%). LCMS (ESI): Rt = 4.85 min, m/z = 440.10 [M+ H-Boc]⁺.

### 4-{[2-Carbamoyl-6-(2-chloro-pyridin-4-yl)-imidazo[1,2-a]pyrazin-8-ylamino]-ethyl}-4-cyano-piperidine-1-carboxylic acid tert-butyl ester (Intermediate XIII)

A mixture of Intermediate **XII** (32 mg, 0.059 mmol) and NH₃ (7N in MeOH, 0.85 mL, 5.926 mmol) was heated in pressure tube at 100 ºC for 22 h. The mixture was evaporated under vacuum to render Intermediate **XIII** (white solid, 27 mg, 89%). LCMS (ESI): Rt = 4.46 min, m/z = 411.10 [M+ H-Boc]⁺.

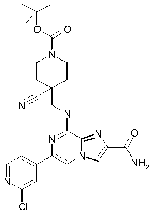

### 6,8-Dibromo-3-methyl-imidazo[1,2-a]pyrazine-2-carboxylic acid ethyl ester (Intermediate XIV)

A mixture of 2-amino-3,5-dibromopyrazine (2.40 g, 9.567 mmol) and 3-bromo-2-oxobutyric acid ethyl ester (2 g, 9.567 mmol) in 1,2-DME (5 mL) was heated at 115 ºC in a pressure tube for 24 h. The reaction was diluted with DCM, and the organic layer was washed with a saturated aqueous 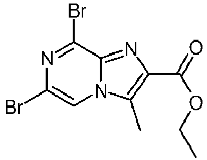 solution of NaHCO₃, dried over Na₂SO₄, filtered and concentrated. The crude was purified by column chromatography on silica gel using a solvent gradient from 0% to 50% of EtOAc in cHex to give Intermediate **XIV** (yellow solid, 918 mg, 29%). LCMS (ESI): Rt = 4.21 min, m/z = 363.90/395.90 [M+ H]⁺. ¹H NMR (300 MHz, CDCl₃) δ ppm 8.07 (s, 1H), 4.51 (q, *J =* 7.1 Hz, 2H), 2.83 (s, 3H), 1.47 (t, *J* = 7.1 Hz, 3H).

### 6-Bromo-8-[(1-tert-butoxycarbonyl-piperidin-4-ylmethyl)-amino]-3-methyl-imidazo[1,2-a]pyrazine-2-carboxylic acid ethyl ester (Intermediate XV)

A mixture of Intermediate **XIV** (85 mg, 0.234 mmol) and 1-Boc-4-(aminomethyl)piperidine (60 mg, 0.281 mmol) in 1,2-DCE (1 mL) was heated at 70 ºC in a pressure tube for 4 h. An additional amount of amine (0.3 eq) was added to the reaction mixture, and it was heated for 2 more hours. The solvent was evaporated under vacuum. The crude was purified by column chromatography on silica gel using a solvent gradient from 0% to 50% of EtOAc in cHex to give Intermediate **XV** (yellow solid, 130 mg, 99%). LCMS (ESI): Rt = 5.04 min, m/z = 396.10/398.10 [M+ H- 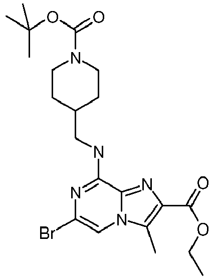 Boc]⁺. ¹H NMR (300 MHz, CDCl₃) δ ppm 7.29 (s, 1H), 4.40 (q, *J* = 7.1 Hz, 2H), 4.05 (dd, *J* = 14.6, 7.5 Hz, 2H), 3.43 (t, *J* = 6.3 Hz, 2H), 2.62 (s, 3H), 1.70 (d, *J* = 12.5 Hz, 2H), 1.39 (s, 9H), 1.37 (s, 3H), 1.22 - 1.11 (m, 3H).

### 8-[(1-tert-Butoxycarbonyl-piperidin-4-ylmethyl)-amino]-3-methyl-6-pyridin-4-yl-imidazo[1,2-a]pyrazine-2-carboxylic acid ethyl ester (Intermediate XVI)

A mixture of Intermediate **XV** (130 mg, 0.262 mmol), pyridine-4-boronic acid (48 mg, 0.363 mmol), Pd(dppf)Cl₂ (21 mg, 0.026 mmol) and Cs₂CO₃ (171 mg, 0.524 mmol) in 1,4-dioxane (2.6 mL) and H₂O (0.33 mL) was heated at 110 ºC in a pressure tube for 4 h. The reaction was partitioned between H₂O and DCM. The aqueous layer was extracted 3 times with DCM. The combined organic layers were dried over Na₂SO₄, filtered and concentrated. The crude was purified by column chromatography on silica 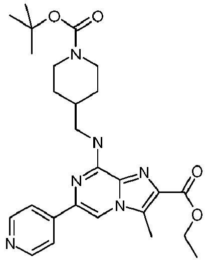 gel using a solvent gradient from 0% to 20% of MeOH in DCM to give Intermediate **XVI** (yellow solid, 95 mg, 73%). LCMS (ESI): Rt = 3.92 min, m/z = 495.30 [M+ H]⁺. ¹H NMR (300 MHz, CDCl₃) δ ppm 8.72 (s, 2H), 7.94 (s, 2H), 7.80 (d, *J* = 4.6 Hz, 1H), 6.43 (t, *J* = 5.6 Hz, 1H), 4.51 (q, *J* = 7.1 Hz, 2H), 4.14 (s, 2H), 3.64 (t, *J* = 6.3 Hz, 2H), 2.83 (d, *J* = 1.0 Hz, 3H), 2.73 (t, *J* = 12.5 Hz, 2H), 1.99 - 1.89 (m, 1H), 1.84 (d, *J* = 13.1 Hz, 2H), 1.50 - 1.45 (m, 13H), 1.37 - 1.21 (m, 2H).

### 4-[(3-Methyl-2-methylcarbamoyl-6-pyridin-4-yl-imidazo[1,2-a]pyrazin-8-ylamino)-methyl]-piperidine-1-carboxylic acid tert-butyl ester (Intermediate XVII)

A mixture of Intermediate **XVI** (30 mg, 0.061 mmol) and MeNH₂ (2M in MeOH, 1.52 mL, 3.033 mmol) was heated at 100 ºC in a pressure tube for 18 h. The solvent was evaporated under vacuum to render Intermediate **XVII** (29 mg) which was used in the next reaction step without further purification. LCMS (ESI): Rt = 3.58 min, m/z = 480.30 [M+ H]⁺.

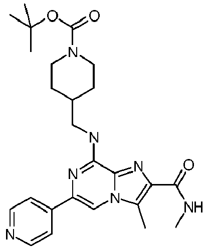

### 8-[(1-tert-Butoxycarbonyl-4-cyano-piperidin-4-ylmethyl)-amino]-6-pyridin-4-yl-imidazo[1,2-a]pyrazine-2-carboxylic acid ethyl ester (Intermediate XVIII)

A mixture of Intermediate **XI** (78 mg, 0.154 mmol), pyridine-4-boronic acid (28 mg, 0.231 mmol), Pd(dppf)Cl₂ (13 mg, 0.015 mmol) and Cs₂CO₃ (100 mg, 0.307 mmol) in 1,4-dioxane (2 mL) and H₂O (0.2 mL) was heated in a pressure tube at 115 ºC for 2 h. The reaction mixture was taken in EtOAc and H₂O. The aqueous phase was extracted 3 times with EtOAc. The combined organic layers were dried over Na₂SO₄, filtered and concentrated. The crude was purified by column chromatography on silica gel using a solvent gradient from 0% to 100% of EtOAc in cHex and then from 0% to 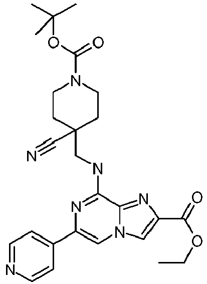 15% of MeOH in EtOAc to render Intermediate **XVIII** (yellow solid, 62 mg, 80%). LCMS (ESI): Rt = 3.71 min, m/z = 506.20 [M+ H]⁺.

### 4-[(2-Carbamoyl-6-pyridin-4-yl-imidazo[1,2-a]pyrazin-8-ylamino)-methyl]-4-cyanopiperidine-1-carboxylic acid tert-butyl ester (Intermediate XIX)

A mixture of Intermediate **XVIII** (62 mg, 0.123 mmol) and NH₃ (7N in MeOH, 1.75 mL, 12.263 mmol) was heated in a pressure tube at 100 ºC for 48 h. The mixture was evaporated under vacuum to render Intermediate **XIX** (yellowish solid, 50 mg, 85%). LCMS (ESI): Rt = 3.23 min, m/z = 477.20 [M+ H]⁺.

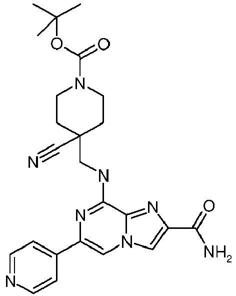

### 4-[(2-Carbamoyl-3-methyl-6-pyridin-4-yl-imidazo[1,2-a]pyrazin-8-ylamino)-methyl]-piperidine-1-carboxylic acid tert-butyl ester (Intermediate XX)

A mixture of Intermediate **XVI** (30 mg, 0.061 mmol) and NH₃ (7N in MeOH, 0.90 mL, 6.066 mmol) was heated in a pressure tube at 100 ºC for 48 h. The solvent was evaporated under vacuum to render Intermediate **XX** (28 mg, 99%) which was used in the next reaction step without further purification. LCMS (ESI): Rt = 3.42 min, m/z = 466.30 [M+ H]⁺.

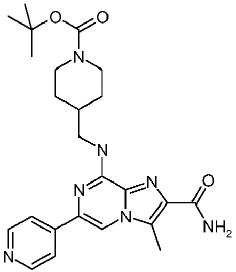

### 4-{[2-(2-Methoxy-ethylcarbamoyl)-3-methyl-6-pyridin-4-yl-imidazo[1,2-a]pyrazin-8-ylamino]-methyl}-piperidine-1-carboxylic acid tert-butyl ester (Intermediate XXI)

AlMe₃ (2M in hexane, 0.05 mL, 0.101 mmol) was added to a mixture of Intermediate **XVI** (25 mg, 0.051 mmol) in EtOH (1 mL) at rt under Ar, followed by the addition of 2-methoxyethylamine (0.04 mL, 0.505 mmol). The reaction was heated at 150 ºC in a pressure tube for 18 h. The reaction was partitioned between H₂O and DCM. The aqueous layer was extracted 3 times with DCM. The combined organic layers were dried over Na₂SO₄, filtered and concentrated to render Intermediate **XXI** (27 mg, 99%). LCMS (ESI): Rt = 3.66 min, m/z = 524.30 [M+ H]⁺.

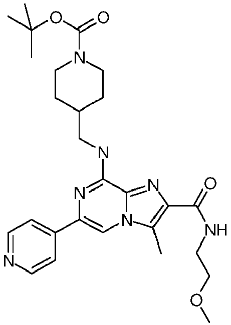

### 8-[(1-tert-Butoxycarbonyl-piperidin-4-ylmethyl)-amino]-6-(2-chloro-pyridin-4-yl)-3-methyl-imidazo[1,2-a]pyrazine-2-carboxylic acid ethyl ester (Intermediate XXII)

A mixture of Intermediate **XV** (134 mg, 0.270 mmol), 2-chloropyridine-4-boronic acid (64 mg, 0.405 mmol), Pd(dppf)Cl₂ (22 mg, 0.027 mmol) and Cs₂CO₃ (176 mg, 0.540 mmol) in 1,4-dioxane (2.70 mL) and H₂O (0.40 mL) was heated at 100 ºC in a pressure tube for 2 h. The reaction was partitioned between H₂O and DCM. The aqueous layer was extracted 3 times with DCM. The combined organic layers were dried over Na₂SO₄, filtered and concentrated. The crude was purified by column chromatography on silica gel using a solvent gradient from 0% to 10% of 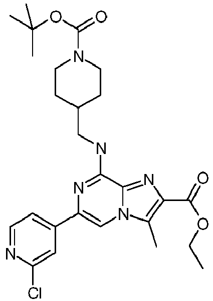 MeOH in DCM to afford the Intermediate **XXII** (yellow solid, 111 mg, 78%). LCMS (ESI): Rt = 5.16 min, m/z = 429.10 [M+ H-Boc]⁺. ¹H NMR (300 MHz, CDCl₃) δ ppm 8.38 (d, *J* = 5.0 Hz, 1H), 7.81 (s, 1H), 7.69 (d, *J* = 5.7 Hz, 2H), 6.42 (t, *J* = 5.6 Hz, 1H), 4.41 (q, *J* = 7.1 Hz, 2H), 4.07 (d, *J* = 9.0 Hz, 2H), 3.54 (t, *J* = 6.2 Hz, 2H), 2.75 (d, *J* = 10.5 Hz, 3H), 2.69 - 2.56 (m, 2H), 2.03 (d, *J* = 6.9 Hz, 1H), 1.93 - 1.67 (m, 2H), 1.42 - 1.36 (m, 12H), 1.27 - 1.13 (m, 2H).

### 4-{[6-(2-Chloro-pyridin-4-yl)-3-methyl-2-methylcarbamoyl-imidazo[1,2-a]pyrazin-8-ylamino]-methyl}-piperidine-1-carboxylic acid tert-butyl ester (Intermediate XXIII)

A mixture of Intermediate **XXII** (30 mg, 0.057 mmol) and MeNH₂ (2M in MeOH, 1.40 mL, 2.835 mmol) was heated at 100 ºC in a pressure tube for 18 h. The solvent was evaporated under vacuum to render Intermediate **XXIII** (29 mg, 99%) which was used in the next reaction step without further purification. LCMS (ESI): Rt = 4.86 min, m/z = 414.10 [M+ H-Boc]⁺.

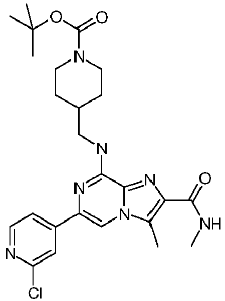

### 4-{[2-Carbamoyl-6-(2-chloro-pyridin-4-yl)-3-methyl-imidazo[1,2-a]pyrazin-8-ylamino]-methyl}-piperidine-1-carboxylic acid tert-butyl ester (Intermediate XXIV)

A mixture of Intermediate **XXII** (30 mg, 0.057 mmol) and NH₃ (7N in MeOH, 0.81 mL, 5.671 mmol) was heated at 100 ºC in a pressure tube for 48 h. The solvent was evaporated under vacuum to render Intermediate **XXIV** (28 mg, 99%) which was used in the next reaction step without additional purification. LCMS (ESI): Rt = 4.74 min, m/z = 400.10 [M+ H-Boc]⁺.

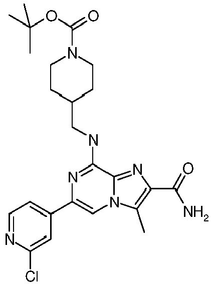

### 4-{[6-(2-Chloro-pyridin-4-yl)-2-(2-methoxy-ethylcarbamoyl)-3-methyl-imidazo[1,2-a]pyrazin-8-ylamino]-methyl}-piperidine-1-carboxylic acid tert-butyl ester (Intermediate XXV)

AlMe₃ (2M in hexane, 0.09 mL, 0.170 mmol) was added to a mixture of Intermediate **XXII** (45 mg, 0.085 mmol) in EtOH (1.70 mL) at rt under Ar, followed by the addition of 2-methoxyethylamine (0.07 mL, 0.851 mmol). The reaction was heated at 150 ºC in a pressure tube for 18 h. An additional amount of AlMe₃ (2M in hexane, 0.01 mL) and 2-methoxyethylamine (0.07 mL) was added to the reaction mixture, and it was heated at 150 ºC for 2 h. The reaction was partitioned between H₂O and DCM. The aqueous layer was extracted 3 times with DCM. The combined organic layers were dried 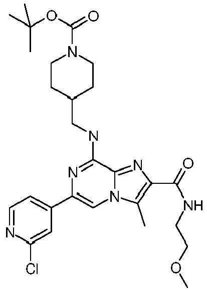 over Na₂SO₄, filtered and concentrated to give Intermediate **XXV** (47 mg, 99%) which was used in the next reaction step without further purification. LCMS (ESI): Rt = 4.94 min, m/z = 458.20 [M+ H-Boc]⁺.

### 6-Bromo-8-[(1-tert-butoxycarbonyl-4-fluoro-piperidin-4-ylmethyl)-amino]-3-methyl-imidazo[1,2-a]pyrazine-2-carboxylic acid ethyl ester (Intermediate XXVI)

A mixture of Intermediate **XIV** (400 mg, 1.102 mmol) and tert-butyl 4-(aminomethyl)-4-fluoropiperidine-I-carboxylate (333 mg, 1.432 mmol) in 1,2-DCE (7.30 mL) was heated at 85 ºC in a pressure tube for 18 h. An additional amount of tert-butyl 4-(aminomethyl)-4-fluoropiperidine-I-carboxylate (103 mg, 0.3 eq) was added to the reaction mixture, and it was heated at 100 ºC for 4 additional hours. The solvent was evaporated under vacuum. The crude was purified by column chromatography on silica gel 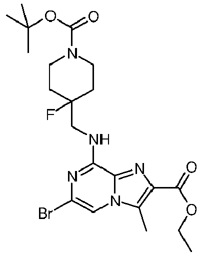 using a solvent gradient from 0% to 40% of EtOAc in cHex to give Intermediate **XXVI** (yellow solid, 364 mg, 64%). LCMS (ESI): Rt = 5.02 min, m/z = 514.20/516.20 [M+ H]⁺. ¹H NMR (300 MHz, CDCl₃) δ ppm 7.37 (s, 1H), 6.59 (t, *J* = 6.3 Hz, 1H), 4.45 (q, *J* = 7.1 Hz, 2H), 3.82 (dd, *J* = 21.7, 6.3 Hz, 4H), 3.10 (t, *J* = 11.8 Hz, 2H), 2.68 (s, 3H), 1.94 - 1.53 (m, 4H), 1.42 (s, 3H), 1.40 (s, 9H).

### 8-[(1-tert-Butoxycarbonyl-4-fluoro-piperidin-4-ylmethyl)-amino]-3-methyl-6-pyridin-4-yl-imidazo[1,2-a]pyrazine-2-carboxylic acid ethyl ester (Intermediate XXVII)

A mixture of Intermediate **XXVI** (180 mg, 0.350 mmol), pyridine-4-boronic acid (65 mg, 0.525 mmol), Pd(dppf)Cl₂ (29 mg, 0.035 mmol) and Cs₂CO₃ (228 mg, 0.700 mmol) in 1,4-dioxane (3.50 mL) and H₂O (0.44 mL) was heated at 110 ºC in a pressure tube for 18 h. The reaction was partitioned between H₂O and DCM. The aqueous layer was extracted 3 times with DCM. The combined organic layers were dried over Na₂SO₄, filtered and concentrated. The crude was purified by column chromatography on silica 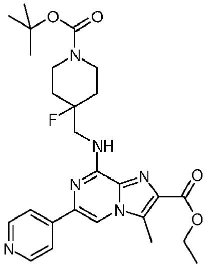 gel using a solvent gradient from 0% to 100% of EtOAc in cHex to give the Intermediate **XXVII** (yellow solid, 132 mg, 74%). LCMS (ESI): Rt = 3.91 min, m/z = 513.20 [M+ H]⁺. ¹H NMR (300 MHz, CDCl₃) δ ppm 8.64 (s, 2H), 7.77 (d, *J* = 22.4 Hz, 3H), 6.54 (s, 1H), 4.42 (dd, *J* = 13.8, 6.8 Hz, 2H), 3.90 (dd, *J* = 20.3, 5.5 Hz, 4H), 3.06 (dd, *J* = 22.9, 12.1 Hz, 2H), 2.75 (s, 3H), 1.95 - 1.80 (m, 2H), 1.75 - 1.55 (m, 2H), 1.40 (d, *J* = 12.9 Hz, 12H).

### 4-Fluoro-4-[(3-methyl-2-methylcarbamoyl-6-pyridin-4-yl-imidazo[1,2-a]pyrazin-8-ylamino)-methyl]-piperidine-1-carboxylic acid tert-butyl ester (Intermediate XXVIII)

A mixture of Intermediate **XXVII** (40 mg, 0.078 mmol) and MeNH₂ (2M in MeOH, 1.95 mL, 3.902 mmol) was heated at 100 ºC in a pressure tube for 18 h. The solvent was evaporated under vacuum to render Intermediate **XXVIII** (39 mg, 99%) which was used in the next reaction step without further purification. LCMS (ESI): Rt = 3.57 min, m/z = 498.20 [M+ H]⁺.

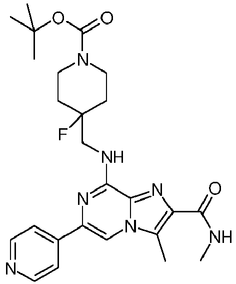

### 6-Bromo-8-[(1-tert-butoxycarbonyl-4-fluoro-piperidin-4-ylmethyl)-amino]-imidazo[1,2-a]pyrazine-2-carboxylic acid ethyl ester (Intermediate XXIX)

A mixture of 6,8-dibromo-imidazo[1,2-a]pyrazine-2-carboxylic acid ethyl ester (250 mg, 0.716 mmol) and (tert-butyl-4-(aminomethyl)-4-fluoropiperidine-I-carboxylate (250 mg, 1.075 mmol) in 1,2-DCE (7 mL) was heated in a pressure tube at 75 ºC for 16 h. An additional amount of amine (83 mg, 0.5 eq) was added to the reaction mixture and it was heated at 80 ºC for 4 days. The reaction mixture was evaporated under vacuum. The crude was purified by column chromatography on silica gel using a solvent gradient from 0% to 40% of EtOAc in DCM to render 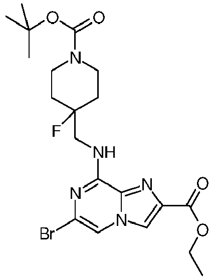 Intermediate **XXIX** (150 mg, 42%). LCMS (ESI): Rt = 4.93 min, m/z = 401.00/402.00 [M+ H-Boc]⁺. ¹H NMR (300 MHz, CDCl₃) δ ppm 8.01 (s, 1H), 7.58 (s, 1H), 4.46 (q, *J* = 7.1 Hz, 2H), 4.00 - 3.90 (m, 2H), 3.84 (dd, *J* = 21.4, 6.3 Hz, 2H), 3.12 (td, *J* = 13.4, 2.5 Hz, 2H), 1.89 (td, *J* = 12.0, 2.0 Hz, 2H), 1.72 (d, *J* = 0.8 Hz, 2H), 1.47 - 1.39 (m, 12H).

### 8-[(1-tert-Butoxycarbonyl-4-fluoro-piperidin-4-ylmethyl)-amino]-6-pyridin-4-yl-imidazo[1,2-a]pyrazine-2-carboxylic acid ethyl ester (Intermediate XXX)

A mixture of Intermediate **XXIX** (100 mg, 0.200 mmol), pyridine-4-boronic acid (37 mg, 0.300 mmol), Pd(dppf)Cl₂ (16 mg, 0.020 mmol) and Cs₂CO₃ (130 mg, 0.400 mmol) in 1,4-dioxane (2 mL) and H₂O (0.25 mL) was heated in a pressure tube at 115 ºC for 48 h. The reaction was taken in EtOAc and H₂O. The layers were separated and the aqueous phase was extracted with EtOAc. The combined organic layers were dried over Na₂SO₄, filtered and concentrated. The crude was purified by column chromatography on silica gel using a solvent gradient from 0% to 100% of EtOAc in DCM and then 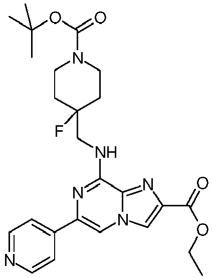 from 0% to 5% of MeOH in EtOAc to render Intermediate **XXX** (52 mg, 52 %). LCMS (ESI): Rt = 3.80 min, m/z = 499.20 [M+ H]⁺.

### 4-[(2-Carbamoyl-6-pyridin-4-yl-imidazo[1,2-a]pyrazin-8-ylamino)-methyl]-4-fluoro-piperidine-1-carboxylic acid tert-butyl ester (Intermediate XXXI)

A mixture of Intermediate **XXX** (52 mg, 0.104 mmol) and NH₃ (7N in MeOH, 1.42 mL, 10.430 mmol) was heated at 100 ºC in a pressure tube for 6 h. The reaction mixture was evaporated under vacuum to render Intermediate **XXXI** (white solid, 18 mg, 37%). LCMS (ESI): Rt = 3.32 min, m/z = 470.10 [M+ H]⁺.

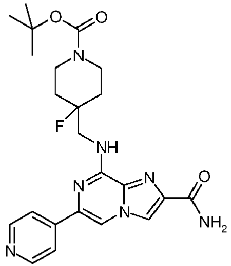

### 4-[(2-Carbamoyl-3-methyl-6-pyridin-4-yl-imidazo[1,2-a]pyrazin-8-ylamino)-methyl]-4-fluoro-piperidine-1-carboxylic acid tert-butyl ester (Intermediate XXXII)

A mixture of Intermediate **XXVII** (40 mg, 0.078 mmol) and NH₃ (7N in MeOH, 1.11 mL, 7.804 mmol) was heated at 100 ºC in a pressure tube for 48 h. The solvent was evaporated under vacuum to render desired Intermediate **XXXII** (38 mg, 99%). LCMS (ESI): Rt = 3.45 min, m/z = 484.30 [M+ H]⁺.

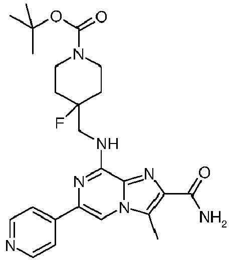

### 6-Bromo-8-[(1-tert-butoxycarbonyl-4-cyano-piperidin-4-ylmethyl)-amino]-3-methyl-imidazo[1,2-a]pyrazine-2-carboxylic acid ethyl ester (Intermediate XXXIII)

A mixture of Intermediate **XIV** (300 mg, 0.826 mmol) and 1-Boc-4-(aminomethyl)piperidine (257 mg, 1.074 mmol) in 1,2-DCE (5.50 mL) was heated at 85 ºC in a pressure tube for 18 h. An additional amount of amine (79 mg, 0.33 eq) was added to the reaction mixture, and it was heated at 100 ºC for 20 h. The solvent was evaporated under vacuum. The crude was purified by column chromatography on silica gel using a solvent gradient from 0% to 50% of EtOAc in cHex to give the desired Intermediate **XXXIII** (yellow solid, 261 mg, 61%). LCMS (ESI): Rt = 4.91 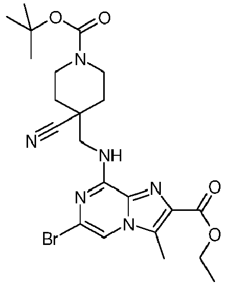 min, m/z = 421.00/423.00 [M+ H-Boc]⁺. ¹H NMR (300 MHz, CDCl₃) δ ppm 7.43 (s, 1H), 6.78 (s, 1H), 4.46 (q, *J =* 7.1 Hz, 2H), 4.23 - 4.09 (m, 2H), 3.89 (d, *J* = 6.6 Hz, 2H), 3.03 (t, *J =* 12.3 Hz, 2H), 2.71 (s, 3H), 1.97 (d, *J* = 13.3 Hz, 2H), 1.63 (td, *J* = 13.0, 4.3 Hz, 2H), 1.44 (s, 3H), 1.42 (s, 9H).

### 8-[(1-tert-Butoxycarbonyl-4-cyano-piperidin-4-ylmethyl)-amino]-3-methyl-6-pyridin-4-yl-imidazo[1,2-a]pyrazine-2-carboxylic acid ethyl ester (Intermediate XXXIV)

A mixture of Intermediate **XXXIII** (130 mg, 0.249 mmol), pyridine-4-boronic acid (46 mg, 0.374 mmol), Pd(dppf)Cl₂ (20 mg, 0.025 mmol) and Cs₂CO₃ (162 mg, 0.499 mmol) in 1,4-dioxane (2.50 mL) and H₂O (0.31 mL) was heated at 110°C in a pressure tube for 18 h. The reaction was partitioned between H₂O and DCM. The aqueous layer was extracted 3 times with DCM. The combined organic layers were dried over Na₂SO₄, filtered and concentrated. The crude was purified by column chromatography on silica 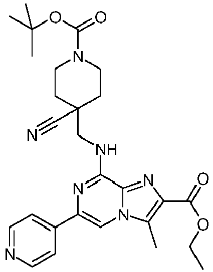 gel using a solvent gradient from 50% to 100% of EtOAc in cHex to give the desired Intermediate **XXXIV** (yellow solid, 84 mg, 65%). LCMS (ESI): Rt = 3.79 min, m/z = 520.30 [M+ H]⁺. ¹H NMR (300 MHz, CDCl₃) δ ppm 8.73 (d, *J* = 4.7 Hz, 2H), 7.86 (d, *J* = 9.2 Hz, 3H), 6.67 (t, *J* = 6.9 Hz, 1H), 4.50 (q, *J* = 7.1 Hz, 2H), 4.18 (s, 2H), 4.03 (d, *J* = 6.7 Hz, 2H), 3.06 (t, *J* = 12.5 Hz, 2H), 2.84 (s, 3H), 2.05 (d, *J* = 13.1 Hz, 2H), 1.67 (td, *J* = 13.2, 4.3 Hz, 2H), 1.51 - 1.45 (m, 12H).

### 4-Cyano-4-[(3-methyl-2-methylcarbamoyl-6-pyridin-4-yl-imidazo[1,2-a]pyrazin-8-ylamino)-methyl]-piperidine-1-carboxylic acid tert-butyl ester (Intermediate XXXV)

A mixture of Intermediate **XXXIV** (25 mg, 0.048 mmol) and MeNH₂ (2M in MeOH, 1.20 mL, 2.406 mmol) was heated at 100 ºC in a pressure tube for 18 h. The solvent was evaporated under vacuum to render Intermediate **XXXV** (24 mg, 99%) which was used in the next reaction step without further purification. LCMS (ESI): Rt = 3.45 min, m/z = 505.30 [M+ H]⁺.

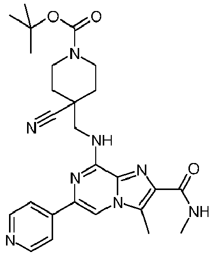

### 4-Cyano-4-{[2-(2-methoxy-ethylcarbamoyl)-3-methyl-6-pyridin-4-yl-imidazo[1,2-a]pyrazin-8-ylamino]-methyl}-piperidine-1-carboxylic acid tert-butyl ester (Intermediate XXXVI)

AIMe3 (2M in hexane, 0.07 mL, 0.131 mmol) was added to a mixture of Intermediate **XXXIV** (34 mg, 0.065 mmol) in EtOH (1.31 mL) at rt under Ar, followed by the addition of 2-methoxyethylamine (0.06 mL, 0.654 mmol). The reaction was heated at 150 ºC in a pressure tube for 18 h. The reaction was partitioned between H₂O and DCM. The aqueous layer was extracted 3 times with DCM. The combined organic layers were dried over Na₂SO₄, filtered and concentrated to render Intermediate **XXXVI,** which was used in the next reaction step without further purification (36 mg, 99%). LCMS (ESI): Rt = 3.55 min, m/z = 549.30 [M+ H]⁺.

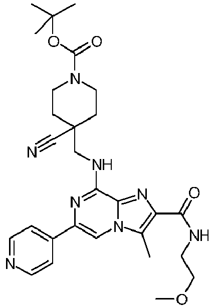

### 4-[(2-Carbamoyl-3-methyl-6-pyridin-4-yl-imidazo[1,2-a]pyrazin-8-ylamino)-methyl]-4-cyano-piperidine-1-carboxylic acid tert-butyl ester (Intermediate XXXVII)

A mixture of Intermediate **XXXIV** (25 mg, 0.048 mmol) and NH₃ (7N in MeOH, 0.69 mL, 4.811 mmol) was heated at 100 ºC in a pressure tube for 48 h. The solvent was evaporated under vacuum to render Intermediate **XXXVII** (24 mg, 99%) which was used in the next reaction step without further purification. LCMS (ESI): Rt = 3.34 min, m/z = 491.10 [M+ H]⁺.

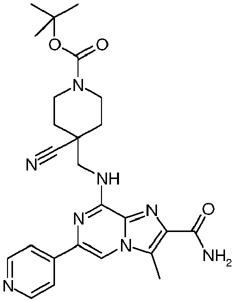

### 8-[(1-tert-Butoxycarbonyl-4-fluoro-piperidin-4-ylmethyl)-amino]-6-(2-chloro-pyridin-4-yl)-3-methyl-imidazo[1,2-a]pyrazine-2-carboxylic acid ethyl ester (Intermediate XXXVIII)

A mixture of Intermediate **XXVI** (170 mg, 0.330 mmol), 2-chloropyridine-4-boronic acid (78 mg, 0.496 mmol), Pd(dppf)Cl₂ (27 mg, 0.033 mmol) and Cs₂CO₃ (215 mg, 0.661 mmol) in 1,4-dioxane (3.30 mL) and H₂O (0.41 mL) was heated at 110 ºC in a pressure tube for 4 h. The reaction was partitioned between DCM and H₂O. The aqueous layer was extracted 3 times with DCM. The combined organic layers were dried over Na₂SO₄, filtered and concentrated. The resulting residue was purified by column chromatography on silica gel using a solvent gradient from 0% to 50% of 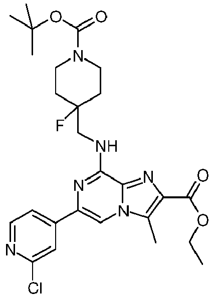 EtOAc in cHex to give the Intermediate **XXXVIII** (yellow solid, 84 mg, 47%). LCMS (ESI): Rt = 5.05 min, m/z = 447.00 [M+ H-Boc]⁺.

### 4-{[6-(2-Chloro-pyridin-4-yl)-3-methyl-2-methylcarbamoyl-imidazo[1,2-a]pyrazin-8-ylamino]-methyl}-4-fluoro-piperidine-1-carboxylic acid tert-butyl ester (Intermediate XXXIX)

A mixture of Intermediate **XXXVIII** (28 mg, 0.051 mmol) and MeNH₂ (2M in MeOH, 1.28 mL, 2.559 mmol) was heated at 100 ºC in a pressure tube for 18 h. The solvent was evaporated under vacuum to render Intermediate **XXXIX** (27 mg, 99%). LCMS (ESI): Rt = 4.78 min, m/z = 532.20 [M+ H]⁺.

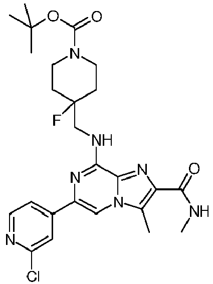

### 8-[(1-tert-Butoxycarbonyl-4-cyano-piperidin-4-ylmethyl)-amino]-6-(2-chloro-pyridin-4-yl)-3-methyl-imidazo[1,2-a]pyrazine-2-carboxylic acid ethyl ester (Intermediate XL)

A mixture of Intermediate **XXXIII** (125 mg, 0.240 mmol), 2-chloropyridine-4-boronic acid (57 mg, 0.360 mmol), Pd(dppf)Cl₂ (20 mg, 0.024 mmol) and Cs₂CO₃ (156 mg, 0.479 mmol) in 1,4-dioxane (2.40 mL) and H₂O (0.30 mL) was heated at 110 ºC in a pressure tube for 4 h. The reaction was partitioned between DCM and H₂O. The aqueous layer was extracted 3 times with DCM. The combined organic layers were dried over Na₂SO₄, filtered and concentrated. The crude was purified by column chromatography on silica gel using a solvent gradient from 0% to 50% of 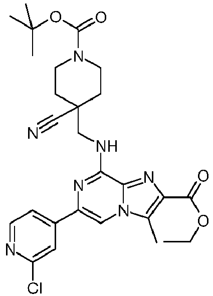 EtOAc in cHex to give the desired Intermediate **XL** (yellow solid, 59 mg, 44%). LCMS (ESI): Rt = 5.00 min, m/z = 455.20 [M+ H-Boc]⁺.

### 4-{[6-(2-Chloro-pyridin-4-yl)-3-methyl-2-methylcarbamoyl-imidazo[1,2-a]pyrazin-8-ylamino]-methyl}-4-cyano-piperidine-1-carboxylic acid tert-butyl ester (Intermediate XLI)

A mixture of Intermediate **XL** (20 mg, 0.036 mmol) and MeNH₂ (2M in MeOH, 0.90 mL, 1.805 mmol) was heated at 100 ºC in a pressure tube for 18 h. The solvent was evaporated under vacuum to give the desired Intermediate **XLI** (19 mg, 98%). LCMS (ESI): Rt = 4.69 min, m/z = 439.10 [M+ H-Boc]⁺.

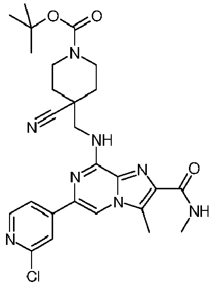

### 4-{[6-(2-Chloro-pyridin-4-yl)-2-(2-methoxy-ethylcarbamoyl)-3-methyl-imidazo[1,2-a]pyrazin-8-ylamino]-methyl}-4-fluoro-piperidine-1-carboxylic acid tert-butyl ester (Intermediate XLII)

AlMe₃ (2M in hexane, 0.05 mL, 0.102 mmol) was added to a mixture of Intermediate **XXXVIII** (28 mg, 0.051 mmol) and 2-methoxyethylamine (0.04 mL, 0.512 mmol) in EtOH (1 mL) at rt under Ar. The reaction was heated at 150 ºC in a pressure tube for 18 h. An additional amount of amine (0.04 mL) and AlMe₃ (2M in hexane, 0.05 mL) was added, and it was heated at 150 ºC for 20 h. The reaction was partitioned between H₂O and DCM. The aqueous layer was extracted 3 times with DCM. The combined organic layers were 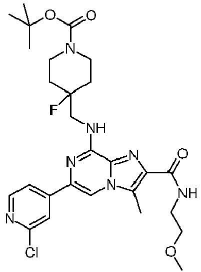 dried over Na₂SO₄, filtered and concentrated to render desired Intermediate **XLII** (29 mg, 98%) which was used in the next reaction step without further purification. LCMS (ESI): Rt = 3.21 min, m/z = 578.20 [M+ H]⁺.

### 4-{[6-(2-Chloro-pyridin-4-yl)-2-(2-methoxy-ethylcarbamoyl)-3-methyl-imidazo[1,2-a]pyrazin-8-ylamino]-methyl}-4-cyano-piperidine-1-carboxylic acid tert-butyl ester (Intermediate XLIII)

AlMe₃ (2M in hexane, 0.04 mL, 0.072 mmol) was added to a mixture of Intermediate **XL** (20 mg, 0.036 mmol) and 2-methoxyethylamine (0.03 mL, 0.361 mmol) in EtOH (0.72 mL) at rt under Ar. The reaction was heated at 150 ºC in a pressure tube for 18 h. An additional amount of amine (0.03 mL) and AlMe₃ (2M in hexane, 0.04 mL) was added, and it was heated at 150 ºC for 20 h. The reaction was partitioned between H₂O and DCM. The aqueous layer was extracted 3 times with DCM. The combined organic layers were dried over Na₂SO₄, filtered and concentrated to render 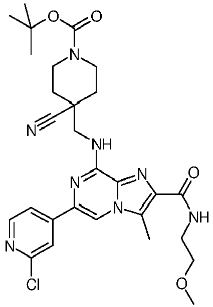 Intermediate **XLIII** (21 mg, 99%) which was used in the next reaction step without further purification. LCMS (ESI): Rt = 4.79 min, m/z = 483.10 [M+ H-Boc]⁺.

### 4-{[2-Carbamoyl-6-(2-chloro-pyridin-4-yl)-3-methyl-imidazo[1,2-a]pyrazin-8-ylamino]-methyl}-4-cyano-piperidine-1-carboxylic acid tert-butyl ester (Intermediate XLIV)

A mixture of Intermediate **XL** (20 mg, 0.036 mmol) and NH₃ (7N in MeOH, 0.52 mL, 3.610 mmol) was heated at 100 ºC in a pressure tube for 48 h. The solvent was evaporated under vacuum to render desired Intermediate **XLIV** (19 mg, 99%) which was used in the next reaction step without further purification. LCMS (ESI): Rt = 4.57 min, m/z = 425.00 [M+ H-Boc]⁺.

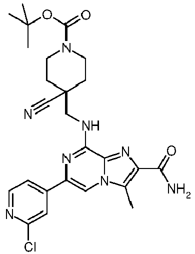

### 4-{[2-Carbamoyl-6-(2-chloro-pyridin-4-yl)-3-methyl-imidazo[1,2-a]pyrazin-8-ylamino]-methyl}-4-fluoro-piperidine-1-carboxylic acid tert-butyl ester (Intermediate XLV)

A mixture of Intermediate **XXXVIII** (28 mg, 0.051 mmol) and NH₃ (7N in MeOH, 0.73 mL, 5.119 mmol) was heated at 100 ºC in a pressure for 48 h. The solvent was evaporated under vaccum to render desired Intermediate **XLV** (26 mg, 98%) which was used in the next reaction step without further purification. LCMS (ESI): Rt = 4.69 min, m/z = 418.00 [M+ H-Boc]⁺.

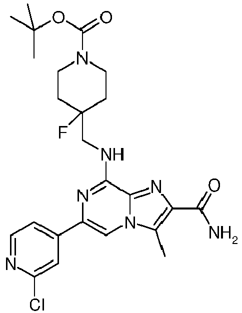

### 8-[(1-tert-Butoxycarbonyl-piperidin-4-ylmethyl)-amino]-6-(3-fluoro-pyridin-4-yl)-imidazo[1,2-a]pyrazine-2-carboxylic acid ethyl ester (Intermediate XLVI)

A mixture of Intermediate **IV** (150 mg, 0.311 mmol), 3-fluoropyridine-4-boronic acid pinacol ester (104 mg, 0.466 mmol), PdCl₂(PPh₃)₂ (22 mg, 0.031 mmol) and 2M Na₂CO₃ aqueous solution (0.46 mL) in 1,4-dioxane (3 mL) was heated at 110 ºC in a pressure tube for 90 min. The mixture was partitioned between EtOAc and H₂O. The aqueous layer was extracted 3 times with EtOAc. The combined organic layers were dried over Na₂SO₄, filtered and concentrated. The crude was purified by column chromatography on silica gel using a solvent gradient from 0% to 60% of EtOAc in cHex to afford Intermediate **XLVI** 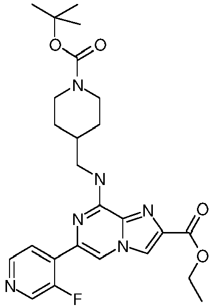 (white solid, 100 mg, 64%). LCMS (ESI): Rt = 4.86 min, m/z = 399.10 [M+ H-Boc]⁺.

### 4-{[2-Carbamoyl-6-(3-fluoro-pyridin-4-yl)-imidazo[1,2-a]pyrazin-8-ylamino]-methyl}-piperidine-1-carboxylic acid tert-butyl ester (Intermediate XLVII)

A mixture of Intermediate **XLVI** (40 mg, 0.080 mmol) and NH₃ (7N in MeOH, 1.14 mL, 8.023 mmol) was heated at 100 ºC in a pressure tube for 18 h. The mixture was evaporated under vacuum to render Intermediate **XLVII** (off-white solid, 37 mg, 99%) which was used as such in the next reaction step. LCMS (ESI): Rt = 4.28 min, m/z = 370.00 [M+ H-Boc]+.

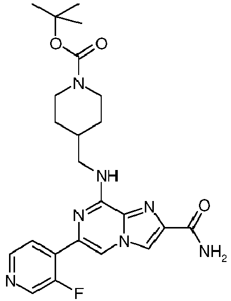

### 4-{[6-(3-Fluoro-pyridin-4-yl)-2-methylcarbamoyl-imidazo[1,2-a]pyrazin-8-ylamino]-methyl}-piperidine-1-carboxylic acid tert-butyl ester (Intermediate XLVIII)

A mixture of Intermediate **XLVI** (58 mg, 0.116 mmol) and MeNH₂ (2M in MeOH, 2.90 mL, 5.817 mmol) was heated in a pressure tube at 100 ºC for 4 h. The mixture was evaporated under vacuum to yield Intermediate **XLVIII** (brownish solid, 56 mg, 99%) which was used in the next reaction step without further purification. LCMS (ESI): Rt = 4.48 min, m/z = 384.10 [M+ H-Boc]⁺.

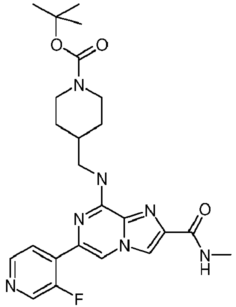

### 8-[(1-tert-Butoxycarbonyl-4-cyano-piperidin-4-ylmethyl)-amino]-6-(3-fluoro-pyridin-4-yl)-imidazo[1,2-a]pyrazine-2-carboxylic acid ethyl ester (Intermediate XLIX)

A mixture of Intermediate **XI** (100 mg, 0.197 mmol), 3-fluoropyridine-4-boronic acid pinacol ester (66 mg, 0.296 mmol), PdCl₂(PPh₃)₂ (14 mg, 0.020 mmol) and 2M Na₂CO₃ aqueous solution (0.30 mL) in 1,4-dioxane (2 mL) was heated at 115 ºC in a pressure tube for 5 h. The mixture was partitioned between EtOAc and H₂O. The aqueous solution was extracted 3 times with EtOAc. The combined organic layers were dried over Na₂SO₄, filtered and concentrated. The crude was purified by column chromatography on silica gel using a solvent gradient from 0% to 60% of EtOAc in cHex to afford Intermediate **XLIX** (90 mg, 87%). 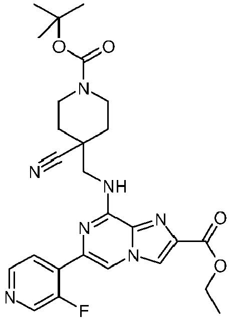 LCMS (ESI): Rt = 4.70 min, m/z = 424.20 [M+ H-Boc]⁺.

### 4-{[2-Carbamoyl-6-(3-fluoro-pyridin-4-yl)-imidazo[1,2-a]pyrazin-8-ylamino]-methyl}-4-cyano-piperidine-1-carboxylic acid tert-butyl ester (Intermediate L)

A mixture of Intermediate **XLIX** (50 mg, 0.095 mmol) in NH₃ (7N in MeOH, 1.36 mL, 9.550 mmol) was heated in a pressure tube at 100 ºC for 16 h. The reaction mixture was evaporated under vacuum to render Intermediate **L** (yellowish solid, 47 mg, 99%) which was used in the next reaction step without further purification. LCMS (ESI): Rt = 4.17 min, m/z = 495.10 [M+ H]⁺.

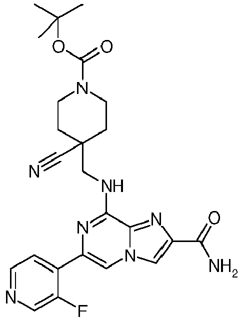

### 8-[(1-tert-Butoxycarbonyl-piperidin-4-ylmethyl)-amino]-6-(3-chloro-pyridin-4-yl)-imidazo[1,2-a]pyrazine-2-carboxylic acid ethyl ester (Intermediate LI)

A mixture of Intermediate **IV** (104 mg, 0.216 mmol), 3-chloro-4-pyridineboronic acid hydrate (57 mg, 0.323 mmol), PdCl₂(PPh₃)₂ (15 mg, 0.022 mmol) and 2M Na₂CO₃ aqueous solution (0.3 mL) in 1,4-dioxane (2 mL) was heated at 115 ºC in a pressure tube for 4 h. The mixture was partitioned between EtOAc and H₂O. The aqueous solution was extracted 3 times with EtOAc. The combined organic layers were dried over Na₂SO₄, filtered and concentrated. The crude was purified by column chromatography on silica gel using a solvent gradient from 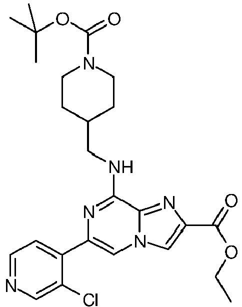 0% to 60% of EtOAc in cHex to afford Intermediate **LI** (72 mg, 64%). LCMS (ESI): Rt = 4.89 min, m/z = 415.20 [M+ H-Boc]⁺.

### 4-{[2-Carbamoyl-6-(3-chloro-pyridin-4-yl)-imidazo[1,2-a]pyrazin-8-ylamino]-methyl}-piperidine-1-carboxylic acid tert-butyl ester (Intermediate LII)

A mixture of Intermediate LI (43 mg, 0.083 mmol) and NH₃ (7N in MeOH, 1.33 mL, 9.514 mmol) was heated in a pressure tube at 100 ºC for 21 h. The mixture was evaporated under vacuum to render Intermediate **LII** (41 mg, 99%). LCMS (ESI): Rt = 4.37 min, m/z = 386.10 [M+ H-Boc]⁺.

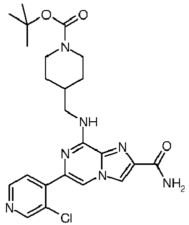

### 4-{[6-(3-Chloro-pyridin-4-yl)-2-methylcarbamoyl-imidazo[1,2-a]pyrazin-8-ylamino]-methyl}-piperidine-1-carboxylic acid tert-butyl ester (Intermediate LIII)

A mixture of Intermediate **LI** (29 mg, 0.056 mmol) and MeNH₂ (2N in MeOH, 1.40 mL, 2.815 mmol) was heated at 100 ºC in a pressure tube for 18 h. The mixture was evaporated under vacuum to render Intermediate **LIII** (yellowish solid, 28 mg, 99%) which was used without further purification in the next step. LCMS (ESI): Rt = 4.49 min, m/z = 400.10 [M+ H-Boc]+.

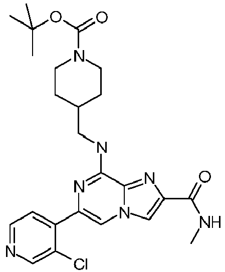

### 4-Cyano-4-{[6-(3-fluoro-pyridin-4-yl)-2-methylcarbamoyl-i midazo[1,2-a]pyrazin-8-ylamino]-methyl}-piperidine-1-carboxylic acid tert-butyl ester (Intermediate LIV)

A mixture of Intermediate **XLIV** (40 mg, 0.076 mmol) and MeNH₂ (2N in MeOH, 1.91 mL, 3.820 mmol) was heated at 100 ºC in a pressure tube for 18 h. The mixture was evaporated under vacuum to render Intermediate **LIV** (off-white solid, 39 mg, 99%). LCMS (ESI): Rt = 4.28 min, m/z = 409.20 [M+ H-Boc]⁺.

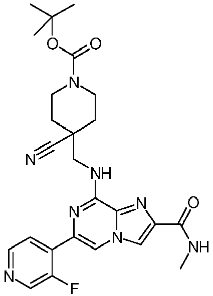

### 4-{[2-Carbamoyl-6-(3-fluoro-pyridin-4-yl)-imidazo[1,2-a]pyrazin-8-ylamino]-methyl}-4-fluoro-piperidine-1-carboxylic acid tert-butyl ester (Intermediate LV)

A mixture of Intermediate **XCIII** (100 mg, 0.194 mmol) and NH₃ (7N in MeOH, 5 mL) was heated in a pressure tube at 100 ºC for 48 h. The reaction mixture was evaporated under vacuum to afford Intermediate **LV** (90 mg, 95%) which was used without further purification. LCMS (ESI): Rt = 4.26 min, m/z = 388.20 [M+ H-Boc]⁺.

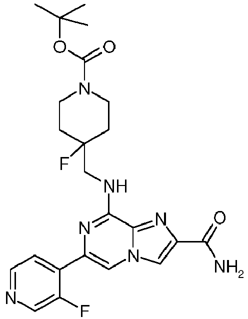

### 8-[(1-tert-Butoxycarbonyl-piperidin-4-ylmethyl)-amino]-6-(3-fluoro-pyridin-4-yl)-3-methyl-imidazo[1,2-a]pyrazine-2-carboxylic acid ethyl ester (Intermediate LVII)

A mixture of Intermediate **XV** (124 mg, 0.250 mmol), 3-fluoropyridine-4-boronic acid pinacol ester (84 mg, 0.375 mmol), PdCl₂(PPh₃)₂ (18 mg, 0.025 mmol) and 2M Na₂CO₃ aqueous solution (0.37 mL) in 1,4-dioxane (2.50 mL) was heated at 110 ºC in a pressure tube for 18 h. Excess of reagents were added till completion of the reaction. The reaction was partitioned between H₂O and DCM. The aqueous layer was extracted with DCM. The combined organic layers were dried over Na₂SO₄, filtered and concentrated. The crude 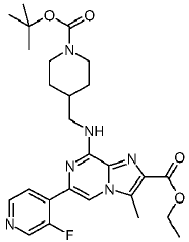 was purified by column chromatography on silica gel using a solvent gradient from 0% to 10% of MeOH in DCM to afford the desired Intermediate **LVII** (pale yellow solid, 130 mg, 99%). LCMS (ESI): Rt = 4.96 min, m/z = 513.50 [M+ H]⁺.

### 4-{[6-(3-Fluoro-pyridin-4-yl)-3-methyl-2-methylcarbamoyl-imidazo[1,2-a]pyrazin-8-ylamino]-methyl}-piperidine-1-carboxylic acid tert-butyl ester (Intermediate LVIII)

A mixture of Intermediate **LVII** (65 mg, 0.127 mmol) and MeNH₂ (2M in MeOH, 3.17 mL, 6.340 mmol) was heated at 100 ºC in a pressure tube for 20 h. The solvent was evaporated under vacuum to render Intermediate **LVIII** (63 mg, 99%). LCMS (ESI): Rt = 4.63 min, m/z = 498.50 [M+ H]⁺.

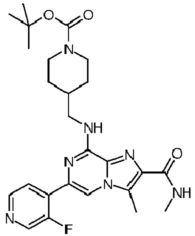

### 8-[(1-tert-Butoxycarbonyl-piperidin-4-ylmethyl)-amino]-6-(2-methyl-pyridin-4-yl)-imidazo[1,2-a]pyrazine-2-carboxylic acid ethyl ester (Intermediate LIX)

A mixture of Intermediate **IV** (100 mg, 0.207 mmol), 2-picoline-4-boronic acid (43 mg, 0.311 mmol), Pd(dppf)Cl₂ (17 mg, 0.021 mmol) and Cs₂CO₃ (135 mg, 0.415 mmol) in 1,4-dioxane (2.10 mL) and H₂O (0.26 mL) was heated at 115 ºC in a pressure tube for 16 h. The reaction was partitioned between H₂O and DCM. The aqueous layer was extracted 3 times with DCM. The combined organic layers were dried over Na₂SO₄, filtered and concentrated. The crude was purified by column chromatography on silica gel using a 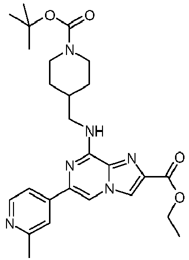 solvent gradient from 10% to 100% of EtOAc in cHex to give Intermediate **LIX** (yellow solid, 60 mg, 59%). LCMS (ESI): Rt = 3.64 min, m/z = 495.30 [M+ H]⁺.

### 4-{[2-Carbamoyl-6-(2-methyl-pyridin-4-yl)-imidazo[1,2-a]pyrazin-8-ylamino]-methyl}-piperidine-1-carboxylic acid tert-butyl ester (Intermediate LX)

A mixture of Intermediate **LIX** (30 mg, 0.061 mmol) and NH₃ (7N in MeOH, 0.85 mL, 6.066 mmol) was heated in a pressure tube at 100 ºC for 20 h. The mixture was evaporated under vacuum to render Intermediate **LX** (white solid, 28 mg, 99%) which was used as such in the next reaction step. LCMS (ESI): Rt = 3.16 min, m/z = 466.20 [M+ H]⁺.

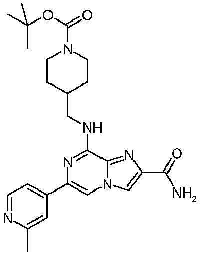

### 6-Bromo-8-[2-(1-tert-butoxycarbonyl-piperidin-4-yl)-ethylamino]-imidazo[1,2-a]pyrazine-2-carboxylic acid ethyl ester (Intermediate LXI)

A mixture of 6,8-dibromo-imidazo[1,2-a]pyrazine-2-carboxylic acid ethyl ester (300 mg, 0.860 mmol), 4-(2-aminoethyl)-1-Boc-piperidine (294 mg, 1.289 mmol) and DIPEA (0.23 mL, 1.289 mmol) in 1,2-DCE (9 mL) was heated at 100 ºC in a pressure tube for 18 h. The solvent was evaporated under vacuum. The crude was purified by column chromatography on silica gel using a solvent gradient from 0% to 10% 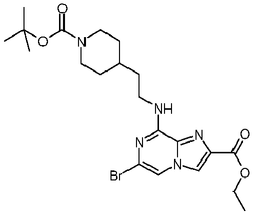 of MeOH in DCM to give Intermediate **LXI** (yellow solid, 450 mg, 99%). LCMS (ESI): Rt = 5.02 min, m/z = 396.00/398.00 [M+ H-Boc]⁺. ¹H NMR (700 MHz, DMSO-d6) δ ppm 8.45 (t, *J* = 5.7 Hz, 1H), 8.40 (s, 1H), 7.96 (s, 1H), 4.32 (q, *J* = 7.1 Hz, 2H), 3.92 (s, 2H), 3.46 (dd, *J* = 12.6, 6.4 Hz, 2H), 1.72 (d, *J* = 12.2 Hz, 2H), 1.54 (q, *J* = 6.8 Hz, 2H), 1.48 (dtd, *J* = 14.7, 7.3, 3.5 Hz, 1H), 1.39 (s, 9H), 1.31 (t, *J* = 7.1 Hz, 3H), 1.01 - 0.94 (m, 2H).

### 8-[2-(1-tert-Butoxycarbonyl-piperidin-4-yl)-ethylamino]-6-pyridin-4-yl-imidazo[1,2-a]pyrazine-2-carboxylic acid ethyl ester (Intermediate LXII)

A mixture of Intermediate **LXI** (290 mg, 0.584 mmol), pyridine-4-boronic acid pinacol ester (180 mg, 0.876 mmol), Pd(dppf)Cl₂ (48 mg, 0.058 mmol) and Cs₂CO₃ (381 mg, 1.168 mmol) in 1,4-dioxane (6 mL) and H₂O (0.73 mL) was heated at 110 ºC in a pressure tube for 16 h. The reaction was partitioned between H₂O and DCM. The aqueous layer was extracted 3 times with DCM. The combined organic layers 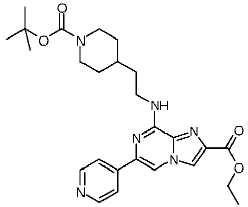 were dried over Na₂SO₄, filtered and concentrated. The crude was purified by column chromatography on silica gel using a solvent gradient from 0% to 10% of MeOH in DCM to give the desired Intermediate **LXII** (yellow solid, 300 mg, 99%). LCMS (ESI): Rt = 4.08 min, m/z = 495.30 [M+ H]⁺. ¹H NMR (700 MHz, DMSO-*d6*) δ ppm 8.67 (dd, J = 4.6, 1.5 Hz, 2H), 8.59 (s, 1H), 8.48 (s, 1H), 8.21 (t, J = 5.7 Hz, 1H), 7.92 (dd, J = 4.6, 1.5 Hz, 2H), 4.35 (q, J = 7.1 Hz, 2H), 3.91 (s, 2H), 3.63 (dd, J = 12.6, 6.4 Hz, 2H), 1.76 (d, J = 11.9 Hz, 2H), 1.64 (dd, J = 13.6, 6.8 Hz, 2H), 1.55 (ddd, J = 10.9, 7.2, 3.8 Hz, 1H), 1.34 (t, J = 7.1 Hz, 3H), 1.08 (s, 11H), 1.06 - 1.00 (m, 2H).

### 4-[2-(2-Carbamoyl-6-pyridin-4-yl-imidazo[1,2-a]pyrazin-8-ylamino)-ethyl]-piperidine-1-carboxylic acid tert-butyl ester (Intermediate LXIII)

A mixture of Intermediate **LXII** (300 mg, 0.607 mmol) and NH₃ (7N in MeOH, 9 mL, 64.656 mmol) was heated at 100 ºC in a pressure tube for 18 h. The solvent was evaporated under vacuum to render Intermediate **LXIII** (269 mg, 95%). LCMS (ESI): Rt = 3.50 min, m/z = 466.30 [M+ H]⁺.

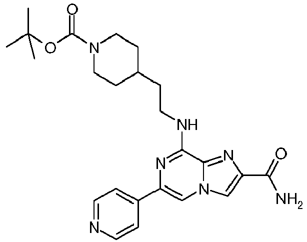

### 6-Bromo-8-(trans-4-tert-butoxycarbonylamino-cyclohexylamino)-imidazo[1,2-a]pyrazine-2-carboxylic acid ethyl ester (Intermediate LXIV)

A mixture of 6,8-dibromo-imidazo[1,2-a]pyrazine-2-carboxylic acid ethyl ester (300 mg, 0.860 mmol), N-Boc-trans-1,4-cyclohexanediamine (276 mg, 1.289 mmol) and DIPEA (0.23 mL, 1.289 mmol) in 1,2-DCE (9 mL) was heated at 110 ºC in a pressure tube for 4 h. The solvent was evaporated under vacuum. The 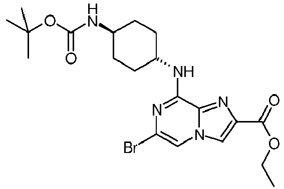 crude was purified by column chromatography on silica gel using a solvent gradient from 0% to 10% of MeOH in DCM to give Intermediate **LXIV** (yellow solid, 283 mg, 68%). LCMS (ESI): Rt = 4.84 min, m/z = 506.10 [M+ Na]⁺. ¹H NMR (700 MHz, DMSO-*d6*) δ ppm 8.39 (s, 1H), 8.16 (d, *J* = 8.4 Hz, 1H), 7.96 (s, 1H), 6.78 (d, *J* = 8.1 Hz, 1H), 4.32 (q, *J* = 7.1 Hz, 2H), 3.94 - 3.87 (m, 1H), 3.25 - 3.18 (m, 1H), 1.82 (dd, *J* = 23.3, 12.1 Hz, 4H), 1.57 (dd, *J* = 24.1, 11.5 Hz, 2H), 1.39 (s, 9H), 1.31 (t, *J* = 7.1 Hz, 3H), 1.26 (dd, *J* = 19.6, 7.8 Hz, 2H).

### 8-(trans-4-tert-Butoxycarbonylamino-cyclohexylamino)-6-pyridin-4-yl-imidazo[1,2-a]pyrazine-2-carboxylic acid ethyl ester (Intermediate LXV)

A mixture of Intermediate **LXIV** (280 mg, 0.580 mmol), pyridine-4-boronic acid pinacol ester (179 mg, 0.871 mmol), Pd(dppf)Cl₂ (47 mg, 0.058 mmol) and Cs₂CO₃ (378 mg, 1.161 mmol) in 1,4-dioxane (5.82 mL) and H₂O (0.73 mL) was heated at 110 ºC in a pressure tube for 20 h. An additional amount of pyridine-4-boronic acid 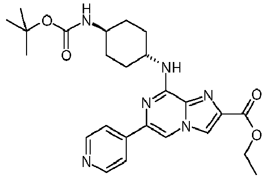 pinacol ester (50 mg), Pd(dppf)Cl₂ (20 mg) and Cs₂CO₃ (80 mg) was added to the reaction mixture, and it was heated at 110 ºC for 5 more hours. The reaction was partitioned between H₂O and DCM. The aqueous layer was extracted 3 times with DCM. The combined organic layers were dried over Na₂SO₄, filtered and concentrated. The crude was purified by column chromatography on silica gel using a solvent gradient from 0% to 10% of MeOH in DCM to give Intermediate **LXV** (yellow solid, 300 mg, 99%). LCMS (ESI): Rt = 3.80 min, m/z = 481.30 [M+ H]⁺. ¹H NMR (700 MHz, DMSO-d6) δ ppm 8.68 (dd, *J* = 4.5, 1.5 Hz, 2H), 8.58 (s, 1H), 8.48 (s, 1H), 7.90 (dd, *J* = 4.5, 1.6 Hz, 2H), 7.87 - 7.83 (m, 1H), 6.80 (d, *J* = 8.0 Hz, 1H), 4.35 (q, *J* = 7.1 Hz, 2H), 4.17 - 4.09 (m, 1H), 3.29 - 3.23 (m, 1H), 1.99 (d, *J* = 11.1 Hz, 2H), 1.85 (s, 2H), 1.60 (q, *J* = 11.2 Hz, 2H), 1.40 (s, 9H), 1.34 (t, *J =* 7.1 Hz, 4H).

### [4-(2-Carbamoyl-6-pyridin-4-yl-imidazo[1,2-a]pyrazin-8-yl-trans-amino)-cyclohexyl]-carbamic acid tert-butyl ester (Intermediate LXVI)

A mixture of Intermediate **LXV** (300 mg, 0.624 mmol) and NH₃ (7N in MeOH, 9 mL, 62.426 mmol) was heated at 100 ºC in a pressure tube for 18 h. The solvent was evaporated under vacuum to render Intermediate **LXVI** (282 mg, 99%). LCMS (ESI): Rt = 3.21 min, m/z = 452.30 [M+ H]⁺.

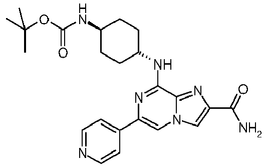

### 6-Bromo-8-(cis-4-tert-butoxycarbonylamino-cyclohexylamino)-imidazo[1,2-a]pyrazine-2-carboxylic acid ethyl ester (Intermediate LXVII)

A mixture of 6,8-dibromo-imidazo[1,2-a]pyrazine-2-carboxylic acid ethyl ester (300 mg, 0.860 mmol), 1-N-Boc-cis-1,4-cyclohexyldiamine (276 mg, 1.289 mmol) and DIPEA (0.23 mL, 1.289 mmol) in 1,2-DCE (9 mL) was heated at 110 ºC in a pressure tube for 4 h. The solvent was evaporated under vacuum. The crude was purified by column 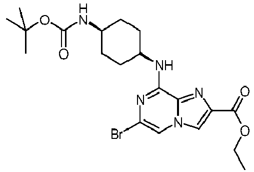 chromatography on silica gel using a solvent gradient from 0% to 10% of MeOH in DCM to give Intermediate **LXVII** (yellow solid, 398 mg, 96%). LCMS (ESI): Rt = 4.88 min, m/z = 482.00/484.00 [M+ H]⁺. ¹H NMR (700 MHz, DMSO-*d6*) δ ppm 8.41 (s, 1H), 8.00 (s, 1H), 7.56 (d, J= 6.8 Hz, 1H), 6.59 (d, *J* = 7.1 Hz, 1H), 4.33 (q, *J* = 7.1 Hz, 2H), 4.07 - 4.00 (brs, 1H), 3.54 (brs, 1H), 1.90 - 1.83 (m, 2H), 1.80 - 1.73 (m, 1H), 1.66 (dd, *J* = 13.8, 10.2 Hz, 3H), 1.56 (t, *J* = 9.4 Hz, 2H), 1.40 (s, 9H), 1.32 (t, *J* = 7.1 Hz, 3H).

### 8-(cis-4-tert-Butoxycarbonylamino-cyclohexylamino)-6-pyridin-4-yl-imidazo[1,2-a]pyrazine-2-carboxylic acid ethyl ester (Intermediate LXVIII)

A mixture of Intermediate **LXVII** (390 mg, 0.808 mmol), pyridine-4-boronic acid pinacol ester (249 mg, 1.213 mmol), Pd(dppf)Cl₂ (66 mg, 0.081 mmol) and Cs₂CO₃ (527 mg, 1.617 mmol) in 1,4-dioxane (8 mL) and H₂O (1 mL) was heated at 110 ºC in a pressure tube for 3 h. The reaction was partitioned between H₂O and DCM. The aqueous 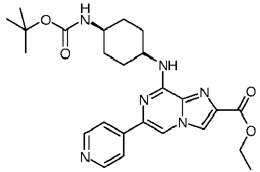 layer was extracted 3 times with DCM. The combined organic layers were dried over Na₂SO₄, filtered and concentrated. The crude was purified by column chromatography on silica gel using a solvent gradient from 0% to 10% of MeOH in DCM to give Intermediate **LXVIII** (yellow solid, 388 mg, 99%). LCMS (ESI): Rt = 3.86 min, m/z = 481.30 [M+ H]⁺. ¹H NMR (700 MHz, DMSO-d6) δ ppm 8.67 (d, *J* = 4.9 Hz, 2H), 8.60 (s, 1H), 8.48 (s, 1H), 7.91 (d, *J* = 5.8 Hz, 2H), 7.24 (d, *J =* 6.8 Hz, 1H), 6.68 (d, *J* = 7.4 Hz, 1H), 4.35 (q, *J* = 7.1 Hz, 2H), 4.27 (s, 1H), 3.56 (s, 1H), 2.00 - 1.92 (m, 2H), 1.79 - 1.71 (m, 2H), 1.71 - 1.60 (m, 4H), 1.41 (s, 9H), 1.34 (t, *J =* 7.1 Hz, 3H).

### [4-(2-Carbamoyl-6-pyridin-4-yl-imidazo[1,2-a]pyrazin-8-ylcis-amino)-cyclohexyl]-carbamic acid tert-butyl ester (Intermediate LXIX)

A mixture of Intermediate **LXVIII** (388 mg, 0.807 mmol) and NH₃ (7N in MeOH, 12 mL, 80.738 mmol) was heated at 100 ºC in a pressure tube for 18 h. The solvent was evaporated under vacuum to render Intermediate **LXIX** (364 mg, 99%). LCMS (ESI): Rt = 3.33 min, m/z = 452.30 [M+ H]⁺.

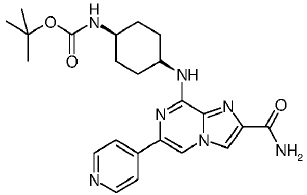

### 6-Bromo-8-[trans-4-(tert-butoxycarbonylamino-methyl)-cyclohexylamino]-imidazo[1,2-a]pyrazine-2-carboxylic acid ethyl ester (Intermediate LXX)

A mixture of 6,8-dibromo-imidazo[1,2-a]pyrazine-2-carboxylic acid ethyl ester (400 mg, 1.146 mmol), tert-butyl trans-4-aminocyclohexylmethylcarbamate (393 mg, 1.719 mmol) and DIPEA (0.30 mL, 1.719 mmol) in 1,2-DCE (11 mL) was heated at 110 ºC in a pressure tube for 4 h. The solvent was evaporated under vacuum. The crude was purified by column chromatography on silica gel using a solvent gradient from 0% 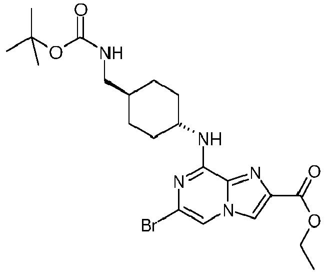 to 10% of MeOH in DCM to give Intermediate **LXX** (yellow solid, 584 mg, 99%). LCMS (ESI): Rt = 4.90 min, m/z = 496.20/498.20 [M+ H]⁺. ¹H NMR (300 MHz, CDCl₃) δ ppm 7.98 (s, 1H), 7.52 (s, 1H), 6.38 (s, 1H), 4.65 (s, 1H), 4.44 (q, *J* = 6.8 Hz, 2H), 4.05 (d, *J* = 7.8 Hz, 1H), 3.01 (t, *J* = 5.8 Hz, 2H), 2.16 (d, *J =* 11.9 Hz, 2H), 1.84 (d, *J* = 12.3 Hz, 2H), 1.45 - 1.38 (m, 12H), 1.33 - 1.06 (m, 5H).

### 8-[trans-4-(tert-Butoxycarbonylamino-methyl)-cyclohexylamino]-6-pyridin-4-yl-imidazo[1,2-a]pyrazine-2-carboxylic acid ethyl ester (Intermediate LXXI)

A mixture of Intermediate **LXX** (300 mg, 0.604 mmol), pyridine-4-boronic acid pinacol ester (186 mg, 0.907 mmol), Pd(dppf)Cl₂ (49 mg, 0.060 mmol) and Cs₂CO₃ (394 mg, 1.209 mmol) in 1,4-dioxane (6 mL) and H₂O (0.80 mL) was heated at 100 ºC in a pressure tube for 5 h. The reaction was partitioned between H₂O and DCM. The aqueous layer was extracted 3 times with DCM. The combined 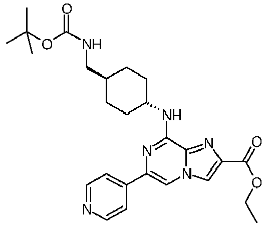 organic layers were dried over Na₂SO₄, filtered and concentrated. The crude was purified by column chromatography on silica gel using a solvent gradient from 0% to 10% of MeOH in DCM to give the desired Intermediate **LXXI** (yellow solid, 129 mg, 43%). LCMS (ESI): Rt = 3.90 min, m/z = 495.40 [M+ H]⁺. ¹H NMR (300 MHz, CDCl₃) δ ppm 8.69 (d, *J* = 5.6 Hz, 2H), 8.12 (s, 1H), 7.98 (s, 1H), 7.85 (d, *J* = 4.6 Hz, 2H), 6.24 (d, *J =* 7.9 Hz, 1H), 4.70 (brs, 1H), 4.47 (q, *J =* 7.1 Hz, 2H), 4.24 - 4.15 (m, 1H), 3.05 (t, *J* = 6.3 Hz, 2H), 2.26 (d, *J* = 10.0 Hz, 2H), 1.90 (d, *J* = 12.7 Hz, 2H), 1.50 - 1.39 (m, 15H), 1.34 (d, *J* = 12.5 Hz, 2H).

### [4-(2-Carbamoyl-6-pyridin-4-yl-imidazo[1,2-a]pyrazin-8-yl-trans-amino)-cyclohexylmethyl]-carbamic acid tert-butyl ester (Intermediate LXXII)

A mixture of Intermediate **LXXI** (129 mg, 0.261 mmol) and NH₃ (7N in MeOH, 4 mL, 26.082 mmol) was heated at 100 ºC in a pressure tube for 18 h. The solvent was evaporated under vacuum to render Intermediate **LXXII** (121 mg, 99%). LCMS (ESI): Rt = 3.39 min, m/z = 466.40 [M+ H]⁺.

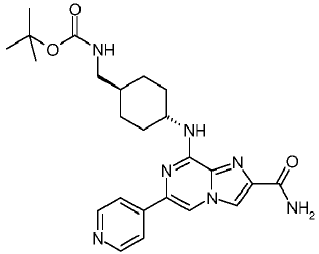

### 6-(2-Amino-pyridin-4-yl)-8-[(1-tert-butoxycarbonyl-4-fluoro-piperidin-4-ylmethyl)-amino]-imidazo[1,2-a]pyrazine-2-carboxylic acid ethyl ester (Intermediate LXXIII)

A mixture of Intermediate **XXIX** (205 mg, 0.410 mmol), 2-aminopyridine-4-boronic acid pinacol ester (135 mg, 0.615 mmol), Pd(dppf)Cl₂ (33 mg, 0.041 mmol) and Cs₂CO₃ (267 mg, 0.819 mmol) in 1,2-dioxane (4 mL) and H₂O (0.50 mL) was heated at 110 ºC in a pressure tube for 4 h. An additional amount of 2-aminopyridine-4-boronic acid pinacol ester (50 mg), Pd(dppf)Cl₂ (15 mg) and Cs₂CO₃ (100 mg) was added to the reaction mixture and it was heated at 110 ºC for 16 h. The mixture was partitioned between H₂O and EtOAc. The aqueous layer was extracted 3 times with 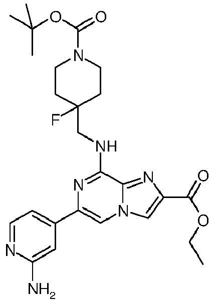 EtOAc. The combined organic layers were dried over Na₂SO₄, filtered and concentrated. The crude product was purified by column chromatography on silica gel using a solvent gradient from 50% to 100% of EtOAc in cHex and then from 0% to 15% of MeOH in EtOAc to afford Intermediate **LXXIII** (200 mg, 95%). LCMS (ESI): Rt = 3.62 min, m/z = 514.30 [M+ H]⁺.

### 4-{[6-(2-Amino-pyridin-4-yl)-2-carbamoyl-imidazo[1,2-a]pyrazin-8-ylamino]-methyl}-4-fluoro-piperidine-1-carboxylic acid tert-butyl ester (Intermediate LXXIV)

A mixture of Intermediate **LXXIII** (25 mg, 0.049 mmol) and NH₃ (7N in MeOH, 0.70 mL, 4.868 mmol) was heated in a pressure tube at 100 ºC for 48 h. The solvent was evaporated under vacuum. The crude was purified by column chromatography on silica gel using a solvent gradient from 0% to 15% of MeOH in EtOAc to render Intermediate **LXXIV** (16 mg, 68%). LCMS (ESI): Rt = 3.18 min, m/z = 485.20 [M+ H]⁺.

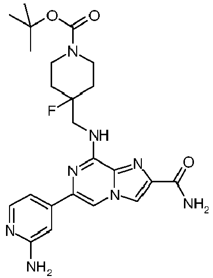

### 6-Bromo-8-[trans(4-tert-butoxycarbonylamino-cyclohexylmethyl)-amino]-imidazo[1,2-a]pyrazine-2-carboxylic acid ethyl ester (Intermediate LXXV)

A mixture of 6,8-dibromo-imidazo[1 ,2-a]pyrazine-2-carboxylic acid ethyl ester (400 mg, 1.146 mmol), tert-butyl (trans-4-(aminomethyl)cyclohexyl)carbamate (393 mg, 1.719 mmol) and DIPEA (0.30 mL, 1.719 mmol) in 1,2-DCE (11 mL) was heated at 110 ºC in a pressure tube for 4 h. The solvent was evaporated under vacuum. The crude was purified by column chromatography on silica gel using a solvent gradient from 0% to 10% of MeOH in DCM to give Intermediate **LXXV** (yellow solid, 520 mg, 91%). LCMS (ESI): Rt = 4.91 min, m/z = 496.00/498.00 [M+ H]⁺. ¹H NMR 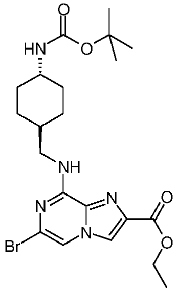 (300 MHz, CDCl₃) δ ppm 7.98 (s, 1H), 7.53 (s, 1H), 6.45 (s, 1H), 4.46 - 4.38 (m, 3H), 3.41 (t, *J* = 6.0 Hz, 2H), 2.02 (s, 2H), 1.86 (s, 2H), 1.46 - 1.35 (m, 12H), 1.09 (t, *J =* 11.0 Hz, 5H).

### 8-[trans-(4-tert-Butoxycarbonylamino-cyclohexylmethyl)-amino]-6-pyridin-4-yl-imidazo[1,2-a]pyrazine-2-carboxylic acid ethyl ester (Intermediate LXXVI)

A mixture of Intermediate **LXXV** (300 mg, 0.604 mmol), pyridine-4-boronic acid pinacol ester (186 mg, 0.907 mmol), Pd(dppf)Cl₂ (49 mg, 0.060 mmol) and Cs₂CO₃ (394 mg, 1.209 mmol) in 1,4-dioxane (6 mL) and H₂O (0.80 mL) was heated at 110 ºC in a pressure tube for 4 h. The reaction was partitioned between H₂O and DCM. The aqueous layer was extracted 3 times with DCM. The combined organic layers were dried over Na₂SO₄, filtered and concentrated. The crude was purified by column chromatography on silica 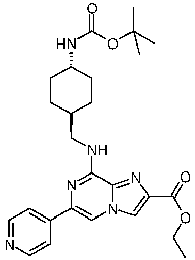 gel using a solvent gradient from 0% to 10% of MeOH in DCM to give the Intermediate **LXXVI** (yellow solid, 270 mg, 90%). LCMS (ESI): Rt = 3.91 min, m/z = 495.30 [M+ H]⁺. ¹H NMR (300 MHz, CDCl₃) δ ppm 8.68 (s, 2H), 8.13 (s, 1H), 7.98 (s, 1H), 7.85 (s, 2H), 6.62 (t, *J* = 5.7 Hz, 1H), 4.53 - 4.38 (m, 3H), 3.54 (t, *J* = 5.8 Hz, 2H), 2.04 (d, *J* = 9.9 Hz, 2H), 1.94 (d, *J* = 10.9 Hz, 2H), 1.26 - 1.23 (m, 15H), 1.13 (d, *J* = 10.8 Hz, 3H).

### 4-[(2-Carbamoyl-6-pyridin-4-yl-imidazo[1,2-a]pyrazin-8-yl-trans-amino)-methyl]-cyclohexyl}-carbamic acid tert-butyl ester (Intermediate LXXVII)

A mixture of Intermediate **LXXVI** (270 mg, 0.546 mmol) and NH₃ (7N in MeOH, 8 mL, 54.590 mmol) was heated at 100 ºC in a pressure tube for 18 h. The solvent was evaporated under vacuum to render Intermediate **LXXVII** (254 mg, 99%). LCMS (ESI): Rt = 3.34 min, m/z = 466.30 [M+ H]⁺.

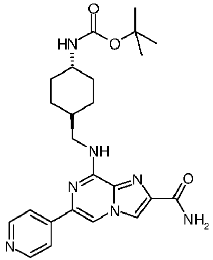

### 8-[(1-tert-Butoxycarbonyl-4-fluoro-piperidin-4-ylmethyl)-amino]-6-(2-chloro-pyridin-4-yl)-imidazo[1,2-a]pyrazine-2-carboxylic acid ethyl ester (Intermediate LXXVIII)

A mixture of Intermediate **XXIX** (200 mg, 0.400 mmol), 2-chloropyridine-4-boronic acid (94 mg, 0.600 mmol), Pd(dppf)Cl₂ (33 mg, 0.040 mmol) and Cs₂CO₃ (260 mg, 0.799 mmol) in 1,4-dioxane (4 mL) and H₂O (0.25 mL) was heated at 110 ºC in a pressure tube for 3 h. The mixture was partitioned between EtOAc and H₂O. The aqueous layer was extracted 3 times with EtOAc. The combined organic layers were dried over Na₂SO₄, filtered and concentrated. The crude product was purified by column chromatography 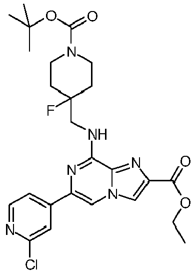 on silica gel using a solvent gradient from 0% to 60% of EtOAc in cHex to yield Intermediate **LXXVIII** (yellow solid, 170 mg, 80%). LCMS (ESI): Rt = 5.02 min, m/z = 433.30 [M+ H-Boc]⁺.

### 6-Bromo-8-[(1-tert-butoxycarbonyl-4-hydroxy-piperidin-4-ylmethyl)-amino]-imidazo[1,2-a]pyrazine-2-carboxylic acid ethyl ester (Intermediate LXXX)

A mixture of 6,8-dibromo-imidazo[1,2-a]pyrazine-2-carboxylic acid ethyl ester (50 mg, 0.143 mmol), tert-butyl 4-(aminomethyl)-4-hydroxypiperidine-1-carboxylate (49 mg, 0.215 mmol) and DIPEA (0.03 mL, 0.215 mmol) in 1,2-DCE (1.43 mL) was heated at 110 ºC in a pressure tube for 4 h. The solvent was evaporated under vacuum. The crude was purified by column chromatography on silica gel using a solvent gradient from 0% to 10% of MeOH 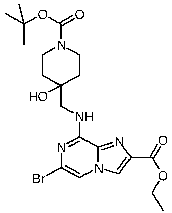 in DCM to give Intermediate **LXXX** (yellow solid, 60 mg, 84%). LCMS (ESI): Rt = 4.62 min, m/z = 398.000/400.00 [M+ H-Boc]⁺. ¹H NMR (700 MHz, DMSO-d6) δ ppm 8.20 (s, 1H), 7.79 (s, 1H), 7.73 (t, *J* = 6.0 Hz, 1H), 4.71 (s, 1H), 4.11 (q, *J* = 7.1 Hz, 2H), 3.44 (s, 2H), 1.24 (dt, *J* = 11.0, 8.6 Hz, 5H), 1.16 (s, 9H), 1.10 (t, *J* = 7.1 Hz, 3H).

### 8-[(1-tert-Butoxycarbonyl-4-hydroxy-piperidin-4-ylmethyl)-amino]-6-pyridin-4-yl-imidazo[1,2-a]pyrazine-2-carboxylic acid ethyl ester (Intermediate LXXXI)

A mixture of Intermediate **LXXX** (60 mg, 0.120 mmol), pyridine-4-boronic acid pinacol ester (37 mg, 0.181 mmol), Pd(dppf)Cl₂ (10 mg, 0.012 mmol) and Cs₂CO₃ (78 mg, 0.241 mmol) in 1,4-dioxane (1.20 mL) and H₂O (0.15 mL) was heated at 110 ºC in a pressure tube for 2 h. The reaction was partitioned between H₂O and DCM. The aqueous layer was extracted 3 times with DCM. 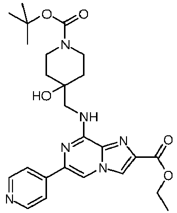 The combined organic layers were dried over Na₂SO₄, filtered and concentrated. The crude was purified by column chromatography on silica gel using a solvent gradient from 0% to 10% of MeOH in DCM to give the Intermediate **LXXXI** (yellow solid, 40 mg, 67%). LCMS (ESI): Rt = 3.57 min, m/z = 497.30 [M+ H]⁺.

### 4-[(2-Carbamoyl-6-pyridin-4-yl-imidazo[1,2-a]pyrazin-8-ylamino)-methyl]-4-hydroxy-piperidine-1-carboxylic acid tert-butyl ester (Intermediate LXXXII)

A mixture of Intermediate **LXXXI** (40 mg, 0.081 mmol) and NH₃ (7N in MeOH, 1.20 mL, 8.055 mmol) was heated at 100 ºC in a pressure tube for 18 h. The solvent was evaporated under vacuum to render Intermediate **LXXXII** (38 mg, 99%). LCMS (ESI): Rt = 2.92 min, m/z = 468.20 [M+ H]⁺.

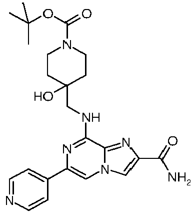

### 8-[(1-tert-Butoxycarbonyl-4-fluoro-piperidin-4-ylmethyl)-amino]-6-(1-methyl-1H-pyrazol-4-yl)-imidazo[1,2-a]pyrazine-2-carboxylic acid ethyl ester (Intermediate LXXXIII)

Intermediate **XXIX** (100 mg, 0.199 mmol), 1-methyl-4-(4,4,5,5-tetramethyl-1,3,2-dioxaborolan-2-yl)-1H-pyrazole (62 mg, 0.299 mmol), Pd(dppf)Cl₂ (16 mg, 0.020 mmol) and Cs₂CO₃ (130 mg, 0.398 mmol) in 1,2-dioxane (2 mL) and H₂O (0.13 mL) was heated at 100 ºC in a pressure tube for 24 h. The reaction mixture was partitioned between EtOAc and H₂O. The aqueous layer was extracted 3 times with EtOAc. The combined organic layers were dried over Na₂SO₄, filtered and concentrated. The crude was purified by column 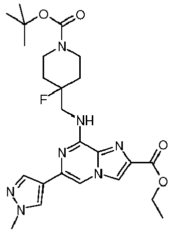 chromatography on silica gel using a solvent gradient from 0% to 100% of EtOAc in cHex to give Intermediate **LXXXIII** (off-white solid, 78 mg, 78%). LCMS (ESI): Rt = 4.68 min, m/z = 524.20 [M+ Na]⁺. ¹H NMR (300 MHz, CDCl₃) δ ppm 8.03 (s, 1H), 7.78 (s, 1H), 7.76 (s, 1H), 7.60 (s, 1H), 6.50 (t, *J =* 6.0 Hz, 1H), 4.44 (q, *J* = 7.1 Hz, 2H), 3.94 (s, 3H), 3.88 (dd, *J* = 14.3, 6.5 Hz, 2H), 3.10 (t, *J* = 12.0 Hz, 2H), 1.90 (t, *J* = 11.6 Hz, 2H), 1.80 - 1.56 (m, 2H), 1.45 - 1.38 (m, 12H).

### 4-{[2-Carbamoyl-6-(1-methyl-1H-pyrazol-4-yl)-imidazo[1,2-a]pyrazin-8-ylamino]-methyl}-4-fluoro-piperidine-1-carboxylic acid tert-butyl ester (Intermediate LXXXIV)

A mixture of Intermediate **LXXXIII** (50 mg, 0.100 mmol) and NH₃ (7N in MeOH, 2 mL) was heated at 100 ºC in a pressure tube for 48 h. The mixture was evaporated under vacuum to give Intermediate **LXXXIV** (light yellow solid, 30 mg, 64%). LCMS (ESI): Rt = 4.06 min, m/z = 473.20 [M+ H]⁺. ¹H NMR (300 MHz, CDCl₃) δ ppm 8.04 (s, 1H), 7.81 (s, 1H), 7.78 (s, 1H), 7.64 (s, 1H), 7.11 (s, 1H), 6.21 (t, *J* = 6.4 Hz, 1H), 5.62 (s, 1H), 4.01 - 3.87 (m, 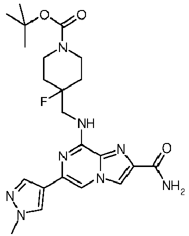 6H), 3.14 (t, *J* = 11.9 Hz, 2H), 1.94 (t, *J* = 11.5 Hz, 2H), 1.83 - 1.60 (m, 3H), 1.45 (s, 9H).

### 8-[(1-tert-Butoxycarbonyl-4-fluoro-piperidin-4-ylmethyl)-amino]-6-isothiazol-5-yl-imidazo[1,2-a]pyrazine-2-carboxylic acid ethyl ester (Intermediate LXXXV)

A mixture of Intermediate **XXIX** (100 mg, 0.200 mmol), 5-(4,4,5,5-tetramethyl-1,3,2-dioxaborolan-2-yl)isothiazole (63 mg, 0.300 mmol), Pd(dppf)Cl₂ (16 mg, 0.020 mmol) and Cs₂CO₃ (130 mg, 0.400 mmol) in 1,4-dioxane (2 mL) and H₂O (0.13 mL) was heated at 100 ºC in a pressure tube for 24 h. An additional amount of 5-(4,4,5,5-tetramethyl-1,3,2-dioxaborolan-2-yl)isothiazole (0.5 eq) was added to the reaction mixture and it was heated at 100 ºC for 24 more 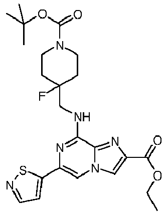 hours. The reaction mixture was partitioned between EtOAc and H₂O. The aqueous phase was extracted 3 times with EtOAc. The combined organic layers were dried over Na₂SO₄, filtered and concentrated. The crude was purified by column chromatography on silica gel using a solvent gradient from 0% to 100% of EtOAc in cHex to give Intermediate **LXXXV** (off-white solid, 20 mg, 20%). LCMS (ESI): Rt = 4.79 min, m/z = 505.10 [M+ H]⁺. ¹H NMR (300 MHz, CDCl₃) δ ppm 8.50 (s, 1H), 8.13 (s, 1H), 7.91 (s, 1H), 7.47 (s, 1H), 6.98 (t, *J* = 6.6 Hz, 1H), 4.48 (q, *J* = 7.1 Hz, 2H), 4.01 - 3.86 (m, 4H), 3.20 - 3.06 (m, 2H), 1.99 - 1.89 (m, 2H), 1.76 - 1.62 (m, 2H), 1.47 - 1.42 (m, 12H).

### 4-[(2-Carbamoyl-6-isothiazol-5-yl-imidazo[1,2-a]pyrazin-8-ylamino)-methyl]-4-fluoro-piperidine-1-carboxylic acid tert-butyl ester (Intermediate LXXXVI)

A mixture of Intermediate **LXXXV** (20 mg, 0.040 mmol) and NH₃ (7N in MeOH, 2 mL) was heated at 100 ºC in a pressure tube for 48 h. The reaction mixture was evaporated under vacuum to render Intermediate **LXXXVI** (light yellow solid, 15 mg, 80%). ¹H NMR (300 MHz, CDCl₃) δ ppm 8.49 (s, 1H), 8.14 (s, 1H), 7.95 (s, 1H), 7.47 (s, 1H), 7.16 (s, 1H), 6.40 (s, 1H), 5.81 (s, 1H), 3.94 (dd, *J* = 19.4, 4.3 Hz, 4H), 3.16 (t, *J* = 11.8 Hz, 2H), 1.98 - 1.88 (m, 2H), 1.86 - 1.63 (m, 2H), 1.45 (s, 9H).

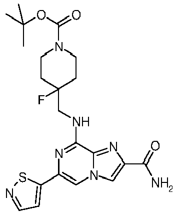

### 8-[(1-tert-Butoxycarbonyl-4-fluoro-piperidin-4-ylmethyl)-amino]-6-(1H-pyrazol-4-yl)-imidazo[1,2-a]pyrazine-2-carboxylic acid ethyl ester (Intermediate LXXXVII)

A mixture of Intermediate **XXIX** (100 mg, 0.200 mmol), 4-(4,4,5,5-tetramethyl-1,3,2-dioxaborolan-2-yl)-1H-pyrazole (58 mg, 0.300 mmol), Pd(dppf)Cl₂ (16 mg, 0.020 mmol) and Cs₂CO₃ (130 mg, 0.400 mmol) in 1,4-dioxane (2 mL) and H₂O (0.13 mL) was heated at 100 ºC in a pressure tube for 16 h. An additional amount of 4-(4,4,5,5-tetramethyl-1,3,2-dioxaborolan-2-yl)-1H-pyrazole (0.2 eq) was added to the reaction and it was heated at 100 ºC for 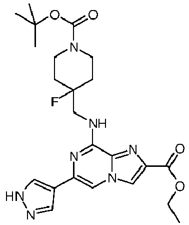 24 h. The reaction mixture was partitioned between EtOAc and H₂O. The aqueous phase was extracted 3 times with EtOAc. The combined organic layers were dried over Na₂SO₄, filtered and concentrated. The crude was purified by column chromatography on silica gel using a solvent gradient from 0% to 70% of EtOAc in cHex to give Intermediate **LXXXVII** (15 mg, 15%). LCMS (ESI): Rt = 4.30 min, m/z = 388.20 [M+ H-Boc]⁺.

### 4-{[2-Carbamoyl-6-(1H-pyrazol-4-yl)-imidazo[1,2-a]pyrazin-8-ylamino]-methyl}-4-fluoro-piperidine-1-carboxylic acid tert-butyl ester (Intermediate LXXXVIII)

A mixture of Intermediate **LXXXVII** (17 mg, 0.035 mmol) and NH₃ (7N in MeOH, 2 mL) was heated at 100 ºC in a pressure tube for 48 h. The reaction mixture was evaporated under vacuum to render Intermediate **LXXXVIII,** which was used in the next step without further purification (10 mg, 63%). LCMS (ESI): Rt = 3.85 min, m/z = 459.10 [M+ H]⁺. ¹H NMR (300 MHz, CDCl₃) δ ppm 8.07 (s, 1H), 7.98 (s, 2H), 7.69 (s, 1H), 7.13 (s, 1H), 6.23 (s, 1H), 5.74 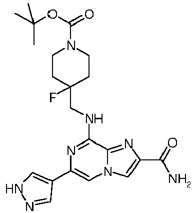 (s, 1H), 3.94 (dd, *J* = 20.6, 6.0 Hz, 4H), 3.16 (t, *J* = 11.8 Hz, 2H), 2.02 - 1.87 (m, 2H), 1.86 - 1.61 (m, 2H), 1.45 (s, 9H).

### 6-Bromo-8-(cis 4-hydroxy-4-methyl-cyclohexylamino)-imidazo[1,2-a]pyrazine-2-carboxylic acid ethyl ester (Intermediate IXC)

A mixture of 6,8-dibromo-imidazo[1,2-a]pyrazine-2-carboxylic acid ethyl ester (490 mg, 1.404 mmol) and cis-4-amino-1-methyl-cyclohexanol (272 mg, 2.106 mmol) in EtOH (5 mL) was heated at 100 ºC in a pressure tube for 4 h. An additional amount of amine (0.5 eq) was added to the reaction mixture and it was heated for 24 more hours. The mixture was evaporated 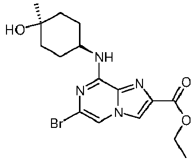 under vacuum. The crude was purified by column chromatography on silica gel using a solvent gradient from 0% to 100% of EtOAc in cHex to afford Intermediate **IXC** (off-white solid, 340 mg, 61%). LCMS (ESI): Rt = 4.40 min, m/z = 397.00/399.00 [M+ H]⁺. ¹H NMR (300 MHz, DMSO-d6) δ ppm 8.38 (s, 1H), 8.10 (d, *J =* 8.0 Hz, 1H), 7.94 (s, 1H), 4.31 (q, *J* = 7.1 Hz, 2H), 3.88 (ddd, *J =* 14.4, 10.5, 2.4 Hz, 1H), 1.85 (dd, *J* = 22.5, 11.8 Hz, 2H), 1.58 (d, *J* = 12.6 Hz, 4H), 1.40 (dd, *J =* 13.4, 3.5 Hz, 2H), 1.30 (t, *J* = 7.1 Hz, 3H), 1.11 (s, 3H).

### 6-(3-Fluoro-pyridin-4-yl)-8-(cis, 4-hydroxy-4-methyl-cyclohexylamino)-imidazo[1,2-a]pyrazine-2-carboxylic acid ethyl ester (Intermediate XC)

A mixture of Intermediate **IXC** (150 mg, 0.378 mmol), 3-fluoropyridine-4-boronic acid pinacol ester (126 mg, 0.566 mmol), Pd(dppf)Cl₂ (31 mg, 0.038 mmol) and Cs₂CO₃ (246 mg, 0.755 mmol) in 1,4-dioxane (2 mL) and H₂O (0.13 mL) was heated at 100 ºC in a pressure tube for 4 h. The reaction mixture was partitioned between EtOAc and H₂O. The aqueous phase was 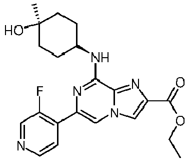 extracted 3 times with EtOAc. The combined organic layers were dried over Na₂SO₄, filtered and concentrated. The crude was purified by column chromatography on silica gel using a solvent gradient from 0% to 100% of EtOAc in cHex to give Intermediate **XC** (100 mg, 64%). LCMS (ESI): Rt = 4.29 min, m/z = 414.20 [M+ H]⁺. ¹H NMR (300 MHz, CDCl₃) δ ppm 8.52 (d, *J* = 3.9 Hz, 2H), 8.19 (s, 1H), 8.16 (d, *J* = 5.3 Hz, 1H), 8.11 (s, 1H), 6.57 (d, *J* = 7.8 Hz, 1H), 4.45 (q, *J* = 7.1 Hz, 2H), 4.22 - 4.08 (m, 1H), 2.03 (d, *J* = 9.3 Hz, 2H), 1.85 (dd, *J* = 19.1, 10.3 Hz, 4H), 1.62 (td, *J* = 13.9, 3.5 Hz, 2H), 1.41 (t, *J* = 7.1 Hz, 3H), 1.35 (s, 3H).

### 8-[(1-tert-Butoxycarbonyl-4-fluoro-piperidin-4-ylmethyl)-amino]-6-thiazol-5-yl-imidazo[1,2-a]pyrazine-2-carboxylic acid ethyl ester (Intermediate XCI)

A mixture of Intermediate **XXIX** (100 mg, 0.200 mmol), 5-(4,4,5,5-tetramethyl-1,3,2-dioxaborolan-2-yl)thiazole (39 mg, 0.300 mmol), Pd(dppf)Cl₂ (16 mg, 0.020 mmol) and Cs₂CO₃ (130 mg, 0.400 mmol) in 1,4-dioxane (2 mL) and H₂O (0.13 mL) was heated at 100 ºC in a pressure tube for 24 h. An additional amount of 5-(4,4,5,5-tetramethyl-1,3,2-dioxaborolan-2-yl)thiazole (39 mg) and Pd(dppf)Cl₂ (16 mg) was added to the reaction mixture, and it was heated at 100 ºC for 48 more hours. The reaction mixture was partitioned between 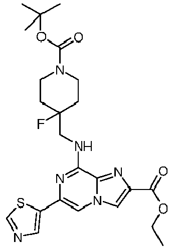 EtOAc and H₂O. The aqueous phase was extracted 3 times with EtOAc. The combined organic layers were dried over Na₂SO₄, filtered and concentrated. The crude was purified by column chromatography on silica gel using a solvent gradient from 0% to 100% of EtOAc in cHex to afford Intermediate **XCI** (70 mg, 69%). LCMS (ESI): Rt = 4.60 min, m/z = 405.10 [M+ H-Boc]⁺. ¹H NMR (300 MHz, CDCl₃) δ ppm 8.79 (s, 1H), 8.22 (s, 1H), 8.10 (s, 1H), 7.85 (s, 1H), 6.64 (t, *J =* 6.3 Hz, 1H), 4.45 (q, *J* = 7.1 Hz, 2H), 3.98 - 3.84 (m, 4H), 3.12 (t, *J* = 11.1 Hz, 2H), 1.89 (t, *J* = 12.0 Hz, 2H), 1.82 - 1.58 (m, 2H), 1.45 - 1.38 (m, 12H).

### 4-[(2-Carbamoyl-6-thiazol-5-yl-imidazo[1,2-a]pyrazin-8-ylamino)-methyl]-4-fluoro-piperidine-1-carboxylic acid tert-butyl ester (Intermediate XCII)

A mixture of Intermediate **XCI** (70 mg, 0.139 mmol) and NH₃ (7N in MeOH, 2 mL) was heated at 100 ºC in a pressure tube for 48 h. The mixture was evaporated under vacuum to render Intermediate **XCII** (30 mg, 45%), which was used in the next step without further purification. LCMS (ESI): Rt = 4.22 min, m/z = 376.10 [M+ H-Boc]⁺.

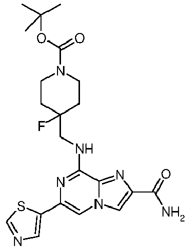

### 8-[(1-tert-Butoxycarbonyl-4-fluoro-piperidin-4-ylmethyl)-amino]-6-(3-fluoro-pyridin-4-yl)-imidazo[1,2-a]pyrazine-2-carboxylic acid ethyl ester (Intermediate XCIII)

A mixture of Intermediate **XXIX** (150 mg, 0.300 mmol), 3-fluoropyridine-4-boronic acid pinacol ester (100 mg, 0.450 mmol), Pd(dppf)Cl₂ (24 mg, 0.030 mmol) and Cs₂CO₃ (195 mg, 0.600 mmol) in 1,4-dioxane (2 mL) and H₂O (0.13 mL) was heated in a pressure tube at 100 ºC for 4 h. The reaction was partitioned between H₂O and DCM. The aqueous layer was extracted 3 times with DCM. The combined organic layers were dried over Na₂SO₄, 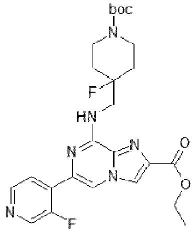 filtered and concentrated. The crude was purified by column chromatography on silica gel using a solvent gradient from 0% to 100% of EtOAc in cHex to afford Intermediate **XCIII** (100 mg, 64%). LCMS (ESI): Rt = 4.73 min, m/z = 517.30 [M+ H]⁺.

### B. Biological tests

**HASPIN biochemical Assay:** The biochemical assay to measure HASPIN activity relies on the ADP-Glo^{™} assay kit (Promega) that determines the amount of ADP as direct product of the kinase enzyme activity. Assay conditions were as indicated by the kit manufacturers with the following adaptations for the kinase activity step: Kinase assay buffer and assay volume (15 mM HEPES pH 7.5, 20 mM NaCl, 1 mM EGTA, 0.02% TWEEN 20, 10 mM MgCl₂, 0.1 mg/mL BGG)/25 µL assay volume). Incubation time and temperature: 60 min at 30 °C. HASPIN final concentration: 0.9 µg/mL. ATP final concentration: 150 µM. HASPIN autophosphorylation was measured. Assays were performed in 384-well plates. The final outcome of the coupled reactions provided by the kit is the release of luciferase and has been measured with a multilabel HTS counter Victor V / Envision. Values were normalized against the control activity included (100% HASPIN activity, without compound). Values were plotted against the inhibitor concentration and fit to a sigmoid dose-response curve using Activity base by IDBS software.

**Cellular HASPIN Inhibition Assay (phosphorylation of H3T3):** Compounds can be screened for their ability to inhibit intracellular HASPIN using a western blot assay to detect phosphorylation of the HASPIN substrate H3T3 in synchronized cells. MV4:11 cells are plated at 400000 cells per well in 6-well plates in RPMI media supplemented with 10% foetal bovine serum, Penicillin/Streptomycin solution diluted 1: 100, and fungizone, and allowed to adhere overnight at 37 °C in 5% CO₂. Then, compounds are added to the cell media from a final concentration of 10 µM in 10-fold serial dilutions and the cells are incubated at 37 °C in 5% CO₂. After 8 hours of treatment with the compounds, the cells are washed in PBS, lysed adding 100 µl of protein lysis buffer (62.5 mM Tris pH 6.8 al 6.25%, 2% SDS y 10% glycerol) incubation 10 minutes at room temperature and heating at 95 ºC 10 min. The protein content of the lysates is determined by DC protein assay. The proteins are resolved by SDS-PAGE and transferred to nitrocellulose membrane. The membranes are incubated overnight at 4 ºC with antibodies specific for H3, phosphothreonine-3 H3 they are washed and then incubated with IRDye800 conjugated antimouse and Alexa Fluor 680 goat anti-rabbit IgG secondary antibodies. The bands are visualized and quantified using an Odyssey infrared imaging system. The percentage of H3 phosphorylation is finally plotted against concentration for each compound and EC₅₀s for intracellular HASPIN inhibition are calculated using a software platform for screening data management.

Compounds of the invention were found to inhibit HASPIN, as tested in the biochemical assay described hereinbefore, with IC₅₀ activities below 5 µM. Biological activity in HASPIN is represented in Table 1.

Table 1: Inhibition of HASPIN activity expressed as IC50 (biochemical) and EC50 (cellular) values [M] for some compounds of the examples.

| **Compound number** | **HASPIN IC₅₀** | **pH3T3 EC₅₀** | **Compound number** | **HASPIN IC₅₀** | **pH3T3 EC₅₀** |
|---|---|---|---|---|---|
| 2 | 3.00E-08 | | 24 | 1.11 E-07 | |
| 3 | 4.52E-08 | < 5.0E-08 | 25 | 1.57E-07 | |
| 4 | 2.04E-08 | < 5.0E-08 | 27 | 9.42E-09 | < 5.0E-08 |
| 5 | 2.29E-07 | | 28 | 1.95E-08 | |
| 6 | 5.96E-08 | | 29 | 6.59E-09 | |
| 7 | 6.00E-08 | | 30 | 1.87E-07 | |
| 8 | 1.97E-08 | < 5.0E-08 | 31 | 1.89E-07 | |
| 9 | 1.80E-07 | | 32 | 7.63E-09 | < 5.0E-08 |
| 11 | 6.50E-08 | | 33 | 1.05E-08 | < 5.0E-08 |
| 12 | 2.53E-07 | | 35 | 9.48E-08 | |
| 14 | 7.80E-08 | | 36 | 2.46E-08 | < 5.0E-08 |
| 15 | 2.59E-08 | | 39 | 1.40E-07 | |
| 16 | 9.60E-08 | | 41 | 3.45E-08 | |
| 17 | 1.82E-08 | | 44 | 4.68E-08 | |
| 18 | 1.38E-07 | | 46 | 8.56E-08 | |
| 19 | 4.57E-08 | | | | |
| 20 | 7.88E-08 | | | | |
| 21 | 1.51 E-07 | | | | |

## Claims

1. A compound of formula (I): 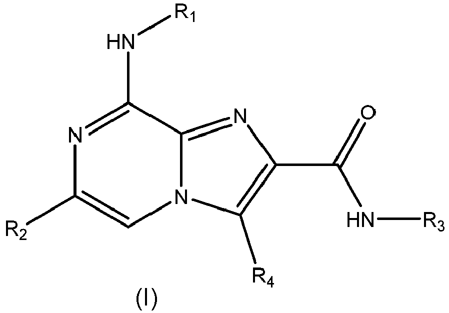 wherein:
R₁ is selected from the following groups:
- alkyl C₁-C₆ optionally substituted by cycloalkyl C₃-C₆ or heterocycloalkyl C₃-C₆, wherein these substituents may be substituted by a group selected from alkyl C₁-C₆, CN, OH, halo,
- cycloalkyl C₃-C₆ optionally substituted by a group selected from OH, NH₂, alkyl C₁-C₆ optionally substituted by NH₂
- heterocycloalkyl C₃-C₆;
R₂ is a C₅-C₆ heteroaryl optionally substituted by a group selected from NH₂, alkyl C₁-C₆ or halo;
R₃ is selected from H or alkyl C₁-C₆ optionally substituted by O-alkyl C₁-C₄;
R₄ is selected from H or alkyl C₁-C₆;
or a pharmaceutically acceptable ester, amide, solvate or salt thereof.

2. The compound according to claim 1, wherein R₁ is selected from the following groups: 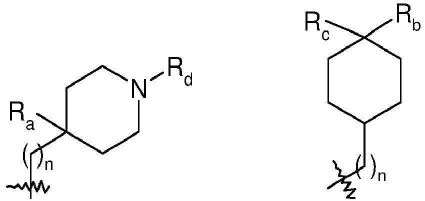 wherein:
n is selected from 0, 1 or 2;
Ra is selected from H, halo, CN, OH;
Rb is selected from H, OH, NH₂;
Rc is selected from H, alkyl C₁-C₄ optionally substituted by NH₂;
Rd is selected from H or alkyl C₁-C₄.

3. The compound according to any one of claims 1 or 2, wherein R₂ is selected from pyridyl, pyrazolyl, thiazolyl or isothiazolyl.

4. The compound according to claim 3, wherein R₂ is 4-pyridyl optionally substituted by halo, NH₂ or alkyl C₁-C₄.

5. The compound according to any one of claims 1 to 4, wherein R₃ is selected from H or alkyl C₁-C₄ optionally substituted by O-alkyl C₁-C₂.

6. The compound according to any one of claims 1 to 5, wherein R₄ is selected from H or alkyl C₁-C₄.

7. The compound according to claim 1, wherein said compound is selected from the following list:
- 8-(Piperidin-4-ylamino)-6-pyridin-4-yl-imidazo[1,2-a]pyrazine-2-carboxylic acid amide (1)
- 8-[(Piperidin-4-ylmethyl)-amino]-6-pyridin-4-yl-imidazo[1,2-a]pyrazine-2-carboxylic acid amide (2)
- 6-(2-Chloro-pyridin-4-yl)-8-[(piperidin-4-ylmethyl)-amino]-imidazo[1,2-a]pyrazine-2-carboxylic acid amide (3)
- 8-[(Piperidin-4-ylmethyl)-amino]-6-pyridin-4-yl-imidazo[1,2-a]pyrazine-2-carboxylic acid methylamide (4)
- 8-[(Piperidin-4-ylmethyl)-amino]-6-pyridin-4-yl-imidazo[1,2-a]pyrazine-2-carboxylic acid (2-methoxy-ethyl)-amide (5)
- 6-(2-Chloro-pyridin-4-yl)-8-[(4-cyano-piperidin-4-ylmethyl)-amino]-imidazo[1,2-a]pyrazine -2-carboxylic acid amide (6)
- 3-Methyl-8-[(piperidin-4-ylmethyl)-amino]-6-pyridin-4-yl-imidazo[1,2-a]pyrazine-2-carboxylic acid methylamide (7)
- 8-[(4-Cyano-piperidin-4-ylmethyl)-amino]-6-pyridin-4-yl-imidazo[1,2-a]pyrazine-2-carboxylic acid amide (8)
- 3-Methyl-8-[(piperidin-4-ylmethyl)-amino]-6-pyridin-4-yl-imidazo[1,2-a]pyrazine-2-carboxylic acid amide (9)
- 3-Methyl-8-[(piperidin-4-ylmethyl)-amino]-6-pyridin-4-yl-imidazo[1,2-a]pyrazine-2-carboxylic acid (2-methoxy-ethyl)-amide (10)
- 6-(2-Chloro-pyridin-4-yl)-3-methyl-8-[(piperidin-4-ylmethyl)-amino]-imidazo[1,2-a] pyrazine-2-carboxylic acid methylamide (11)
- 6-(2-Chloro-pyridin-4-yl)-3-methyl-8-[(piperidin-4-ylmethyl)-amino]-imidazo[1,2-a] pyrazine-2-carboxylic acid amide (12)
- 6-(2-Chloro-pyridin-4-yl)-3-methyl-8-[(piperidin-4-ylmethyl)-amino]-imidazo[1,2-a] pyrazine-2-carboxylic acid (2-methoxy-ethyl)-amide (13)
- 8-[(4-Fluoro-piperidin-4-ylmethyl)-amino]-3-methyl-6-pyridin-4-yl-imidazo[1,2-a]pyrazine-2-carboxylic acid methylamide (14)
- 8-[(4-Fluoro-piperidin-4-ylmethyl)-amino]-6-pyridin-4-yl-imidazo[1,2-a]pyrazine-2-carboxylic acid amide (15)
- 8-[(4-Fluoro-piperidin-4-ylmethyl)-amino]-3-methyl-6-pyridin-4-yl-imidazo[1,2-a]pyrazine-2-carboxylic acid amide (16)
- 8-[(4-Cyano-piperidin-4-ylmethyl)-amino]-3-methyl-6-pyridin-4-yl-imidazo [1,2-a]pyrazine-2-carboxylic acid methylamide (17)
- 8-[(4-Cyano-piperidin-4-ylmethyl)-amino]-3-methyl-6-pyridin-4-yl-imidazo[1,2-a]pyrazine-2-carboxylic acid (2-methoxy-ethyl)-amide (18)
- 8-[(4-Cyano-piperidin-4-ylmethyl)-amino]-3-methyl-6-pyridin-4-yl-imidazo[1,2-a]pyrazine-2-carboxylic acid amide (19)
- 6-(2-Chloro-pyridin-4-yl)-8-[(4-fluoro-piperidin-4-ylmethyl)-amino]-3-methyl-imidazo[1,2-a]pyrazine-2-carboxylic acid methylamide (20)
- 6-(2-Chloro-pyridin-4-yl)-8-[(4-cyano-piperidin-4-ylmethyl)-amino]-3-methyl-imidazo[1,2-a]pyrazine-2-carboxylic acid methylamide (21)
- 6-(2-Chloro-pyridin-4-yl)-8-[(4-fluoro-piperidin-4-ylmethyl)-amino]-3-methyl-imidazo[1,2-a]pyrazine-2-carboxylic acid (2-methoxy-ethyl)-amide (22)
- 6-(2-Chloro-pyridin-4-yl)-8-[(4-cyano-piperidin-4-ylmethyl)-amino]-3-methyl-imidazo[1,2-a]pyrazine-2-carboxylic acid (2-methoxy-ethyl)-amide (23)
- 6-(2-Chloro-pyridin-4-yl)-8-[(4-cyano-piperidin-4-ylmethyl)-amino]-3-methyl-imidazo[1,2-a]pyrazine-2-carboxylic acid amide (24)
- 6-(2-Chloro-pyridin-4-yl)-8-[(4-fluoro-piperidin-4-ylmethyl)-amino]-3-methyl-imidazo[1,2-a]pyrazine-2-carboxylic acid amide (25)
- 8-[(1-Propyl-piperidin-4-ylmethyl)-amino]-6-pyridin-4-yl-imidazo[1,2-a]pyrazine-2-carboxylic acid methylamide (26)
- 6-(3-Fluoro-pyridin-4-yl)-8-[(piperidin-4-ylmethyl)-amino]-imidazo[1,2-a]pyrazine-2-carboxylic acid amide (27)
- 6-(3-Fluoro-pyridin-4-yl)-8-[(piperidin-4-ylmethyl)-amino]-imidazo[1,2-a]pyrazine-2-carboxylic acid methylamide (28)
- 8-[(4-Cyano-piperidin-4-ylmethyl)-amino]-6-(3-fluoro-pyridin-4-yl)-imidazo[1,2-a]pyrazine-2-carboxylic acid amide (29)
- 6-(3-Chloro-pyridin-4-yl)-8-[(piperidin-4-ylmethyl)-amino]-imidazo[1,2-a]pyrazine-2-carboxylic acid amide (30)
- 6-(3-Chloro-pyridin-4-yl)-8-[(piperidin-4-ylmethyl)-amino]-imidazo[1,2-a]pyrazine-2-carboxylic acid methylamide (31)
- 8-[(4-Cyano-piperidin-4-ylmethyl)-amino]-6-(3-fluoro-pyridin-4-yl)-imidazo[1,2-a]pyrazine-2-carboxylic acid methylamide (32)
- 8-[(4-Fluoro-piperidin-4-ylmethyl)-amino]-6-(3-fluoro-pyridin-4-yl)-imidazo[1,2-a]pyrazine-2-carboxylic acid amide (33)
- 6-(3-Fluoro-pyridin-4-yl)-3-methyl-8-[(piperidin-4-ylmethyl)-amino]-imidazo[1,2-a]pyrazine-2-carboxylic acid methylamide (35)
- 6-(2-Methyl-pyridin-4-yl)-8-[(piperidin-4-ylmethyl)-amino]-imidazo[1,2-a]pyrazine-2-carboxylic acid amide (36)
- 8-(2-Piperidin-4-yl-ethylamino)-6-pyridin-4-yl-imidazo[1,2-a]pyrazine-2-carboxylic acid amide (37)
- 8-(4-trans-Amino-cyclohexylamino)-6-pyridin-4-yl-imidazo[1,2-a]pyrazine-2-carboxylic acid amide (38)
- 8-(cis-4-Amino-cyclohexylamino)-6-pyridin-4-yl-imidazo[1,2-a]pyrazine-2-carboxylic acid amide (39)
- 8-(trans-4-Aminomethyl-cyclohexylamino)-6-pyridin-4-yl-imidazo[1,2-a]pyrazine-2-carboxylic acid amide (40)
- 6-(2-Amino-pyridin-4-yl)-8-[(4-fluoro-piperidin-4-ylmethyl)-amino]-imidazo[1,2-a]pyrazine-2-carboxylic acid amide (41)
- 8-[trans-(4-Amino-cyclohexylmethyl)-amino]-6-pyridin-4-yl-imidazo[1,2-a]pyrazine-2-carboxylic acid amide (42)
- 8-[(4-Hydroxy-piperidin-4-ylmethyl)-amino]-6-pyridin-4-yl-imidazo[1,2-a]pyrazine-2-carboxylic acid amide (44)
- 8-[(4-Fluoro-piperidin-4-ylmethyl)-amino]-6-(1-methyl-1H-pyrazol-3-yl)-imidazo[1,2-a]pyrazine-2-carboxylic acid amide (45)
- 8-[(4-Fluoro-piperidin-4-ylmethyl)-amino]-6-isothiazol-5-yl-imidazo[1,2-a]pyrazine-2-carboxylic acid amide (46)
- 8-[(4-Fluoro-piperidin-4-ylmethyl)-amino]-6-(1H-pyrazol-4-yl)-imidazo[1,2-a]pyrazine-2-carboxylic acid amide (47)
- 6-(3-Fluoro-pyridin-4-yl)-8-(4-hydroxy-4-methyl-cyclohexylamino)-imidazo[1,2-a] pyrazine-2-carboxylic acid amide (48)
- 8-[(4-Fluoro-piperidin-4-ylmethyl)-amino]-6-thiazol-5-yl-imidazo[1,2-a]pyrazine-2-carboxylic acid amide (49)

8. The compound according to claim 1, wherein said compound is selected from the following list:
- 8-[(Piperidin-4-ylmethyl)-amino]-6-pyridin-4-yl-imidazo[1,2-a]pyrazine-2-carboxylic acid amide (2)
- 6-(2-Chloro-pyridin-4-yl)-8-[(piperidin-4-ylmethyl)-amino]-imidazo[1,2-a]pyrazine-2-carboxylic acid amide (3)
- 8-[(Piperidin-4-ylmethyl)-amino]-6-pyridin-4-yl-imidazo[1,2-a]pyrazine-2-carboxylic acid methylamide (4)
- 6-(2-Chloro-pyridin-4-yl)-8-[(4-cyano-piperidin-4-ylmethyl)-amino]-imidazo[1,2-a]pyrazine-2-carboxylic acid amide (6)
- 3-Methyl-8-[(piperidin-4-ylmethyl)-amino]-6-pyridin-4-yl-imidazo[1,2-a]pyrazine-2-carboxylic acid methylamide (7)
- 8-[(4-Cyano-piperidin-4-ylmethyl)-amino]-6-pyridin-4-yl-imidazo[1,2-a]pyrazine-2-carboxylic acid amide (8)
- 6-(2-Chloro-pyridin-4-yl)-3-methyl-8-[(piperidin-4-ylmethyl)-amino]-imidazo[1,2-a] pyrazine-2-carboxylic acid methylamide (11)
- 8-[(4-Fluoro-piperidin-4-ylmethyl)-amino]-3-methyl-6-pyridin-4-yl-imidazo[1,2-a]pyrazine-2-carboxylic acid methylamide (14)
- 8-[(4-Fluoro-piperidin-4-ylmethyl)-amino]-6-pyridin-4-yl-imidazo[1,2-a]pyrazine-2-carboxylic acid amide (15)
- 8-[(4-Fluoro-piperidin-4-ylmethyl)-amino]-3-methyl-6-pyridin-4-yl-imidazo[1,2-a]pyrazine-2-carboxylic acid amide (16)
- 8-[(4-Cyano-piperidin-4-ylmethyl)-amino]-3-methyl-6-pyridin-4-yl-imidazo [1,2-a]pyrazine-2-carboxylic acid methylamide (17)
- 8-[(4-Cyano-piperidin-4-ylmethyl)-amino]-3-methyl-6-pyridin-4-yl-imidazo[1,2-a]pyrazine-2-carboxylic acid amide (19)
- 6-(2-Chloro-pyridin-4-yl)-8-[(4-fluoro-piperidin-4-ylmethyl)-amino]-3-methyl-imidazo[1,2-a]pyrazine-2-carboxylic acid methylamide (20)
- 6-(3-Fluoro-pyridin-4-yl)-8-[(piperidin-4-ylmethyl)-amino]-imidazo[1,2-a]pyrazine-2-carboxylic acid amide (27)
- 6-(3-Fluoro-pyridin-4-yl)-8-[(piperidin-4-ylmethyl)-amino]-imidazo[1,2-a]pyrazine-2-carboxylic acid methylamide (28)
- 8-[(4-Cyano-piperidin-4-ylmethyl)-amino]-6-(3-fluoro-pyridin-4-yl)-imidazo[1,2-a]pyrazine-2-carboxylic acid amide (29)
- 8-[(4-Cyano-piperidin-4-ylmethyl)-amino]-6-(3-fluoro-pyridin-4-yl)-imidazo[1,2-a]pyrazine-2-carboxylic acid methylamide (32)
- 8-[(4-Fluoro-piperidin-4-ylmethyl)-amino]-6-(3-fluoro-pyridin-4-yl)-imidazo[1,2-a]pyrazine-2-carboxylic acid amide (33)
- 6-(3-Fluoro-pyridin-4-yl)-3-methyl-8-[(piperidin-4-ylmethyl)-amino]-imidazo[1,2-a]pyrazine-2-carboxylic acid methylamide (35)
- 6-(2-Methyl-pyridin-4-yl)-8-[(piperidin-4-ylmethyl)-amino]-imidazo[1,2-a]pyrazine-2-carboxylic acid amide (36)
- 8-(cis-4-Amino-cyclohexylamino)-6-pyridin-4-yl-imidazo[1,2-a]pyrazine-2-carboxylic acid amide (39)
- 6-(2-Amino-pyridin-4-yl)-8-[(4-fluoro-piperidin-4-ylmethyl)-amino]-imidazo[1,2-a]pyrazine-2-carboxylic acid amide (41)
- 8-[(4-Hydroxy-piperidin-4-ylmethyl)-amino]-6-pyridin-4-yl-imidazo[1,2-a]pyrazine-2-carboxylic acid amide (44)
- 8-[(4-Fluoro-piperidin-4-ylmethyl)-amino]-6-isothiazol-5-yl-imidazo[1,2-a]pyrazine-2-carboxylic acid amide (46).

9. A compound of formula (I) according to any one of claims 1 to 8 for use as a medicament.

10. A compound of formula (I) according to any one of claims 1 to 8 for use in the prevention or treatment of cancer.

11. The compound for use according to claim 10, wherein the cancer is selected from Burkitt's lymphoma, chronic lymphocytic leukemias, pancreatic cancer, gallbladder carcinoma, bladder cancer, prostate cancer, melanoma, breast cancer, ovarian cancer.

12. A composition comprising a compound of formula (I) according to any one of claims 1 to 8 and a pharmaceutically acceptable excipient, diluent or carrier.
